Europäisches Patentamt

European Patent Office

Office européen des brevets

(19)

(11) **EP 1 547 464 A1**

(12) **EUROPEAN PATENT APPLICATION**
published in accordance with Art. 158(3) EPC

(43) Date of publication:
**29.06.2005 Bulletin 2005/26**

(51) Int Cl.⁷: **A01M 1/20**, A61L 9/03

(21) Application number: **03730503.4**

(86) International application number:
**PCT/JP2003/006160**

(22) Date of filing: **16.05.2003**

(87) International publication number:
**WO 2003/105579 (24.12.2003 Gazette 2003/52)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: 16.05.2002 JP 2002141087
31.05.2002 JP 2002160506
19.07.2002 JP 2002211532
19.07.2002 JP 2002211533
24.07.2002 JP 2002215896

(71) Applicant: **Earth Chemical Co., Ltd.**
**Tokyo 101-0048 (JP)**

(72) Inventors:
• **NOMURA, Yoshiharu**
**Ako-shi, Hyogo 678-0221 (JP)**
• **YUNO, Hidetoshi**
**Ako-shi, Hyogo 678-0172 (JP)**
• **KADO, Katsuyoshi**
**Ako-gun, Hyogo 678-1241 (JP)**
• **TATAMI, Kenji**
**Ako-shi, Hyogo 678-0172 (JP)**
• **HATAYAMA, Tomoko**
**Ako-shi, Hyogo 678-0172 (JP)**

(74) Representative: **Portal, Gérard et al**
**Cabinet Beau de Loménie**
**158, rue de l'Université**
**75340 Paris Cédex 07 (FR)**

(54) **METHOD OF THERMAL TRANSPIRATION, THERMAL TRANSPIRATION CONTAINER FOR USE THEREIN AND THERMAL TRANSPIRATION DEVICE**

(57)    A heat transpiration method for vermin termination in which an insecticidal technical product or a modified insecticidal technical product containing an additive is heated to transpire. For example, the insecticidal technical product 3 is put into a container 5 and the container 5 is heated so as to transpire the insecticidal technical product. With this method, an insecticide component can be transpired efficiently without need for a solvent during heat transpiration. Therefore, it becomes unnecessary to distinguish water-soluble and oil-based properties which is required when a solvent is used, and it is enabled to make the container and the heating apparatus compact.

FIG. 9

53

54

EP 1 547 464 A1

Printed by Jouve, 75001 PARIS (FR)

## Description

[TECHNICAL FIELD]

The present invention relates to a heat transpiration method used for exterminating insects indoors, a heat transpiration container used in the method, a heat transpiration apparatus for transpiring a chemical used for killing or repelling insects, giving out fragrant, deodorization or other purpose in a stable manner for an extended period time, a heat transpiration container and a method thereof preferably employed for the same, and a method of controlling the heat transpiration temperature for transpiring an insecticidal technical product indoors.

[BACKGROUND ART]

For heat transpiration of insecticidal chemicals, such methods have been known as heating an transpiring liquid that moves up in an absorbent wick so as to transpire the transpiring liquid, and heating an insecticidal chemical adsorbed into a porous base such as fibrous plate (solid mat) so as to transpire the insecticide, among others.
In the case of the former method, since the insecticide is used in the form of solution, preparation of the insecticide with water added thereto leads to poor stability of transpiration which makes the method not suitable for evaporating the chemical over a long time.
Although the latter method using the mat does not employ a solvent, substantial part of the chemical remains without being transpired, and transpiration sustained over a long period of time cannot be expected.
Thus main object of the present invention is to provide a heat transpiration method capable of efficiently transpiring the chemical and a heat transpiration container used for the same.
Another object of the present invention is to provide a heat transpiration apparatus capable of transpiring the chemical stably over a long period of time, a heat transpiration container used for the same and a method thereof.

[DISCLSURE OF THE INVENTION]

(I)

Through a research to solve the problems described above, the inventors of the present invention found a fact that transpiration of insecticidal chemical with much higher efficiency can be achieved by heating an insecticidal technical product than using an transpiring liquid to which water is added, making it possible to transpire the insecticidal chemical stably over a long period of time, thus completing the present invention. According to the present invention, since the insecticidal technical product is transpired by heating, it becomes unnecessary to use a solvent for heat transpiration and accordingly to distinguish between water-soluble and oil-based properties as in the case of insecticidal chemical in liquid phase, enabling it to make the container and the apparatus compact.
The heat transpiration method according to the present invention is a method for exterminating or repel vermin that is characterized in that the insecticidal technical product or a modified insecticidal technical product made by adding an additive to the former is heated to transpire.
The insecticidal technical product according to the present invention refers to an insecticide component per se that does not include solvent, and may be a mixture of two or more insecticide components. Since the insecticidal technical product is the insecticide component per se, an insecticidal mat of non-solvent type does not fall within the scope of the present invention. The insecticidal technical product used in the present invention refers to those prepared by adding effectiveness increasing agents, transpiration regulators, particle size regulators, stabilizers, antioxidants, ultraviolet inhibitors and colorants to the insecticidal technical product.
According to the heat transpiration method of the present invention, it is preferable to put the insecticidal technical product or the modified insecticidal technical product in a container and heat the container thereby to transpire the insecticidal technical product or the modified insecticidal technical product.
The heat transpiration method of the present invention makes it possible to transpire the insecticidal technical product or the modified insecticidal technical product while a gas-permeable film is put into contact with the liquid surface of the insecticidal technical product or the modified insecticidal technical product. This increases the amount of an insecticidal component that is transpired.
The heat transpiration method of the present invention also makes it possible to transpire the insecticidal technical product or the modified insecticidal technical product while the liquid surface of the insecticidal technical product or the modified insecticidal technical product is open to the transpiration space. This increases the amount of the insecticidal component that is transpired. The transpiration space herein refers to a space filled with air from which vermin is to be got rid of by means of the heat transpiration method of the present invention, such as a living room or a kitchen of a residence, factory, aircraft, automobile, railroad car and ship.

For the heat transpiration container used in the heat transpiration method of the present invention as described above, such a container may be used that comprises a container body that houses an insecticidal technical product or a modified insecticidal technical product consisting of the insecticidal technical product and an additive, and a gas-permeable film that closes an transpiration aperture of the container body through which the insecticidal chemical transpires. Alternatively, such a heat transpiration container may be used that comprises a container body that houses an insecticidal technical product or a modified insecticidal technical product consisting of the insecticidal technical product and an additive, and a gas-permeable film that touches the liquid surface of the insecticidal technical product or the modified insecticidal technical product at least when the insecticidal chemical is heated to transpire.

(II)

The heat transpiration apparatus of the present invention comprises a container body that contains a transpiring liquid, an inner film and an outer film that are put one on another and close an transpiration aperture of the container body, feeding means for supplying the transpiring liquid inbetween the inner film and the outer film and a heater that heats the container body, while at least the outer film of the inner film and the outer film is a gas-permeable film.

With such a construction as the inner film and the outer film (gas-permeable films) are put one on another and the transpiring liquid is supplied by the feeding means into the gap between the films, the transpiring liquid spreads by capillary effect between the inner and outer films and makes contact with the inner surface of the outer film over a large area. As a result, since the contact area of the transpiring liquid with the films does not decrease even when the amount of the transpiring liquid contained in the container body has decreased after use over a long time, stable transpiration is ensured over an extended period of time. To achieve this effect, the inner film and the outer film are put one on another in such a manner as capillary effect phenomenon occurs.

In the heat transpiration apparatus of the present invention, the feeding means is preferably a liquid introduction hole provided at a position where the inner film touches the transpiring liquid when the container body is held at such an angle as the transpiring liquid touches the inner film. This configuration makes it possible to quickly supply the transpiring liquid inbetween the inner and outer films when the container body is tilted.

The heat transpiration container of the present invention suitable for use in the heat transpiration apparatus comprises the container body that contains the transpiring liquid and the inner film and the outer film that are put one on another and close the transpiration aperture of the container, wherein at least the outer film among the inner film and the outer film is a gas-permeable film. The heat transpiration container of the present invention may be embossed to form a channel for supplying the transpiring liquid to at least one of the inner film and the outer film.

The heat transpiration container of the present invention is produced by closing the transpiration aperture of the container body with the inner film, inserting a feeder nozzle, that is used for supplying the transpiring liquid through the inner film, into the container body, supplying the transpiring liquid through the feeder nozzle into the container body and placing the outer film onto the inner film for sealing. According to the method of the present invention, such troubles can be prevented from occurring as the transpiring liquid that has spilled out when filling the container with the liquid wets the flange and causes unsatisfactory bonding.

Another heat transpiration apparatus of the present invention comprises the container body that contains the transpiring liquid, the inner film and the outer film that are put one on another and close the transpiration aperture of the container body and a heater that heats the container body, wherein the inner film is a liquid absorbent film, the outer film is a gas-permeable film and a part of the inner film makes contact with the transpiring liquid. Since a part of the inner film that is a liquid absorbent film makes contact with the transpiring liquid, the transpiring liquid is absorbed into the inner film through this portion by the capillary effect so as to infiltrate and diffuse over a large surface area on the inside of the inner film. As a result, the transpiring liquid can be brought into contact with the outer film that is put on the inner film over a large area of the inside of the outer film.

Another heat transpiration container of the present invention suitable for use in the heat transpiration apparatus described above comprises a container body that contains the transpiring liquid and an inner film and an outer film that are put one on another and close an transpiration aperture of the container body, wherein the inner film is a liquid absorbent film and the outer film is a gas-permeable film. The liquid absorbent film may be an unwoven fabric.

The transpiring liquid of the present invention may be, besides the insecticidal technical product described previously, such a material as a vermin repellent, a disinfectant, a fragrant, a deodorant or an insecticide.

(III)

Since the insecticidal technical product is a insecticide component per se that does not include a solvent, only several milliliters of the insecticidal technical product is used at the most even in an apparatus designed for long period of heat transpiration, such that 12 hours of transpiration per day is repeated for 30 to 60 days. Accordingly, the heat transpiration container is a small container comprising a sheet about 30 mm square with a recess formed at the center thereof to a depth of about 1 to 2 mm. Such a small container requires fine adjustment of the temperature in order to regulate the amount of transpiration. However, it is difficult to precisely control the temperature of the heater that heats the container so as to keep the optimum temperature for transpiration. It is also difficult to set the optimum temperature through fine adjustment of the heater for each setting when the heating temperature is changed to set the duration of transpiration for the insecticide component.

Therefore the inventors of the present invention investigated on a method of controlling the heat transpiration temperature for setting the amount of transpiring liquid such as insecticide component to be transpired, and reached a finding that surface temperature in the recess that contains the transpiring liquid can be controlled by changing the depth of the recess of the heat transpiration container and/or the flange size, even when the heater temperature is kept constant, thus enabling it to adjust the amount of transpiring liquid to transpire.

The method of controlling the heat transpiration temperature according to the present invention has been completed on the basis of the finding described above, and consists of the following methods.

(1) A method of controlling a heat transpiration temperature, which comprises controlling a surface temperature in a recess by means of the depth of the recess, in case that a container having the recess that contains a transpiring liquid is heated so as to transpire the transpiring liquid.
(2) A method of controlling a heat transpiration temperature, which comprises controlling a surface temperature in a recess by means of the surface area of a flange, in case that a container having the flange provided on the periphery of the recess that contains a transpiring liquid is heated so as to transpire the transpiring liquid.
(3) A method of controlling a heat transpiration temperature, which comprises controlling a surface temperature in a recess by means of the volume of a rib, in case that a container having the rib that protrudes from the bottom of the recess that contains a transpiring liquid into the recess space is heated to transpire the transpiring liquid.
(4) The method of controlling the heat transpiration temperature according to any one of (1) to (3), wherein the transpiring liquid is an insecticidal technical product.

In order to transpire the transpiring liquid by heating the container having the recess and the flange provided on the periphery of the recess, surface temperature in the recess may be controlled by means of either the depth of recess or the surface area of the flange.

In order to transpire the transpiring liquid by heating the container having the recess that contains the transpiring liquid, the flange provided on the periphery of the recess and the rib that protrudes from the bottom of the recess into the recess space, surface temperature in the recess may be controlled by means of at least one selected from among the depth of the recess, the surface area of the flange and the volume of the rib.

(IV)

As described previously, only a very small amount of the insecticidal technical product is used since the insecticidal technical product is the insecticide component per se that does not include a solvent. Therefore, the heat transpiration container is also small and it has been very difficult to visually recognize the amount of remaining transpiring liquid such as insecticidal technical product when the container is simply placed on the heater surface. And it is preferable that the amount of transpiration is stable when the insecticidal technical product is transpired over an extended period of time.

Therefore the inventors made a research on the heat transpiration apparatus and heat transpiration method that allow it to easily check the amount of the transpiring liquid remaining in the heat transpiration container and are capable of maintaining stable transpiration over an extended period of time. And it was found that, when the heat transpiration container filled with the transpiring liquid is placed or held on the heater surface while being tilted for heat transpiration, since the transpiring liquid makes contact with the transparent or semi-transparent gas-permeable film that closes the transpiration aperture, the liquid surface in the container can be seen through the film and the amount of the transpiring liquid that remains after a period of transpiration can be easily checked. That is, according to the present invention, the heat transpiration container serves as an indicator of the remaining amount of the transpiring liquid as it is placed or held on the heater surface at such an angle as the transpiring liquid touches the gas-permeable film.

The inventors further found such a surprising fact that the amount of transpiration of the transpiring liquid contained in the container is stabilized over a long period of time from the early stage to the latter stage of transpiration when the heat transpiration container is placed or held on the heater surface while being tilted by such an angle as the transpiring liquid touches the gas permeable film, and thereby completed the present invention. As to the reason why the amount of transpiration of the transpiring liquid contained in the container is stabilized when the heat transpiration container is placed or held on the heater surface while being tilted by such an angle as the transpiring liquid touches the gas permeable film, it is supposedly because the gas-permeable film and the transpiring liquid are kept always in stable contact with each other.

Accordingly, the heat transpiration apparatus and the heat transpiration method of the present invention have the constitution as described below.

(1) A heat transpiration apparatus comprising a heat transpiration container comprising a container body that contains a transpiring liquid and a transparent or semi-transparent gas-permeable film that closes a transpiration aperture of the container body, which is placed or held on a heater surface at such an angle as the transpiring liquid touches the film.

(2) A heat transpiration apparatus comprising a heat transpiration container comprising a container body that contains a transpiring liquid and a transparent or semi-transparent gas-permeable film that closes a transpiration aperture of the container body, which is placed or held on a heater surface that is disposed at such an angle as the transpiring liquid touches the film.

(3) The heat transpiration apparatus according to (2), wherein the heat transpiration container is placed or held on the heater surface that is disposed at such an angle from 10 to 90° from the horizontal plane.

(4) The heat transpiration apparatus according to any one of (1) to (3), wherein the transpiration aperture of the container body has inclined lower end that becomes narrower toward the end.

(5) The heat transpiration apparatus according to any one of (1) to (4), wherein the transpiring liquid is an insecticidal technical product.

(6) The heat transpiration apparatus according to any one of (1) to (5), wherein the transpiring liquid contains a colorant.

(7) A heat transpiration method, which comprises placing or holding a heat transpiration container comprising a container body that contains a transpiring liquid and a transparent or semi-transparent gas-permeable film that closes a transpiration aperture of the container body on the heater surface at such an angle that the transpiring liquid touches the film, and heating the transpiring liquid so as to transpire.

In the heat transpiration apparatus according to (1), the transpiration aperture is preferably tilted by an angle from 10 to 90° from the horizontal plane, similarly to the case of (2). Also in the heat transpiration apparatus according to (1), the heater surface may be disposed substantially horizontal (specifically, for example, 0 to 10° from the horizontal surface) as long as the transpiring liquid touches the gas-permeable film. For the transpiring liquid, besides the insecticidal technical product, such a material as a vermin repellent, a fragrant, a deodorant or an insecticide may be used.

(V)

In the heat transpiration apparatus having the heat transpiration container that comprises the transparent or semi-transparent gas-permeable film is disposed at a tilted position as described above, the liquid surface in the container can be seen through the film and the amount of the transpiring liquid that remains in the container can be easily checked. Moreover, since the transpiring liquid is caused to touch the film by the tilted positioning, the amount of transpiration becomes stabilized over a long period of time from the early stage to the latter stage of transpiration.

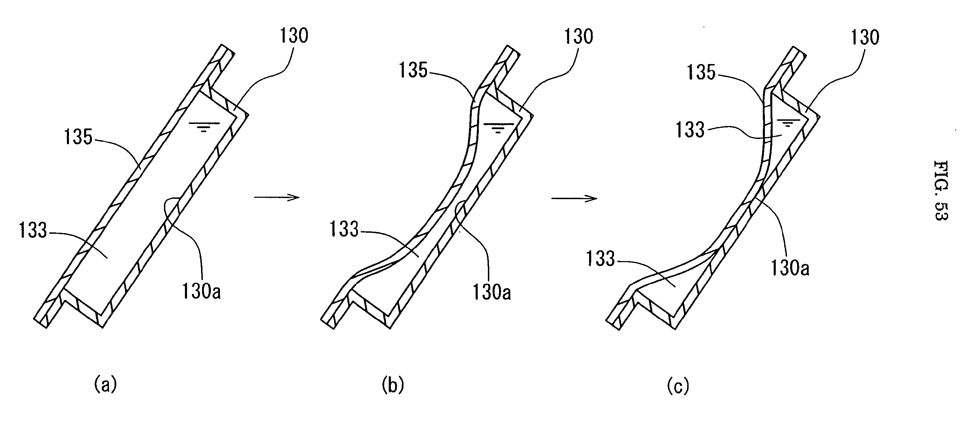

In the heat transpiration apparatus described above, in case the container is made in a very shallow construction and is substantially completely filled with the transpiring liquid, the film 135 sometimes touches the bottom 130a of the container body 130 near the center thereof as the transpiring liquid 133 decreases through transpiration, as shown in Fig. 53(a) to (c). That is, before transpiration of the transpiring liquid 133 begins as shown in Fig. 53(a), the container body 130 of which aperture is closed by the gas-permeable film 135 is filled with the transpiring liquid 133. However, a negative pressure develops in the container as the transpiring liquid 133 transpires, and therefore the film 135 approaches the bottom 130a of the container 130 as shown in Fig. 53(b) and eventually touches the bottom 130a as shown in Fig. 53(c). When the film 135 touches the bottom surface 130a of the container body 130, the transpiring liquid 133 is forced toward the periphery of the container in an uneven distribution, thus making it difficult to precisely know the amount of remaining transpiring liquid 133 since the transpiring liquid sometimes cannot flow down smoothly toward the lower end of the container body 130.

Therefore, the inventors made a research on the heat transpiration container that allows it to precisely know the amount of the transpiring liquid remaining therein, and the heat transpiration apparatus and the heat transpiration method that employ the heat transpiration container. Through the research, it was found that, when the heat transpiration container is used in vertical or tilted posture so that the transpiring liquid touches the film while an air reservoir is provided in the container body that is closed by the transparent or semi-transparent gas-permeable film so as to prevent the gas-permeable film from touching the bottom of the container body as the transpiring liquid transpires, entire transpiring liquid flows toward the bottom of the container body, and therefore it is made possible to precisely know the amount of transpiring liquid that remains in the heat transpiration container. Upon this finding, the inventors completed the present invention.

Accordingly, the heat transpiration container of the present invention comprises the container body that opens at the top and contains the transpiring liquid therein, and the transparent or semi-transparent gas-permeable film that closes the aperture of the container body, while the container body closed by the gas-permeable film has an air reservoir for preventing the gas-permeable film from touching the bottom of the container body as the transpiration of the transpiring liquid progresses. With this constitution, the heat transpiration container does not lose the function of indicator when the remaining amount of the transpiring liquid decreases so that the amount of remaining transpiring liquid can be precisely known and the amount of transpiration remains also stable.

The heat transpiration container may have such a constitution that comprises the container body that opens at the top and contains the transpiring liquid therein, and the transparent or semi-transparent gas-permeable film that closes the aperture of the container body, wherein the container body has, on one end thereof, an extended portion that is formed to protrude in the direction of depth and/or direction of width of the container body for forming the air reservoir. Providing the extended portion makes it possible to set the liquid level in the container higher and prevent the appearance of the apparatus as the commercial value from being degraded while ensuring the predetermined volume of the air reservoir.

In the heat transpiration container of the present invention, it is preferable that the bottom surface of the extended portion is inclined so that the transpiring liquid flows down from the inside of the extended portion toward the bottom of the container body, while the container body is set in vertical or inclined posture with the extended portion located in an upper position. In this posture, since the transpiring liquid does not remain in the extended portion when the heat transpiration container is held in a vertical or tilted posture, the amount of the remaining transpiring liquid can be more precisely known and the transpiring liquid can be used up to the end without waste.

The heat transpiration apparatus of the present invention is characterized in that the heat transpiration container is placed or held in the vertical or tilted posture on the heater surface so that the transpiring liquid makes contact with the gas-permeable film.

The heat transpiration apparatus of the present invention is characterized in that the heat transpiration container is heated in vertical or tilted posture so that the transpiring liquid makes contact with the gas-permeable film, to transpire the transpiring liquid.

Examples of the transpiring liquid of the present invention include insecticides, vermin repellents, fragrants, deodrants, bactericides, antifungal agents and mothproofing agents. The transpiring liquid is preferably used in the form of the effective component (for example, insecticide component) per se like the insecticidal technical product.

(VI)

The present invention provides a heat transpiration apparatus that can maintain the chemical agent such as the insecticidal technical product under such a condition that it can be transpired efficiently, and is convenient for use. This object of the invention can be achieved by the following constitution.

(1) A heat transpiration apparatus for heating a transpiring liquid holding body in a tilted posture by means of a heating section to transpire a chemical, comprising an instrument body that incorporates the heating section and a lid provided on the instrument body so as to cover the entire transpiring liquid holding body, wherein a plurality of elongated apertures are formed in the lid surface that faces the outside when the lid is closed on the instrument body, proportion of the plurality of apertures to the area of the lid surface is set to 40% or more, and each of the apertures is formed to be 5 mm or smaller along the shorter side thereof.

(2) A heat transpiration apparatus for heating a transpiring liquid holding body by means of a heating section to transpire a chemical, comprising an instrument body that incorporates the heating section and a lid provided on the instrument body so as to cover the transpiring liquid holding body, wherein aperture section is formed in the lid surface that faces the outside when the lid is closed on the instrument body, while proportion of the aperture section to the area of the lid surface is set to 40% or more, and maximum size of the aperture section is formed to be 5 mm or smaller.

(3) The heat transpiration apparatus according to (1), wherein the plurality of apertures are formed in such a manner as the length of the shorter side becomes larger toward the transpiring liquid holding body.

(4) The heat transpiration apparatus according to (3), wherein the lid has a plurality of pillars that delimit the plurality of apertures in the longitudinal direction and each of the pillars has triangular cross section.

(5) The heat transpiration apparatus according to (1), wherein the transpiration aperture is formed in the gap between the instrument body and the upper end of the lid , and/or in the instrument body, when the lid is closed on the instrument body.

(6) The heat transpiration apparatus according to (5), wherein the transpiration aperture is provided so as to open vertically upward on the top end of the transpiring liquid holding body.

(7) The heat transpiration apparatus according to (1), wherein a first air intake port is formed in the gap between the instrument body and the upper end of the lid, and/or in the instrument body when the lid is closed on the instrument body, and the first air intake port opens at a position lower in the vertical direction than the lower end of the transpiring liquid holding body.

(8) The heat transpiration apparatus according to (1), wherein a second air intake port is formed at a position opposite to the lid in substantially horizontal direction in the instrument body.

The heat transpiration apparatus according to the present invention has such a constitution as the heating section is covered by the lid that has aperture occupying a predetermined proportion of the surface area.

Specifically, the plurality of elongated apertures occupy 40% or more of the surface area of the lid. This configuration enables it to avoid such a situation as the transpired chemical deposits and remains in the gap of the aperture and the air flow is hampered resulting in a decrease in the rate of transpiration which may occur when the proportion of aperture is less than 40%. Thus the transpiration efficiency of the chemical can be maintained at a satisfactory level by setting the proper proportion of aperture.

Length of the plurality of elongated apertures along the shorter side thereof is set to 5 mm or less. This configuration prevents a foreign matter from passing through the aperture and touching the heating section or the transpiring liquid holding body that has been heated. Moreover, since the lid has such a construction that can be opened and closed, a foreign matter that has stuck in the aperture can be prevented from touching the heating section by opening the lid. Thus the present invention provides the heat transpiration apparatus that can be conveniently used while maintaining the transpiration efficiency of chemical at a satisfactory level.

The heat transpiration apparatus of the present invention may also be made in such a constitution as the plurality of elongated apertures occupy 40% or more of the surface area of the lid and the maximum dimension of the aperture is 5 mm or less.

Thus the transpiration efficiency of the chemical can be maintained at a satisfactory level by employing such a constitution as the proportion of aperture area is 40% or higher, for a reason similar to that described above.

Moreover, foreign matter can be prevented from passing through the aperture regardless of what shape the aperture has, being not limited to slit, as long as the apertures are formed with maximum dimension not larger than 5 mm in addition to the constitution described above. In addition, since the lid can be opened and closed, a foreign matter that has stuck in the aperture can be prevented from touching the heating section by opening the lid. The opening may be formed in such a shape as, for example, square, rectangle, triangle, pentagon or other polygonal shape, circle or oval.

The lid may also be, instead of open/close construction, such that can be removed and reattached.

In the heat transpiration apparatus described above, the plurality of apertures are preferably formed so that the length along the shorter side thereof increases toward the transpiring liquid holding body. This construction makes it unlikely that the transpired chemical (liquid) deposits in the gap of the aperture and enables it to maintain the transpiration efficiency of chemical at a satisfactory (normal) level. The lid has a plurality of pillars that delimit the plurality of apertures in the longitudinal direction and each of the pillars may be formed to have, for example, triangular cross section (section of the pillar that is revealed when the pillar is cut perpendicular to the longitudinal direction) with the base thereof lying on the front or back side of the lid.

In the heat transpiration apparatus described above, the transpiration aperture is preferably formed either between the instrument body and the upper end of the lid when the lid is closed on the instrument body, or in the instrument body. The transpired chemical tends to deposit particularly heavily on a portion of the instrument body located above the transpiring liquid holding body. When the transpired chemical deposits and accumulates on the portion located above the transpiring liquid holding body, rate of transpiration decreases as a part of the accumulating chemical sticks also onto the lid and hampers the air flow. Providing the transpiration aperture at this upper position enables the transpired chemical to diffuse to the outside of the instrument body through the transpiration aperture without accumulating. Thus the transpiration efficiency of chemical of the heat transpiration apparatus does not decrease.

The transpiration aperture is preferably provided so as to open facing upward in the vertical direction over the upper end of the transpiring liquid holding body.

In the heat transpiration apparatus described above, the first air intake port is preferably formed either between the instrument body and the lower end of the lid when the lid is closed on the instrument body, or in the instrument body. With this construction, since air is introduced through the first air intake port into the instrument body, the transpiration efficiency of chemical can be improved further.

It is preferable that the second air intake port is formed at a position opposite to the lid in substantially horizontal direction in the instrument body. With this construction, since air is introduced through the second air intake port into the instrument body, the transpiration efficiency of the chemical can be improved further.

The instrument body may also be provided with a retainer that elastically retains the transpiring liquid holding body. While there is no limitation on the form of the retainer, for example, leaf spring, coil spring, elastic resin plate, rubber, sponge or the like may be employed.

For the transpiring liquid holding body, besides the form of mat, such a form may also be employed as the container that contains the transpiration liquid is closed on the mouth thereof with a gas-permeable film.

The heating section may have such a configuration, for example, as the transpiring liquid holding body is heated on the back so as to transpire the chemical from the front surface of the transpiring liquid holding body. Such a heating section is preferably used that heats the transpiring liquid holding body over a certain extent of surface. Disposing the heater surface that involves the heating section at an angle of 45° with respect to the horizontal plane makes it possible to use both a power outlet and a table-tap receptacle unit having a power outlet in the top surface thereof. Also a movable plug may be provided so that both a power outlet and a table-tap receptacle unit having a power outlet in the top surface thereof can be used.

(VII)

According to the present invention, such a transpiring liquid bag may also be used as the heat transpiration container or the transpiring liquid holding body that is made of a flexible gas-permeable film on at least one side thereof and is filled with the transpiring liquid and is sealed. The transpiring liquid bag is preferably transparent or semi-transparent at least in a part thereof.

During use, the transpiring liquid bag is heated so that the transpiring liquid is transpired through the gas-permeable film provided at least on one side thereof. The transpiring liquid is heated and transpired while in contact with the gas-permeable film that is provided on one side.

[BRIEF DESCRIPTION OF THE DRAWINGS]

Fig. 1(a) is a plan view showing an example of heat transpiration container according to the present invention and Fig. 1(b) is a sectional view thereof along line X-X.

Fig. 2 is a sectional view showing the heat transpiration container sealed with a gas-permeable film that is in use.

Fig. 3(a) is a sectional view showing the gas-permeable film and the insecticidal technical product brought into contact with each other, and Fig. 3(b) is a sectional view showing the recess without the gas-permeable film attached on top thereof.

Fig. 4(a) is a sectional view showing the heat transpiration container made in such a structure as the insecticidal technical product is less likely to spill over, and Fig. 4(b) is a plan view of the container with the gas-permeable film removed from the top thereof.

Fig. 5 is a sectional view showing the heating instrument for heating the insecticidal technical product and the heat transpiration container that is housed in the heating instrument.

Fig. 6 is a perspective view showing the heating instrument for heating the insecticidal technical product.

Fig. 7(a) and Fig. 7(b) are sectional views showing another form of the heat transpiration container.

Fig. 8 is a schematic diagram showing the heat transpiration apparatus according to the second aspect of the present invention.

Fig. 9 is a perspective view showing an embossed pattern provided on the inner film used in the heat transpiration container of the present invention.

Fig. 10 is a schematic diagram showing an example where the heat transpiration container of the present invention is held on a heater surface that is erected vertically.

Fig. 11 is a plan view showing the heat transpiration container according to another embodiment of the present invention.

Fig. 12(a) to (d) are plan views showing the heat transpiration container according to further another embodiment of the present invention.

Fig. 13(a) to (d) are schematic diagrams showing the method of producing the heat transpiration container of the present invention.

Fig. 14 is a sectional view showing an example of a case where the heat transpiration container of the present invention is used in horizontal posture.

Fig. 15(a) is a plan view showing an example of heat transpiration container according to the present invention and Fig. 15(b) is a sectional view thereof along line X-X.

Fig. 16 is a sectional view showing the heat transpiration container sealed with a gas-permeable film that is in use.

Fig. 17 is a schematic sectional view showing the heat transpiration apparatus according to one embodiment of the present invention.

Fig. 18(a) is a plan view showing an example of the container body according to the present invention and Fig. 18(b) is a sectional view thereof along line X-X.

Fig. 19(a) to (c) are plan views showing another example of the container body.

Fig. 20(a) to (f) are plan views showing another example of the container body.

Fig. 21(a) to (c) are plan views showing another example of the container body.

Fig. 22(a) to (c) are plan views showing another example of the container body.

Fig. 23(a) and (b) are plan views showing another example of the container body.

Fig. 24(a) to (c) are plan views showing another example of the container body.

Fig. 25(a) to (c) are plan views showing another example of the container body.

Fig. 26 is a sectional view along the side showing another example of the heat transpiration container.

Fig. 27 is a schematic sectional view showing the heat transpiration apparatus according to the present invention holding the heat transpiration container on the heater surface erected vertically.

Fig. 28 is a schematic sectional view showing the heat transpiration apparatus according to another embodiment of the present invention.

Fig. 29 is a schematic sectional view showing the heat transpiration apparatus according to the present invention wherein the transpiration aperture is erected vertically.

Fig. 30 is a schematic sectional view showing the heat transpiration container according to the present invention and the heat transpiration apparatus using the same.

Fig. 31 is a plan view showing an example of the container body of the present invention.

Fig. 32(a) to (c) are explanatory diagrams showing the process of the insecticidal technical product in the heat transpiration container being transpired.

Fig. 33 is a schematic sectional view showing a case where the heat transpiration container of the present invention is held on the heater surface that is erected vertically.

Fig. 34 is a schematic sectional view showing the heat transpiration container of the present invention of which container body does not include an extended portion being held in an inclined posture.

Fig. 35 is an overall perspective view showing a first embodiment of the heat transpiration apparatus according to the present invention.

Fig. 36(a) shows the heat transpiration apparatus of Fig. 1 with the lid opened, and Fig. 36(b) is a sectional view of the lid shown in Fig. 36(a).

Fig. 37 is a perspective view showing an example of the transpiring liquid holding body.

Fig. 38 shows the transpiring liquid holding body attached to the lid.

Fig. 39 is an overall sectional view the heat transpiration apparatus with the transpiring liquid holding body mounted thereon.

Fig. 40 is a sectional view of the heat transpiration apparatus viewed in the direction of arrow A of Fig. 39.

Fig. 41 is a rear view of the heat transpiration apparatus.

Fig. 42 is an overall perspective view showing a second embodiment of the heat transpiration apparatus according to the present invention.

Fig. 43 is an overall perspective view showing a third embodiment of the heat transpiration apparatus according to the present invention.

Fig. 44 is a longitudinal sectional view of the heat transpiration apparatus of Fig. 43.

Fig. 45 shows the transpiring liquid holding body of the heat transpiration apparatus shown in Fig. 44.

Fig. 46(a) shows the transpiring liquid holding body of Fig. 45, Fig. 46(b) shows a variation of the transpiring liquid holding body of Fig. 46(a), and Fig. 46(c) shows another variation of the transpiring liquid holding body of Fig. 46(a).

Fig. 47(a) is a plan view showing the heat transpiration container used in third and fourth embodiments, and Fig. 47(b) is a sectional view thereof along line Z-Z.

Fig. 48 is a plan view showing the sample container used in example 9.

Fig. 49(a) to (c) are plan views showing the sample container used in Example 10.

Fig. 50(a) to (c) are plan views showing the sample container used in Example 11.

Fig. 51(a) is a plan view showing the container body used in Example 12, and Fig. 51(b) is a sectional view thereof along line Y-Y.

Fig. 52 is a plan view showing the shape of the recess of the container body used in Example 17.

Fig. 53(a) to (c) are schematic sectional views of a conventional heat transpiration container disposed in an inclined posture, showing a film brought into contact with the bottom of the container body as the transpiring liquid is consumed.

[BEST MODE FOR CARRRYING OUT THE INVENTION]

(I)

The insecticidal technical product, that can be used in the present invention, may be transpired by heating and exerts a control effect on noxious insects. Examples of the insecticidal technical product include, but are not limited to, transfluthrin, "S-1264" {common name: metofluthrin, manufactured by Sumitomo Chemical Industries Co., Ltd.}, vaporthrin, furamethrin, d-T80-furamethrin, empenthrin, phenothrin, resmethrin, phthalthrin, d-T80-phthalthrin, (1S)-1-ethynyl-2-methyl-2-pentenyl-(1R)-trans-3-(2,2-dichloroethenyl)-2,2-dimethylcyclopropanecarboxylate, 1-ethynyl-2-methyl-2-pentenyl-3-(2,2-dichloroethenyl)-2,2-dimethylcyclopropanecarboxylate, and isomers thereof, and "S-1864" {manufactured by Sumitomo Chemical Industries Co., Ltd.}, Stackle (trade name of dinotefuran), bifenthrin and Amidoflumet. The insecticidal technical product may be in the form of liquid or solid, and preferably liquid. In case of the liquid, vapor pressure of the insecticidal technical product is preferably $4.8 \times 10^{-6}$ (25°C) mmHg or higher, and more preferably from $4.8 \times 10^{-6}$ to $6.0 \times 10^{-3}$ (25°C) mmHg.

Examples of additives to be added to the modified insecticidal technical product in the present invention include stabilizers, antioxidants, ultraviolet inhibitors and colorants. If necessary, one or more kinds of these additives are added.

Examples of the antioxidants include 3,5-di-t-butyl-4-hydroxytoluene, 3-t-butyl-4-hydroxyanisole, 3,5-di-t-butyl-4-hydroxyanisole, mercaptobenzimidazole, dilauryl-thio-di-propionate, 2-t-butyl-4-methoxyphenol, 3-t-butyl-4-methoxyphenol, 2,6-di-t-butyl-4-ethylphenol, stearyl-β-(3,5-di-t-butyl-4-hydroxyphenyl)propionate, α-tocopherol, ascorbic acid, erythorbic acid, 2,2'-methylene-bis(6-t-butyl-4-methylphenol), 2,2'-methylene-bis(6-t-butyl-4-ethylphenol), 4,4'-methylene-bis(2,6-di-t-butylphenol), 4,4'-butylidene-bis(6-t-butyl-3-methylphenol), 4,4'-thio-bis(6-t-butyl-3-methylphenol), 1,1-bis(4-hydroxyphenyl)cyclohexane, 1,3,5-trimethyl-2,4,6-tris(3, 5-di-t-butyl-4-hydroxybenzyl)benzene, tris(2-methyl-4-hydroxy-5-t-butylphenyl)butane, tetrakis[methylene(3,5-di-t-butyl-4-hydroxyhydrocinnamate)] methane, octadecyl-3,5-di-t-butyl-4-hydroxyhydrocinnamate, phenyl-β-naphthylamine, N,N-diphenyl-p-phenylenediamine, 2,2,4-trimethyl-1,3-dihydroquinoline polymer, 6-ethoxy-2,2,4-trimethyl-1,3-dihydroquinoline and tetrakis[methylene3-(3,5-di-t-butyl-4-hydroxyphenyl)propionate]methane.

The colorant can be selected from azo dyes, anthraquinone dyes and combinations of two or more kinds of them. Specific examples thereof include Blue No. 403 Orange No. 403, Green No. 202, Violet No. 201, Red No. 225 and Yellow No. 204.

The amount of these additives may be an irreducible minimum amount. The amount of the stabilizers is preferably about 5% by weight or less, the amount of the antioxidant is about 5% by weight or less and the amount of the ultraviolet inhibitor is preferably about 5% by weight or less, based on the total amount of the modified insecticidal technical product. The amount of the colorant is from 0.001 to 0.1% by weight, and preferably from 0.01 to 0.1% by weight, based on the total amount. Consequently, an adverse influence is not exerted on transpiration properties of the insecticidal technical product and it becomes easier to visually recognize so that the liquid level can be easily checked from a distance and it becomes easier to know when the insecticidal technical product has been used up.

In addition, there can be optionally added commonly used various additives such as effectiveness increasing agents, transpiration regulators, particle size regulators, transpiration rate improvers, deodorizers and aromatic agents. Examples of the effectiveness increasing agent include piperonyl butoxide, N-propylisome, MGK-264, Synepirin 222, Synepirin 500, Lethan 384, IBTA, S421, amphipathic materials that exert a sufficient effectiveness increasing effect in case of transpiration by heating, and aliphatic hydrocarbons; examples of the transpiration rate improver include phenethyl isothiocyanate and high-mix acid dimethyl; examples of the deodorizer include lauric acid methacrylate (LMA); and examples of the aromatic agent include citral and citronellal.

The insecticidal technical product or the modified insecticidal technical product (hereinafter represented by the insecticidal technical product) is housed in the container and is transpired by heating the container. The heat transpiration container to be used may be any type as long as the container has a structure that allows the insecticide component to transpire.

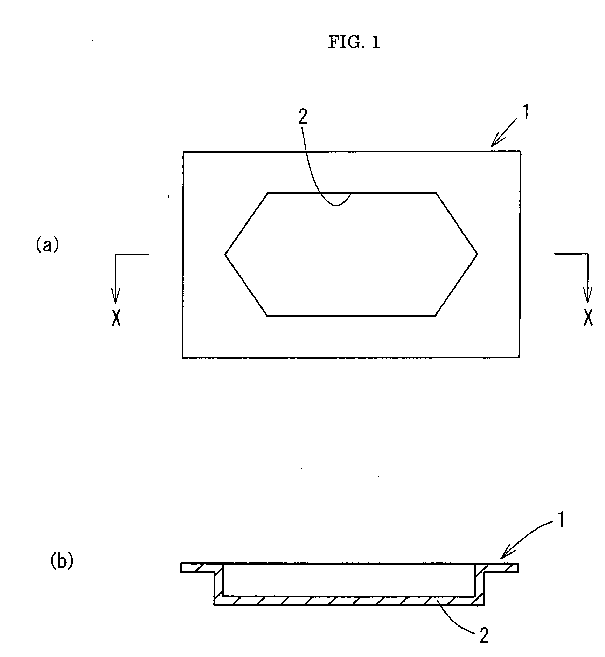

An example of the heat transpiration container as described above is shown in Fig. 1. The heat transpiration container comprises a heat resistant sheet 1 made of a metal such as aluminum or a heat resistant resin having a recess 2 formed at the center thereof, so as to contain the insecticidal technical product in the recess 2.

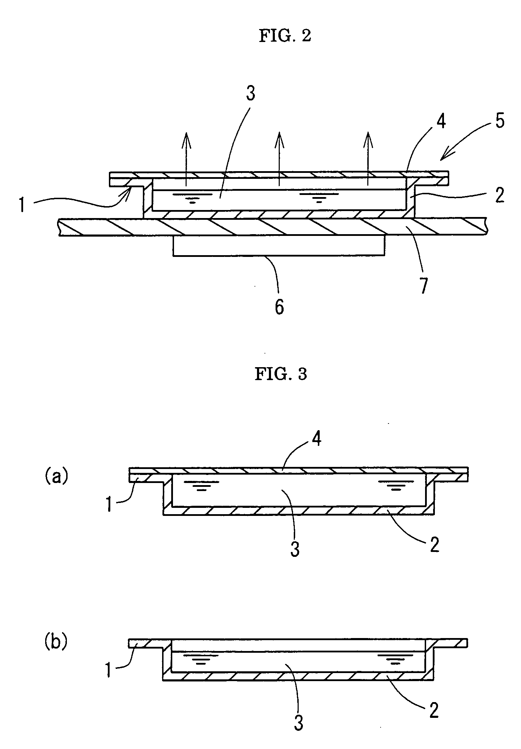

According to the present invention, as shown in Fig. 2, the heat transpiration container may have such a structure as the transpiration aperture at the top of the recess 2 that contains the insecticidal technical product 3 is closed by the gas-permeable film 4. This structure prevents the insecticidal technical product 3 from spilling from the container 5 and makes it easier to handle including transportation.

To use the heat transpiration container 5, it is placed on a radiation plate 7 having a heating section 6 disposed below thereof as shown in Fig. 2, so as to heat the insecticidal technical product 3 contained inside by the heated from the radiation plate 7. The insecticide component that has transpired from the insecticidal technical product 3 diffuses through the gas-permeable film 4 (transpiration of the insecticide component is indicated by arrows).

The amount of the insecticidal technical product 3 housed in the container 5 is not specifically limited because it is appropriately decided according to transpiration properties of chemicals to be used, transpiration amount per hour and target transpiration duration time, and is commonly from about 0.05 to 3 ml, and preferably from about 0.1 to 1 ml. The amount of the insecticidal technical product 3 is commonly from about 75 to 4500 mg, and preferably from 100 to 1500 mg. If the transpiration amount of the insecticide transfluthrin is 1.67 mg/2 hours and if transpiration is conducted by the heat transpiration method of the present invention for 12 hours per day, the amount of the insecticidal technical product 3 for 30 days is about 300 mg, while the amounts of the insecticidal technical products for 60 days and 120 days are respectively about 600 mg and 1200 mg. By the way, the effective transpiration amount of transfluthrin is preferably at least 0.15 mg/2 hours so as to control mosquito.

While there is no limitation on the depth and capacity of the recess 2 which depend on the amount of the insecticidal technical product contained therein, depth of the recess 2 may be set in a range from 0.3 to 5 mm, preferably from 0.5 to 4 mm according to the amount of the insecticidal technical product.

The gas-permeable film 4 is not specifically limited as far as it can pass the chemical component transpired from the insecticidal technical product therethrough. Examples of the gas-permeable film 4 include those made of oriented or cast polypropylene, polyethylene (for example, linear low-density polyethylene, low-density polyethylene, etc.), polyethylene terephthalate and polymethylpentene. These films may be used alone, or two or more kinds of them may be laminated with each other.

A microporous film having multitude of tiny holes formed therein by means of laser beam may also be used individually as the gas-permeable film or in combination with another gas-permeable film being laminated therewith. Moreover, a porous film having holes formed by piercing with a needle or the like may also be used individually as the gas-permeable film or in combination with another gas-permeable film being laminated therewith. Thickness of the gas-permeable film 4 is in a range from about 10 to 100 $\mu$m, preferably from 30 to 70 $\mu$m. For the microporous film or the porous film, a film impermeable to gas such as nylon may also be used besides the gas-permeable film.

The gas-permeable film 4 to be used is preferably such that is effective in evaporating the chemical and does not undergo significant change in the appearance (degradation such as swelling and breakage and crystallization (powder formation) of the effective component on the film surface). As such a film, for example, composite film comprising two or more kinds of film laminated together may be used. For example, composite film comprising a sealant layer made of polyethylene or cast polypropylene, and an oriented polypropylene film, a polyterephthalate film or a perforated film (such as perforated polyterephthalate film) laminated on the outside thereof may be used. The sealant film herein refers to a film that is directly bonded onto the heat transpiration container 5 so as to ensure close contact with the heat transpiration container 5 and liquid tightness. Thickness of the composite film may be the same as that of the gas-permeable film 4 in a range from about 10 to 100 $\mu$m. The sealant layer may also be provided individually on top surface of the container 5 (adhering surface with the film 4).

In order to prevent the gas-permeable film 4 from touching the bottom of the recess 2 when a negative pressure develops in the recess as the chemical transpires, an air intake port (not shown) may be provided in the heat transpiration container 5 so that air is supplied into the recess 2 that is sealed by the gas-permeable film 4. Such an air intake port is provided, for example, in a part of the gas-permeable film 4.

While the insecticidal technical product may be brought into direct contact with a heat source to heat and transpire the insecticidal technical product, the insecticidal technical product may also be heated by placing the container 5 on the heater such as the radiation plate 7 or by providing a gap or other intermediate object between the container 5 and the heater.

While there is no limitation on the heating temperature of the insecticidal technical product which may be set to any level that is high enough to transpire the effective amount of the insecticide component, the heater temperature (for example, surface temperature of the radiation plate 2) is normally in a range from about 50 to 170°C, preferably from 70 to 130°C, in the case of the heating method described above that uses the container.

Alternatively, the surface temperature of the gas-permeable film 4 may be set in a range from about 40 to 160°C, preferably from 60 to 120°C. Temperature of the insecticidal technical product during heating the container 5 is in a range from about 45 to 165°C, preferably from about 65 to 125°C. The heating temperature may be determined according to the effective amount of insecticide component to be transpired required to get rid of vermin such as mosquitoes.

Thus according to the present invention, the insecticide component can be transpired continuously over a long period of 30 days or longer, by heating the insecticidal technical product to such a temperature as effective amount of the insecticide component is transpired for about 12 hours per one day.

When heating the chemical to transpire, it is preferable to hold the container 5 and the radiation plate 7 in a tilted posture so that the insecticidal technical product 3 and the gas-permeable film 4 come into contact with each other before or when the apparatus is used. This makes it easier to check the decrease in the amount of insecticidal technical product in the container 5 based on the change in the liquid level of the insecticidal technical product, so that the container serves as an indicator of the remaining amount of the insecticidal technical product and the amount of transpiration can be stabilized over a long period. Tilting angle of the container 5 from the horizontal surface is in a range from 10 to 90°, preferably from 40 to 60°.

According to the heat transpiration method of the present invention, such a method may be employed as the heat transpiration container, as shown in Fig. 3(a), comprising the heat resistant sheet 1 (container body) that has the recess 2 containing the insecticidal technical product 3 and the gas-permeable film 4 that touches the liquid surface of the insecticidal technical product 3 is used, so as to heat and transpire the insecticidal technical product 3 under the condition that the gas-permeable film 4 that touches the liquid surface of the insecticidal technical product 3, or as shown in Fig. 3(b), the insecticidal technical product 3 is heated under the condition that the liquid surface of the insecticidal technical product 3 is exposed to the transpiration space, namely without the gas-permeable film 4 placed thereon, which further increases the amount of the insecticide component transpired.

In the case shown in Fig. 3(a), in order to keep the gas-permeable film 4 in contact with the insecticidal technical product 3 even when only a small amount of the insecticidal technical product 3 remains with the transpiration of the insecticide component, for example, the gas-permeable film 4 may be slackened in advance, a insecticidal technical product supply means (not shown) that maintains the liquid level constant may be provided, or the container body may be held in a tilted posture (from 10 to 90°, preferably from 40 to 60°) from the horizontal surface. In the case of the heat transpiration container 5 that is held horizontally as shown in Fig. 2, the gas-permeable film 4 can be brought into contact with the liquid surface of the insecticidal technical product 3 by tilting the container 5 with respect to the horizontal surface at least during heating and transpiration, even if the gas-permeable film 4 is not in contact with the liquid surface of the insecticidal technical product 3 in the horizontal posture.

In the case shown in Fig. 3(a), those listed above as the examples may be used as the gas-permeable film 4. When a film through which the insecticidal technical product 3 can easily permeate and transpire from the surface thereof is used, the amount of insecticide component transpired can be increased. Such a liquid-permeable film through which the insecticidal technical product 3 can permeate may be composed of polypropylene, polyethylene or copolymer of ethylene and propylene. These films may be used either alone or in combination of two or more kinds being laminated together. Thickness of the gas-permeable film 4 is an important factor that is related to the ease of the insecticide component to permeate and the amount of transpiration, and is preferably from about 10 to 100 μm, and more preferably from about 25 to 75 μm.

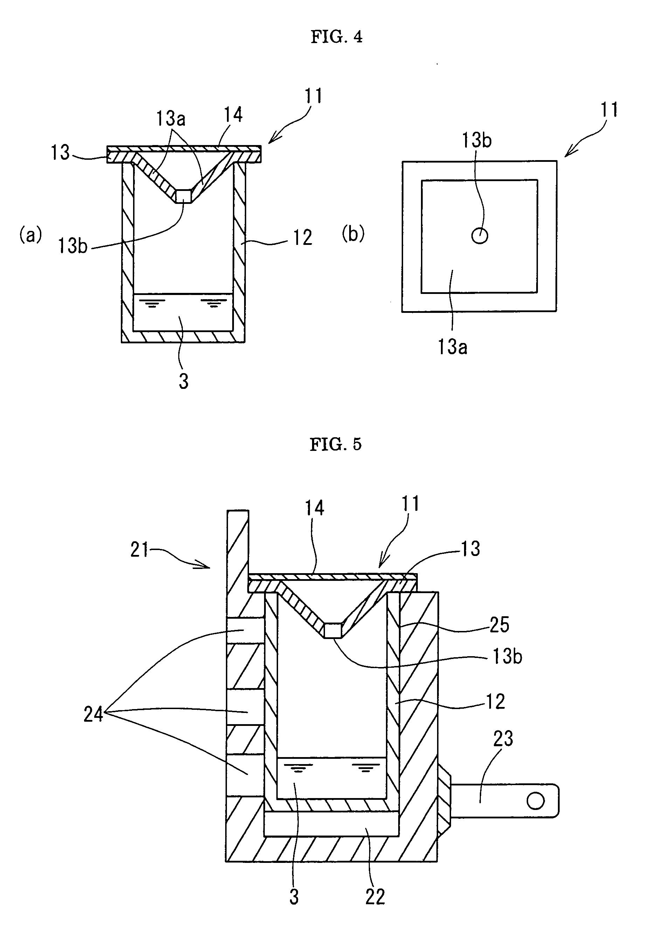

In the case shown in Fig. 3(b), where the gas-permeable film 4 is not used, since the recess 2 opens at the top (insecticidal technical product 3 is exposed to the transpiration space) as shown in the drawing, it is preferable to use a heat transpiration container 11, for example, as shown in Fig. 4(a) and (b).

Fig. 4(a) and (b) are a sectional view of the heat transpiration container 11 having such a structure that the insecticidal technical product 3 is unlikely to spill over, and a plan view of the heat transpiration container 11 with a film 14 removed, respectively. As shown in Fig. 4(a) and (b), the heat transpiration container 11 comprises a container body 12 formed in a box shape from a metal such as aluminum or a heat resistant resin and a lid 13 thereof, and contains the insecticidal technical product 3 in the container body 12. The lid 13 has an inclined portion 13a that inclines from the upper end of the rim of the container body 12 downward to the inside, and an transpiration aperture 13b at the center of the inclined portion 13a. A film 14 that is not gas-permeable is attached to the top surface of the lid 13, and transpiration of the insecticide component is enabled by peeling off the film 14 before use. The lid 13 may be simply placed on the container body 14, but it is preferable to integrate the lid and the container body by adhesion or to make the lid 13 and the container body 12 by integrated molding.

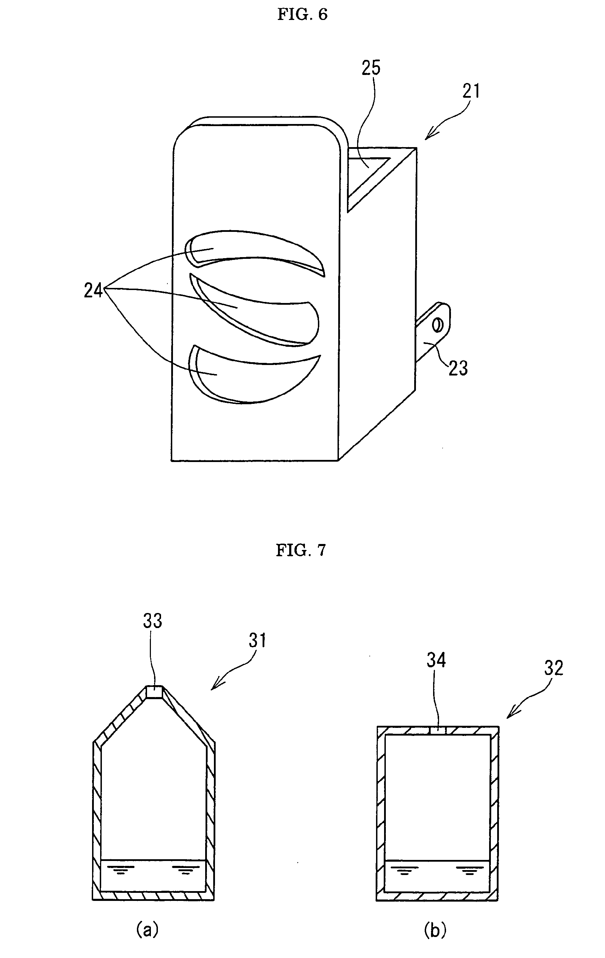

When the heat transpiration container 11 is used, a heating instrument 21 may be used for heating the insecticidal technical product 3 as shown in Fig. 5 and Fig. 6. The heating instrument 21 is a box-shaped object that comprises a recess 25 that detachably accommodates the heat transpiration container 11, a heater 22 mounted at the bottom of the recess 25 for heating the heat transpiration container 11 and an electric plug 23 for supplying electric power to the heater 22. The heating instrument 21 has a window 24 for checking the liquid level of the insecticidal technical product 3 in the surface opposite to the side where the electric plug 23 is provided. Thus the remaining amount of the insecticidal technical product 3 can be easily checked through the window 24. Accordingly, if the heating instrument 21 is used, the heat transpiration container 11 is preferably made of a transparent or semi-transparent heat resistant resin.

During thermal transpiration, the insecticidal technical product 3 is heated by the heat from the heater 22 under the condition that the heat transpiration container 11 is accommodated in the recess 21, so that the insecticide component transpires through the transpiration aperture 13b. When all of the insecticidal technical product 3 in the heat transpiration container 11 has been consumed through transpiration, the heat transpiration container 11 is taken out of the heating instrument 21 and replaced with new heat transpiration container 11 filled with the insecticidal technical product 3. Alternatively, the insecticidal technical product 3 may be replenished into the heat transpiration container 11 of which insecticidal technical product 3 has been used up.

The heating instrument 21 may be provided with an open/close lid or detachable lid (not shown) to close the top of the heat transpiration container 11 when not in use. The heat transpiration container 11 and the heating instrument 21 may have polygonal or circular shape instead of rectangular in the plan view.

The heat transpiration container may have a construction other than that comprising the box-shaped container body 12 and the lid 13 shown in Fig. 4(a) and (b), as long as transpiration aperture 33, 34 is provided on top of container 31, 32 as shown in Fig. 7(a) and (b), for example, so that the liquid surface of the insecticidal technical product can be exposed to the transpiration space. There is no limitation on the shape of the container 31, 32 in plan view, which may be rectangular, polygonal other than rectangular or circular shape.

The heat transpiration method of the present invention can be applied for control of various noxious insects that should be controlled, for example, sanitary noxious insects or blood-sucking noxious insects, such as cockroach, fly, mosquito, biting midge, horsefly, flea and bedbug; clothing noxious insects such as clothes moth and Tineola bisseliells; stored grain noxious insects such as rust-red flour beetle and rusty grain beetle; mites such as Ornithonyssus, Cheyletidae and Acaridae; and ant, termite and slug.

Therefore, the heat transpiration method of the present invention can be used in various locations, and can be preferably used in the living room, the kitchen or the dining room of a home, livestock barn, kennel, green house, clothes storage such as closet or wardrobe, food cellar, etc.

While the foregoing description has dealt with cases where the insecticidal technical product or the modified insecticidal technical product is heated to transpire, the insecticidal technical product or the modified insecticidal technical product may be volatile at normal temperature, in which case the insecticidal technical product or the modified insecticidal technical product (for example, the compound A or B described previously) may be exposed to open air so as to spontaneously transpire at the normal temperature.

(II)

The heat transpiration apparatus, the heat transpiration container and the method of producing the same according to the present invention will now be described below by way of example where the insecticidal technical product is used as the transpiring liquid. The insecticidal technical product is similar to that described previously and may include the additive.

When a colorant such as pigment is added to the insecticidal technical product (transpiring liquid), in particular, it becomes easier to visually recognize so that the liquid level can be easily checked from a distance and it becomes easier to know when the insecticidal technical product has been used up. The colorant is preferably such that does not affect the transpiration of the insecticidal technical product, does not experience color fading due to exposure to heat or light over the period of use, and can be easily seen from a distance so that it can be known when the insecticidal technical product has been used up.

Such a colorant can be selected from azo dyes, anthraquinone dyes, and combinations of two or more kinds of these dyes. Specific examples thereof include dyes such as Orange No. 403, Green No. 202, Violet No. 201, Red No. 225 and Yellow No. 204. The amount of the colorants is commonly for 0.001 to 0.1% by weight, and preferably from 0.01 to 0.1% by weight, based on the total amount. Consequently, an adverse influence is not exerted on transpiration properties of the insecticidal technical product and it becomes easier to visually recognize so that the liquid level can be easily checked from a distance and it becomes easier to know when the insecticidal technical product has been used up.

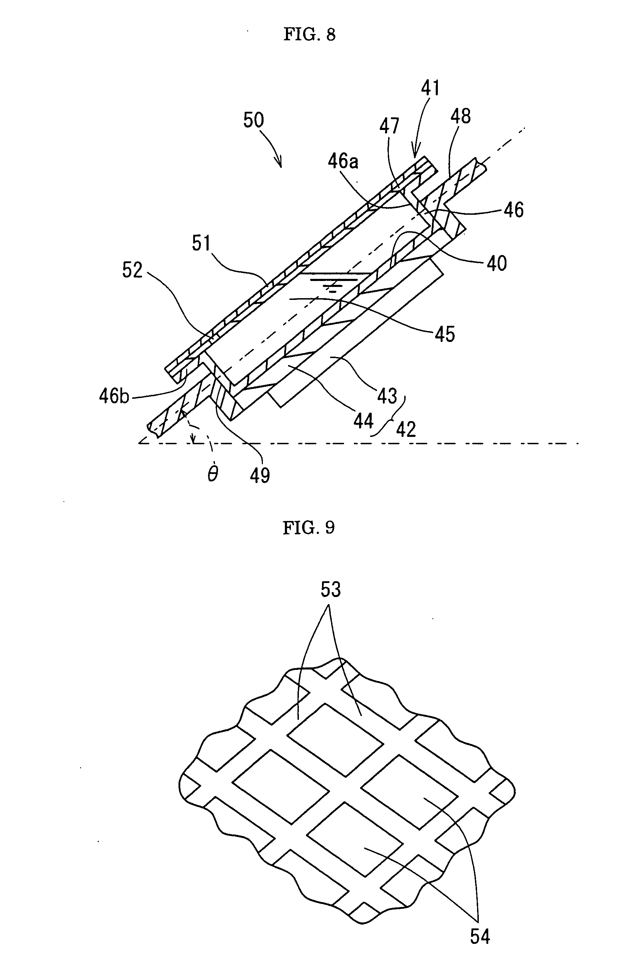

Fig. 8 is a schematic diagram showing the heat transpiration apparatus according to this embodiment. As shown in Fig. 8, the heat transpiration apparatus 50 has a heat transpiration container 41 that comprises a container body 46 which contains insecticidal technical product 45, inner film 47 and outer film 51 which are laminated with each other and close the transpiration aperture of the container body 46 and a liquid introducing port 52 (supplying means) provided on the inner film 47 for supplying the insecticidal technical product 45 between the films 47, 51, and a heater 43 that heats the container body 46.

The heater 42 comprises a heating element 43 and a radiation plate 44. The radiation plate 44 is installed inside of the inclined wall 48 of the heat transpiration apparatus 50, and forms the bottom of the recess 49 that is formed in the wall 48. The heat transpiration container 41 is dropped into the recess 49 so that the heat transpiration container 41 is held on the heater surface 40.

The outer film 51 may be composed of any gas-permeable film that does not allow the liquid insecticidal technical product 45 to permeate but allows gaseous chemical component transpired from the insecticidal technical product 45. For the outer film 51 that is permeable to gas, a material similar to the gas-permeable film 4 may be used.

For the inner film 47, on the other hand, a film not permeable to gas that does not allow the liquid insecticidal technical product 45 and the chemical component that has transpired therefrom to permeate may be used, in addition to a gas-permeable film similar to the outer film 51. Thickness of the inner film 47 is in a range from about 10 to 100 µm, and preferably from 30 to 70 µm. The inner film 47 is preferably such that does not change the properties during use, similarly to the outer film 51.

The inner film 47 has a liquid introducing port 52 (supplying means) for supplying the insecticidal technical product 45 between the inner and outer films 47, 51 as shown in Fig. 8. The liquid introducing port 52 is formed at a position where the inner film 47 touches the insecticidal technical product 45 when the container body 46 is held at such an angle as the insecticidal technical product 45 makes contact with the inner film 47. While there is no limitation on the diameter of the liquid introducing port 52, it is preferably around 0.5 to 2 mm.

The container body 46 is made of a metal such as aluminum or a heat resistant resin having a recess 46a formed at the center thereof, so as to contain the insecticidal technical product 45 therein, and a flange 46b is provided on the periphery of the transpiration aperture of the recess 46a. The inner film 47 is placed so that the periphery thereof is located on the flange 46b, which are bonded by thermal fusing or the like, and the outer film 51 is placed so that the periphery thereof is located on the fused portion, which are bonded by thermal fusing or the like.

Other than the periphery, the inner film 47 and outer film 51 are simply placed one on another so as to allow the capillary phenomenon of the insecticidal technical product 45, and are not bonded. In order to allow the capillary phenomenon of the insecticidal technical product 45 between the inner film 47 and outer film 51, the inner film 47 and outer film 51 may be bonded together on the periphery so that the films will not be wrinkled or slackened and the gap between the films does not expand excessively. If the gap becomes too large, it makes it difficult for the capillary phenomenon to occur, and the insecticidal technical product 45 may not be able to contact the inside of the outer film 51 over a sufficient area.

When placing the inner film 47 and outer film 51 one on another, a spacer comprising a film may be interposed between the films along the periphery to ensure a predetermined gap between the inner and outer films 47, 51 so that capillary phenomenon occurs satisfactorily.

At least one of the inner film and outer film may be embossed to form a pattern of projection and recess so as to provide channel for supplying the liquid. Fig. 9 is a perspective view showing the embossed pattern formed on the inner film 47. As recess 53 and projection 54 are formed at predetermined intervals between the inner and outer films 47, 51 as shown in the drawing by embossing, the insecticidal technical product 45 can be supplied stably by the capillary phenomenon. Also because the contact area between the insecticidal technical product 45 and the outer film 51 can be adjusted, the amount of the insecticidal technical product 45 transpired can be controlled. There is not limitation on the pattern of projection and recess as long as the insecticidal technical product 45 is obstructed from spreading over the inner surface of the outer film 51, and grating, slit or other shape may be employed. The gap thus formed is preferably 1 mm or smaller.

The inner film 47 and outer film 51 are preferably transparent or semi-transparent, which makes it possible to easily recognize the liquid level of the insecticidal technical product 45 in the heat transpiration container 41, and easily check the remaining amount of the insecticidal technical product 45 after a period of thermal transpiration. Visual recognition of the liquid level can be improved further by adding a colorant as described previously.

Angle θ of inclination of the heater surface 40 from the horizontal surface shown in Fig. 8 is preferably such as the transpiring liquid makes contact with the inner film 47, specifically from 10 to 90°, preferably from 40 to 60° and more from 45 to 55°. When the angle θ of inclination is in the range described above, even when the insecticidal technical product 45 decreases, the insecticidal technical product 45 can be supplied through the liquid introducing port 52 between the inner and outer films 47, 51 so that the insecticidal technical product 45 makes contact with the outer film 51 over a large surface area by capillary effect. With this configuration, since contact area between the outer film and the transpiring liquid does not decrease with the decrease of the transpiring liquid in the container body after a long period of use, transpiration can be sustained over a long period of time. When the angle θ of inclination is less than the range described above, it becomes difficult to supply the insecticidal technical product 45 through the liquid introducing port 52 between the inner and outer films 47, 51 when amount of the insecticidal technical product 45 decreases.

There is no limitation on the amount of the insecticidal technical product 45 contained in the heat transpiration container 41 which may be determined by taking into consideration the transpiration performance of the chemical that is used, amount of transpiration per unit of time and the target duration of transpiration, and is usually from about 0.05 to 3 ml, preferably from about 0.5 to 5 ml.

Target temperature of heating the insecticidal technical product is determined by taking into consideration the amount of insecticide component transpired per unit of time, and is set so that the heater temperature (temperature on the heater surface 40) is from about 50 to 170°C, preferably from 70 to 130°C.

While the heat transpiration container 41 is held in the recess 49 formed in the wall 48 around the heater surface 40 in the embodiment described above, the holding means may be anything that can place or hold the heat transpiration container 41 on the heater surface 40 and, for example, a stopper such as a projection may be provided on the inclined heater surface or at an end of the heater surface so as to place or hold the heat transpiration container.

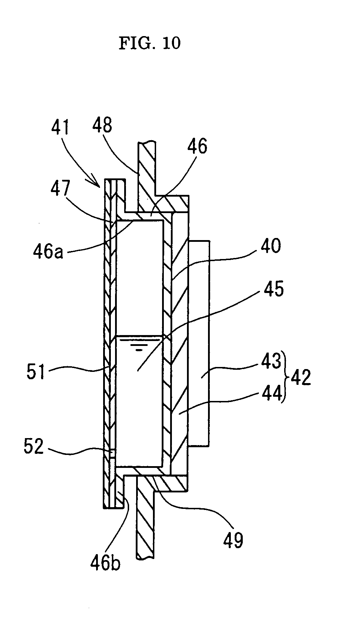

Fig. 10 shows an example of holding the heat transpiration container 41 on the heater surface 40 that is erected vertically. This form also makes it possible to supply the insecticidal technical product 45 through the liquid introducing port 52 between the inner and outer films 47, 51 so that the insecticidal technical product 45 is brought into contact with the outer film 51 over substantially the entire area thereof by the capillary phenomenon, similarly to the case of inclined heater surface. With other respect, this embodiment is the same as that shown in Fig. 8, and description will be omitted with the identical reference numerals assigned to the corresponding components.

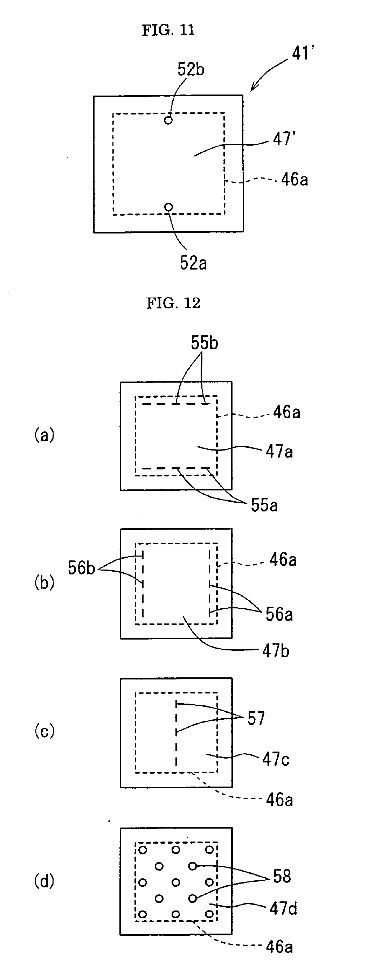

Fig. 11 is a plan view showing another embodiment of the heat transpiration container of the present invention. In Fig. 11, only the inner film 47' is shown while omitting the outer film 51 for the convenience of explanation. As shown in Fig. 11, the inner film 47' has two liquid introducing ports 52a, 52b (supplying means) provided near the opposing wall surfaces of the container body 46 for supplying the insecticidal technical product 45 between the inner and outer films 47' and 51. With this configuration, the object can be achieved regardless of which of the liquid introducing port 52a, 52b of the heat transpiration container 41' is located at the lower position. The liquid introducing port located at the upper position can serve the function of degassing from the container body 46.

The liquid introducing port used in the heat transpiration apparatus 50 is not limited to the forms shown in Fg. 8, Fig. 10 and Fig. 11, and may also be such as shown in Fig. 12(a) to (d). The outer film is not shown in Fig. 12(a) to (d), similarly to Fig. 11. The liquid introducing port 55a, 55b shown in Fig. 12(a) consist of notches provided along lateral lines on top and bottom of the inner film 47a when it is placed in the heat transpiration apparatus 50. The liquid introducing port 56a, 56b shown in Fig. 12(b) consist of notches provided along longitudinal lines on the left and right sides of the inner film 47b. The liquid introducing port 57 shown in Fig. 12(c) consists of notches provided along longitudinal line at the center of the inner film 47c. The liquid introducing port 58 shown in Fig. 12(d) consists of a plurality of holes provided in the inner film 47d to such an extent as the liquid does not spill.

For the method of heating the insecticidal technical product 45, besides placing the heat transpiration container 41 on the radiation plate 44 as in the embodiment described above, a gap or other intermediate object may be provided between the heat transpiration container 41 and the radiation plate 44.

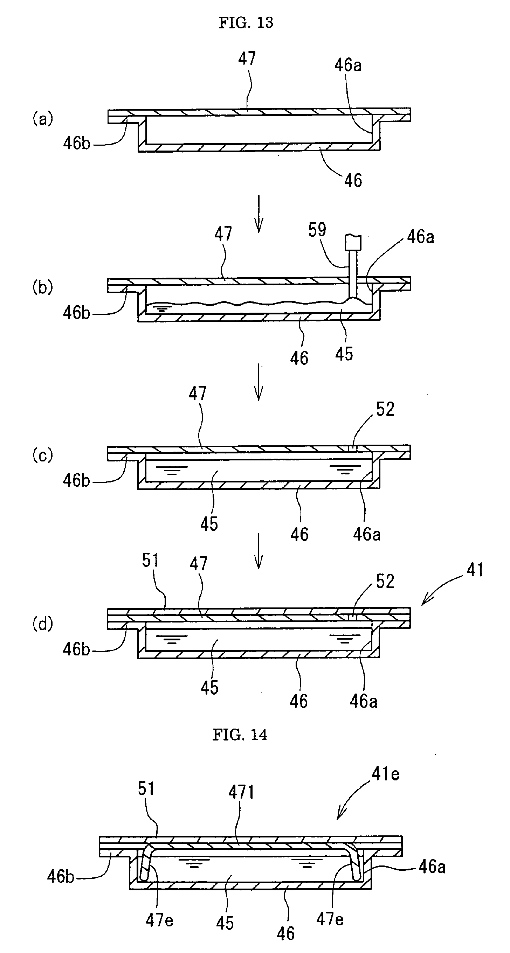

Now the method of producing the heat transpiration container 41 may be described below. Fig. 13(a) to (d) are schematic diagrams showing the method of producing the heat transpiration container 41.

First, the container body 46 having the recess 46a provided at the center thereof and the inner film 47are prepared. The inner film 47 is placed with the periphery thereof located on the flange 46b of the container body 46, and the flange and the film are bonded by thermal bonding or the like so as to close the transpiration aperture of the container body 46 with the inner film 47 (Fig. 13(a)). Then a feeder nozzle 59 that supplies the insecticidal technical product 45 is put into the container body 46 by piercing the inner film 47, and the insecticidal technical product 45 is supplied through the feeder nozzle 59 into the container body 46 (Fig. 13(b)). After supplying a predetermined amount of insecticidal technical product 45, the feeder nozzle 59 is pulled out of the container body 46. The hole that is left after pulling the feeder nozzle 59 out of the container body 46 can be used as the liquid introducing port 52 (feeding means) (Fig. 13(c)). Then the outer film 51 that is permeable to gas is placed on the outside of the inner film 47, and the two films are bonded by thermal bonding or the like along the periphery thereby to seal the container body 46 (Fig. 13(d)). Thus, the heat transpiration container 41 is obtained. Tip diameter of the feeder nozzle 59 may be determined according to the size of the liquid introducing port 52. Instead of using the hole made by the feeder nozzle 59 as the liquid introducing port 52 as described above, the liquid introducing port 52, 13a, 13b, 55a, 55b, 24a, 24b, 25, 26 may be provided in the inner film 47 as shown in Fig. 8 and Fig. 10-5 before or after bonding the inner film 47 with the container body 46.

While the embodiment described above is a case where the liquid introducing port 52 is used as the feeding means, an end of one of the inner film and outer film that are placed one on another and immersed in the insecticidal technical product may also be used as the means of supplying the insecticidal technical product between the inner and outer films. That is, when the end of the inner film and outer film is immersed in the insecticidal technical product, the insecticidal technical product rises through the immersed portion to spread between the inner and outer films by the capillary phenomenon, so that the insecticidal technical product can be brought into contact with the outer film over a large area of the inner surface thereof. This enables it to use the container body 46 while being placed horizontally.

According to the present invention, for example, unwoven fabric or woven fabric made of a polymer that has high capability to absorb oil, blotting paper or other liquid absorbing film may be use as the inner film 47. Since such a liquid absorbing film absorbs the insecticidal technical product 45, the insecticidal technical product can be caused to infiltrate the inner film 47 over a large area thereof, by tilting the container body 46 or otherwise bringing the insecticidal technical product 45 into contact with a part of the inner film 47. Thus the insecticidal technical product can be brought into contact with the outer film 51 that is put on the inner film 47, over a large area of the inner surface thereof.

While the inner film 47 (liquid absorbing film) and the outer film 51 (gas-permeable film) are bonded along the periphery thereof onto the flange 46b of the container body 46 also in the case of using the liquid absorbing film as described above, it is preferable that the inner film 47 is bonded with the outer film 51 in a part (the portion that covers the transpiration aperture of the container body 46) other than the periphery or over the whole area of the inner film 47, and it is more preferable that the whole area of the inner film 47 is bonded with the outer film 51. This makes it possible to bring the insecticidal technical product 45 into stable contact with the outer film 51 over a larger area of the inner surface thereof, since there is no gap created between the inner film and outer film. Thus when a liquid absorbing film is used as the inner film 47, the inner film 47 may or may not provided with the liquid introducing port 52.

When a liquid absorbing film is used as described above, the container body 46 can be used while being placed in horizontal posture. An example of using the container body 46 in horizontal posture is shown in Fig. 14. In this heat transpiration container 41e, as shown in Fig. 14, a piece 47e formed by tearing the inner film 471 from an end thereof is hang down so as to immerse in the insecticidal technical product 45. By using the liquid absorbing film for the inner film 471, it is made possible to suck up the insecticidal technical product 45 by means of the piece 47e and cause the insecticidal technical product 45 to infiltrate the inner film 471 over a large area thereof. Thus the insecticidal technical product 45 can be caused to make contact with the outer film 51 that is placed on the inner film 471 over a large area of the inner surface of the outer film 51. When tearing the piece 47e, a part of the outer film 51 may be torn at the same time to hang these pieces that are put one on another into the insecticidal technical product 45.

Even when the inner film 471 is not a liquid absorbing film, if the piece 47e of the inner film 471 and the inner surface of the recess 46a are located near to each other, the insecticidal technical product 45 can be sucked up and supplied between the inner and outer films 471 and 51 by the capillary phenomenon generated between the inner surface and the piece 47e.

The heat transpiration apparatus of the present invention and the heat transpiration container used therefor can be applied for control of noxious insects that should be controlled, for example, sanitary noxious insects or blood-sucking noxious insects, such as cockroach, fly, mosquito, biting midge, horsefly, flea and bedbug; clothing noxious insects such as clothes moth and Tineola bisseliells; stored grain noxious insects such as rust-red flour beetle and rusty grain beetle; mites such as Ornithonyssus, Cheyletidae and Acaridae; and ant, termite and slug. Therefore, the heat transpiration apparatus and the heat transpiration container of the present invention can be used in various locations, and can be preferably used in the living room, the kitchen or the dining room of a home, livestock barn, kennel, green house, clothes storage such as closet or wardrobe, food cellar, etc.

According to the present invention, besides the insecticidal technical product described in the embodiments described above, aromatic agents, deodorizers and mothproofing agents may also be used as the transpiring liquid. The transpiring liquid used in the present invention may have such a degree of fluidity as the capillary phenomenon occurs.

While the foregoing description has dealt with cases where the transpiring liquid is heated to transpire, the transpiring liquid may be volatile at normal temperature, in which case the transpiring liquid may be exposed to open air so as to spontaneously transpire at the normal temperature.

(III)

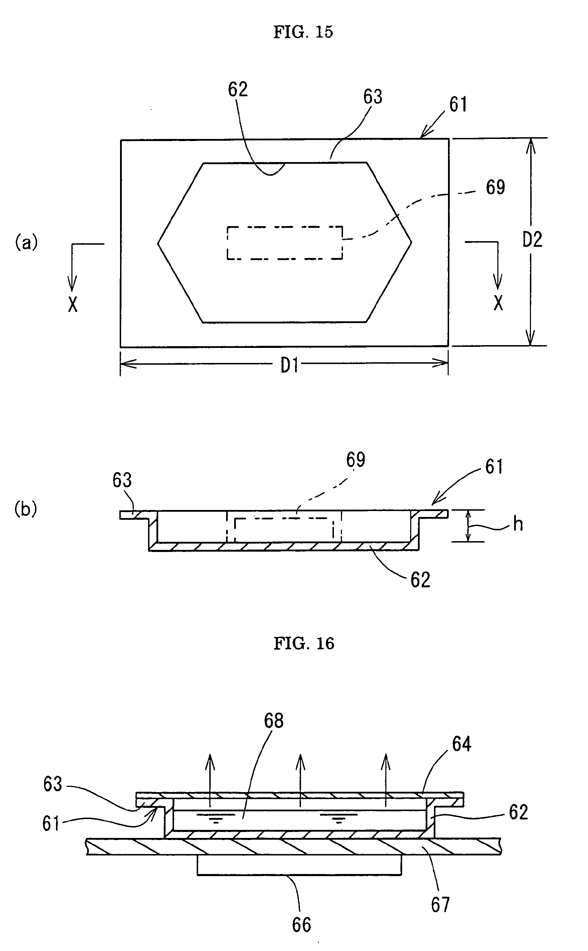

An example of container used for heating and evaporating the insecticidal technical product is shown in Fig. 15. The container 61 comprises a heat resistant sheet made of aluminum or other metal or a heat resistant resin having a recess 62 formed at the center thereof, so as to contain the insecticidal technical product therein, and a flange 63 is provided on the periphery of the aperture of the recess 62. The container 61 may be reinforced as required by providing a rib 69 protruding from the bottom of the recess 62 toward the inner space of the recess 62.

The container 61 is preferably used by covering the aperture (transpiration aperture) opening at the top of the recess 62 that contains the insecticidal technical product 68 with a gas-permeable film 64 as shown in Fig. 16. This structure prevents the insecticidal technical product 68 from spilling from the container 61 and makes it easier to handle including transportation. The gas-permeable film 64 may be made of the same material as the gas-permeable film 4 described previously.

To use the container 61, it is placed on a radiation plate 67 having a heating section 68 disposed below thereof as shown in Fig. 16, so as to heat the insecticidal technical product 68 contained inside by the heated from the radiation plate 67. The insecticide component that has transpired from the insecticidal technical product 68 diffuses through the gas-permeable film 64 (transpiration of the insecticide component is indicated by arrows).

There is no limitation on the amount of the insecticidal technical product 68 contained in the container 61 which may be determined by taking into consideration the rate of transpiration of the chemical that is used, amount of transpiration per unit of time and the target duration of transpiration, and is usually from about 0.05 to 3 ml, preferably from about 0.1 to 1 ml.

Target temperature of heating the insecticidal technical product is determined by taking into consideration the amount of insecticide component transpired per unit of time, and is set so that the heater temperature (surface temperature of the radiation plate 67) is from about 50 to 170°C, preferably from 70 to 130°C. What is important is the temperature of the container 61, particularly the surface temperature of the recess 62, and the amount of insecticide component transpired varies as the surface temperature varies. The surface temperature of the recess 62 is not necessarily the same as the heater temperature and is subjected to significant fluctuation depending on the heat conduction to the flange 63 and heat dissipation into the atmosphere. An advantageous use can be made of this phenomenon by changing the depth of the recess or the size of the flange 63 so as to change the heat conduction to the flange 63 and/or heat dissipation into the atmosphere thereby to control the surface temperature of the recess 62 even when the heater temperature remains constant. Amount of transpired insecticide component can be controlled in this way.

Factors that affect the surface temperature variation of the recess 62 include the depth h of the recess 62, whether the flange 63 is provided or not and, if provided, size (surface area) of the flange 63 and, in case the rib 69 is formed in the recess 62, size (volume) of the rib 69.

Depth "h" of the recess 62 can be set within a range from about 0.3 to 5 mm, preferably from about 0.5 to 4 mm, so that surface temperature of the recess 62 becomes an optimum level. Surface temperature of the recess 62 becomes lower as the depth "h" is larger, and conversely becomes higher as the depth "h" is smaller.

Size (surface area) of the flange 63 can be set so as to obtain the optimum surface temperature of the recess 62. When the size of the flange 63 is represented in terms of the proportion to the total area (D1 × D2) in the plan view shown in Fig. 15, proportion of the flange 63 may be set in a range from 0 to about 85%, and preferably from 0 to about 70%. Proportion of the flange 63 being 0 means that there is no flange. Usually, the larger the flange 63, the lower the surface temperature of the recess 62, and the smaller the flange 63, the higher the surface temperature of the recess 62. There is not limitation on the shape of the flange 63.

Size (volume) of the rib 69 can be set so as to obtain the optimum surface temperature of the recess 62. Size of the rib 69 is preferably represented in terms of the volume. Height of the rib 69 is usually approximately equal to the depth "h" of the recess 62, but may be larger than or smaller than the depth "h". When height of the rib 69 is usually approximately equal to the depth "h" of the recess 62, proportion of the horizontal cross sectional area of the rib 69 (namely the area of the cross section that is parallel to the bottom of the recess 62) to the horizontal cross sectional area of the recess 62 may be set in a range from 0 to about 30%, and preferably from 0 to about 25%. Proportion of the rib 69 being 0 means that there is no rib. Usually, the larger the volume of the rib 69, the lower the surface temperature of the recess 62, and the smaller the volume of the rib 69, the higher the surface temperature of the recess 62. There is no limitation on the shape of the rib 69.

Temperature of the insecticidal technical product when heating the container 61 is set to an optimum temperature in a range from about 45 to 165°C, preferably from about 65 to 125°C as described above. Specifically, the heating temperature is determined by taking into consideration the effective amount of the chemical required for exterminating the vermin such as mosquitoes and the target duration of transpiration.

The surface temperature of the container 61 is changed, and accordingly the amount of evaporating insecticide component can be controlled by changing the depth h of the recess 62, size of the flange 63 and size of the rib 69. Thus the optimum amount of evaporating insecticide component can be set according to the insecticidal technical product in use, and it is made possible to provide the heat transpiration container that can be set to various duration of transpiration such as 30 days, 60 days and more by adjusting the rate of transpiration even when the heater temperature remains constant (the number of days described above are based on such a usage as the insecticidal technical product is heated for about 12 hours a day).

When heating the chemical to transpire, it is preferable to hold the container 61 and the radiation plate 67 in a tilted posture so that decrease in the amount of the insecticidal technical product 68 held in the container 61 through transpiration can be easily known from the change in the liquid level of the insecticidal technical product, namely the container serves as an indicator of the remaining amount of the insecticidal technical product. Tilting angle of the container 5 from the horizontal surface is in a range from 40 to 60°.

(IV)

The heat transpiration apparatus, the heat transpiration container and the method of producing the same according to the present invention will now be described below by way of example where the insecticidal technical product is used as the transpiring liquid. The insecticidal technical product is similar to that described previously and may include the additive described before. When a colorant such as pigment is added to the insecticidal technical product (transpiring liquid), in particular, it becomes easier to visually recognize so that the liquid level can be easily checked from a distance and it becomes easier to know when the insecticidal technical product has been used up.

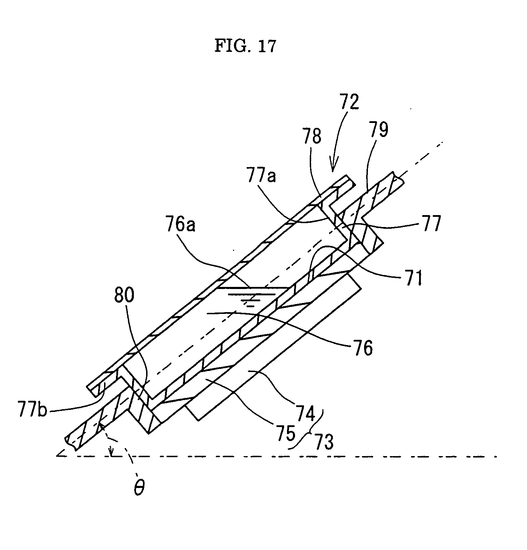

Fig. 17 is a schematic sectional view showing an embodiment of the liquid level indicating structure of the heat transpiration container according to the present invention. As shown in Fig. 17, the heat transpiration container 72 is held on a heater surface 71 that is inclined to such an angle that the transpiring liquid 76 touches the gas-permeable film 78. The heat transpiration container 72 comprises a container 77 that contains the insecticidal technical product 76 and a transparent or semi-transparent gas-permeable film 78 that closes a transpiration aperture formed at the top of the container 77.

The heater 73 has a heating element 74 and a radiation plate 75. The heater 73 is installed inside of the inclined wall 79 of the heat transpiration apparatus, and forms the bottom of the recess 80 that is formed in the wall 79. The heat transpiration container 72 is dropped into the recess 80 so that the heat transpiration container 72 is held on the heater surface 71.

Under this condition, as shown in Fig. 17, since the heat transpiration container 72 is held on the heater surface 71 in a tilted posture, the liquid level 76a of the insecticidal technical product 76 can be visually checked easily through the gas-permeable film 78. Thus the remaining amount of the insecticidal technical product 76 can be easily known.

The gas-permeable film 78 may be made of a material similar to that of the gas-permeable film 4 mentioned above.

The container body 77 comprises a heat resistant sheet made of aluminum or other metal or a heat resistant resin, that has a recess 77a formed at the center thereof, so as to contain the insecticidal technical product 76 therein, and a flange 77b is provided on the periphery of the transpiration aperture of the recess 77a. The gas-permeable film 78 is placed with the periphery thereof located on the flange 77, and bonded together by thermal fusion or the like.

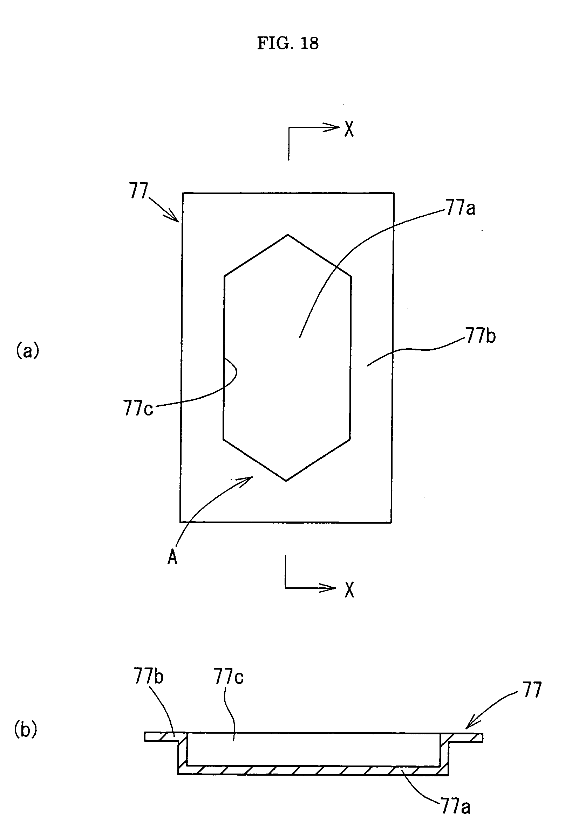

Fig. 18(a) and (b) are a plan view of the container body 77 and a sectional view thereof along line X-X, respectively. The recess 77a of the container body 77 has a transpiration aperture of hexagonal shape. When one end A of the transpiration aperture 77c shown in Fig. 18(a) corresponds to the lower end of the container body 77, for example, the lower end portion becomes narrower toward the end. Therefore, as the remaining liquid dwindles, lowering speed of the liquid surface 76a of the insecticidal technical product 76 increases and it becomes easier to know the end of the heat transpiration. In the case of the transpiration aperture 77c shown in Fig. 18(a), it is also made easier to check the liquid amount at the start of use since the upper end becomes narrower toward the tip, too.

Tilting angle θ of the heater surface 71 from the horizontal plane as shown in Fig. 17 is in a range from 10 to 90°, preferably from 40 to 60°. When the tilting angle θ is smaller than the range described above, it becomes difficult to check the liquid level of the insecticidal technical product 76.

There is no limitation on the amount of the insecticidal technical product 76 contained in the heat transpiration container 72 which may be determined by taking into consideration the transpiration performance of the chemical that is used, amount of transpiration per unit of time and the target duration of transpiration, and is usually from about 0.05 to 3 ml, preferably from about 0.1 to 1 ml. Depth of the recess 77a is preferably determined within a range from 0.5 to 5 mm depending on the kind of insecticidal technical product 5 contained therein. Amount of the insecticidal technical product is from about 75 to 4500 mg in weight, preferably from 150 to 1500 mg.

Target temperature of heating the insecticidal technical product is determined by taking into consideration the amount of insecticide component transpired per unit of time, and is set so that the heater temperature (surface temperature of the heater 71) is from about 50 to 170°C, preferably from 70 to 130°C.

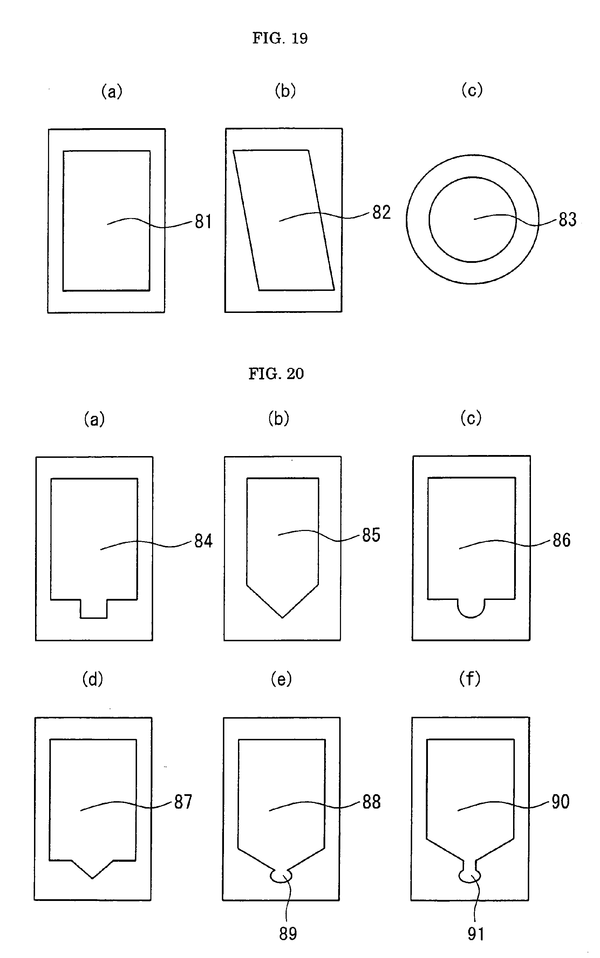

The transpiration aperture of the container body according to the present invention preferably has such a shape that allows easy recognition of the changing liquid amount of the transpiring liquid 76, enables large transpiration area and can be formed easily. Fig. 19 to Fig. 25 shows various shapes of the transpiration aperture other than that of the transpiration aperture 77c shown in Fig. 18. Any of these transpiration apertures can be used in the present invention. In each case shown in the drawing, lower end of the transpiration aperture corresponds to the lower end of the tilted container.

Transpiration apertures 81, 82, 83 having rectangular or circular shape shown in Fig. 19(a) to (c) are examples in which the transpiration area is made large.

Transpiration apertures 84, 85, 86, 87 shown in Fig. 20(a) to (d) have the advantage that it is easier to know the end of the heat transpiration because the transpiration area is large and the width becomes smaller toward the lower end. Transpiration apertures 88, 90 shown in Fig. 20(e), (f) have liquid reservoirs 89, 91 formed at the lower end so as to indicate the end of the transpiring liquid more clearly.

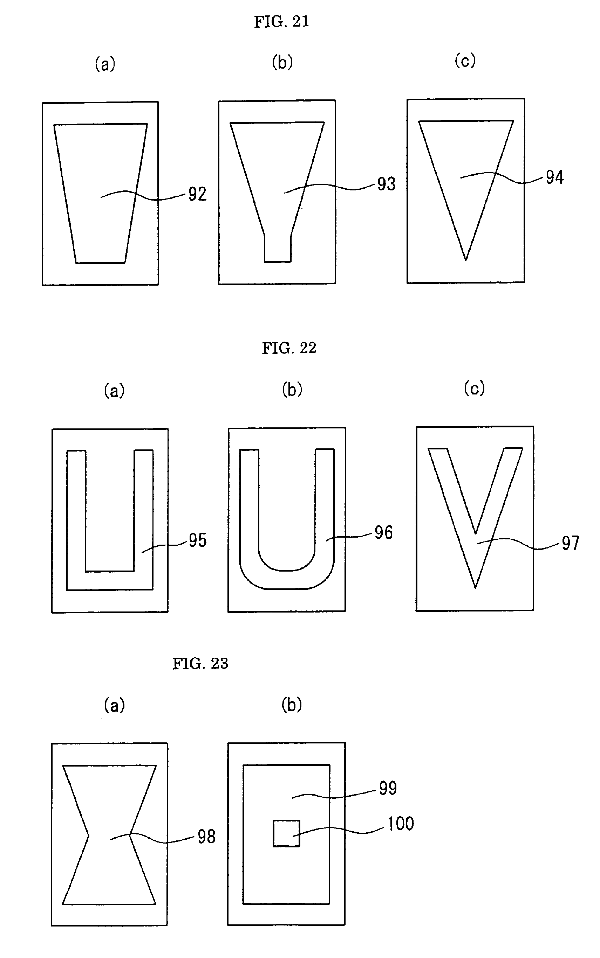

Transpiration aperture 92 shown in Fig. 21(a) has a large transpiration area and a width that gradually decreases from the upper end toward the lower end, so that the lowering speed of the liquid level increases with time. Transpiration apertures 93, 94 shown in Fig. 21(b) and (c) have the same feature as that of transpiration aperture 92 shown in Fig. 21(a) and have shapes that make it easier to know the decreasing amount near the end.

Transpiration apertures 95, 96, 97 shown in Fig. 22(a) to (c) have U-shape or V-shape being bifurcated toward the top, which makes it easier to check the decrease in liquid amount from the start of use. The transpiration aperture 97 shown in Fig. 22(c) has a shape that makes it particularly easier to check the decrease in liquid amount.

Transpiration aperture 98 shown in Fig. 23(a) has such a shape as the width is smaller at the middle than the upper end and the lower end, which makes it easier to check the decrease in liquid amount in the middle of the period of use. Transpiration aperture 99 shown in Fig. 23(b) has such a shape as a rib 100 (projection) is formed and the width is smaller at the middle, thus making it easier to check the decrease in liquid amount in the middle of the period of use and strength of the container can be increased by bonding a gas-permeable film on top of the rib 100 and sealing.

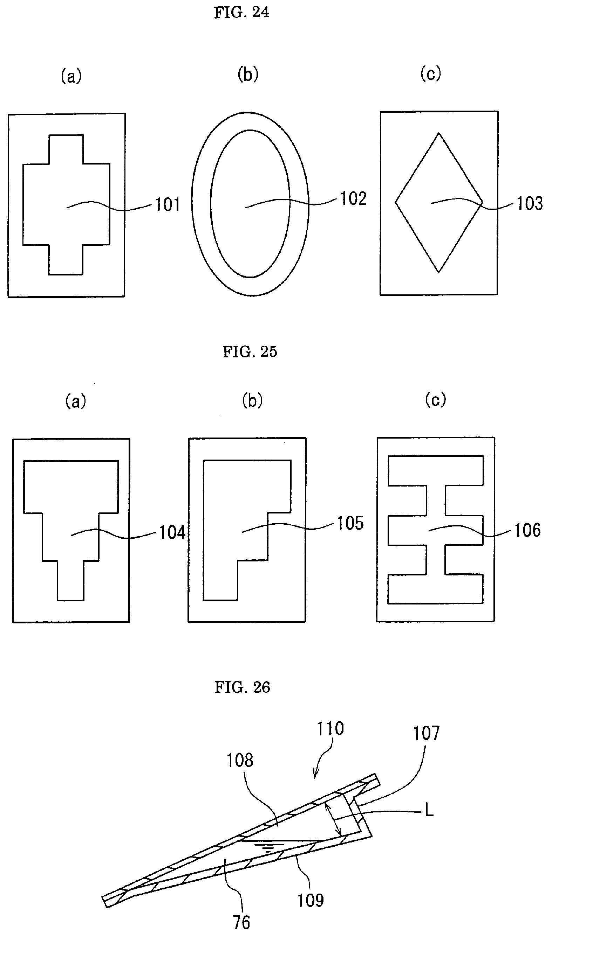

Transpiration aperture 101 shown in Fig. 24(a) has such a shape as the width is larger at the middle than the upper end and the lower end, which makes it easier to check the decrease in liquid amount in the early and latter stages of the period of use. Transpiration apertures 102, 103 shown in Fig. 24(b) and (c) also have such a shape as the width is larger at the middle than the upper end and the lower end, thus making it easier to check the decrease in liquid amount in the early and latter stages of the period of use.

Transpiration apertures 104, 105 shown in Fig. 25(a) and (b) have such a shape as the width decreases stepwise from the top toward the bottom so that it is easier to see whether the liquid amount is in the early, intermediate or latter stage of use from the liquid level. Transpiration aperture 106 shown in Fig. 25(c) has wider portions in the upper end, center and the lower end, so that it is easier to see whether the liquid amount is in the early, intermediate or latter stage of use from the liquid level.

Amount of the remaining transpiring liquid in the latter stage of transpiration can be made easier to check, also by decreasing the depth L of the container 54 at the lower end as shown in Fig. 26, instead of forming the transpiration aperture in a particular shape. For example, a recess 107 shown in Fig. 26 has inclined bottom surface with the depth L decreasing from the upper end toward the lower end, so that the decreasing rate of liquid level increases as the transpiration proceeds and the state of decreasing becomes easier to recognize. The depth L may be decreased stepwise or made smaller only at the lower end. While the shape of a transpiration aperture 108 of the container 110 shown in Fig. 26 is not specified, width of the transpiration aperture may be made smaller at the lower end by tapering toward the lower end or by providing liquid reservoir 61, 62 as shown in Fig. 18(a), Fig. 20(a) to (f), Fig. 21(a) to (c), Fig. 22(c), Fig. 24(a), (c) and Fig. 25(a), (b), thus making it easier to check the decrease in liquid amount in the latter stages of the period of use because of the narrower width as well as the small depth L at the lower end.

When the liquid reservoirs 89, 91 are provided in the lower portion of the recesses 88, 90 as shown in Fig. 20(e) and (f), depth of only the liquid reservoirs 89, 91 may be made smaller.

While the heat transpiration container 72 is held in the recess of the wall 79 formed around the heater surface 71 in the embodiment shown in Fig. 17, the holding means may be anything that can place or hold the heat transpiration container 72 on the heater surface 71 and, for example, a stopper such as a projection may be provided on the inclined heater surface or at an end of the heater surface so as to place or hold the heat transpiration container.

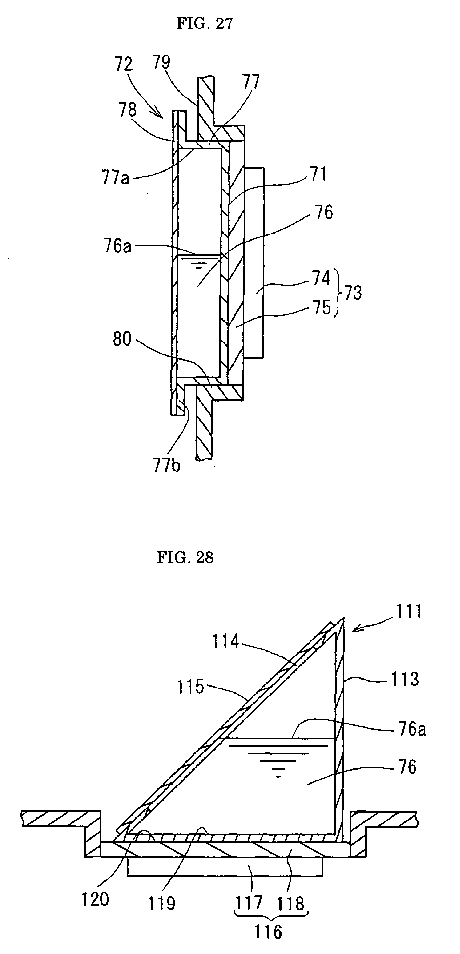

Fig. 27 shows an example of holding the heat transpiration container 72 on the heater surface 71 that is erected vertically. This form also makes it possible to check the liquid level 76a of the insecticidal technical product 76 through the gas-permeable film 78 so as to easily know the remaining amount of the insecticidal technical product 76 in the heat transpiration container 72. With other respect, this embodiment is the same as that shown in Fig. 17, and description will be omitted with the identical reference numerals assigned to the corresponding components.

Another embodiment of the present invention is shown in Fig. 28. The heat transpiration container 111 used in this embodiment comprises a container body 113 that contains the insecticidal technical product 76 (transpiring liquid) and a transparent or semi-transparent gas-permeable film 115 that closes an transpiration aperture 114 of the container body 113, where the transpiration aperture 114 is inclined with respect to the bottom 119.

The heater 116 has a heating element 117 and a radiation plate 118, and the heater surface 120 is substantially horizontal. When the heat transpiration container 111 is placed on the heater surface 120, the transpiration aperture 114 is inclined so that the transpiring liquid 76 touches the gas-permeable film 115, and therefore liquid surface 76a of the insecticidal technical product 76 can be easily observed visually through the gas-permeable film 111 located in front of it. Thus the remaining amount of the insecticidal technical product 76 in the heat transpiration container 111 can be easily known.

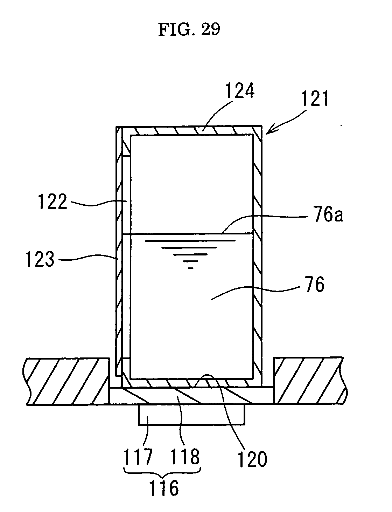

Fig. 29 shows further another embodiment of the present invention. The heat transpiration container 121 used in this embodiment comprises a container body 124 of box shape that contains the insecticidal technical product 76 and has an transpiration aperture 122 provided in the front surface, the transpiration aperture 122 being closed with a gas-permeable film 123. When the heat transpiration container 121 is placed on the heater surface 120 that is substantially horizontal, the transpiration aperture 122 is erected almost vertically so that the transpiring liquid 76 touches the gas-permeable film 123. As a result, liquid surface 76a of the insecticidal technical product 76 can be easily observed visually through the gas-permeable film 123 located in front of it, thus making it possible to easily know the remaining amount of the insecticidal technical product 76 in the heat transpiration container 121. With other respect, this embodiment is the same as that shown in Fig. 28, and description will be omitted with the identical reference numerals assigned to the corresponding components.

(V)

The heat transpiration apparatus, the heat transpiration container and the method of producing the same according to the present invention will now be described below by way of example where the insecticidal technical product is used as the transpiring liquid. The insecticidal technical product is similar to that described previously and may include the additives. When a colorant such as pigment is added to the insecticidal technical product (transpiring liquid), in particular, it becomes easier to visually recognize so that the liquid level can be easily checked from a distance and it becomes easier to know when the insecticidal technical product has been used up.

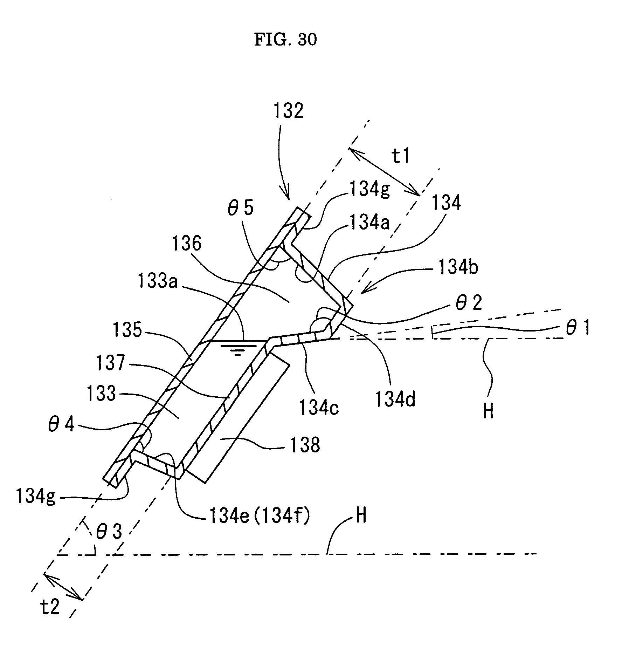

Fig. 30 is a schematic sectional view showing an embodiment of the heat transpiration container and the heat transpiration apparatus using the same according to the present invention. As shown in Fig. 30, the heat transpiration container 132 comprises a container body 134 that opens at the top and contains insecticidal technical product (transpiring liquid) 133 therein and a transparent or semi-transparent gas-permeable film 135 that closes the opening of the container body 134, wherein an extended portion 134b is formed at one end of the container body 134 to protrude in the direction of depth of the container body 134, so as to form an air reservoir 136.

Ratio of the capacity (air volume) of the air reservoir 135 to the volume (liquid volume) of the insecticidal technical product 133 in the container body 134 before starting the transpiration is about 1: 0.5 to 10, preferably about 1: 1 to 7 and more preferably 1: 2 to 5.

Amount of the insecticidal technical product 133 contained in the heat transpiration container 132 may be similar to that of the insecticidal technical product 3 in the container 5 described previously.

There are no limitations on the depth of the bottom from the opening in the section where the extended portion 134b is formed, the depth of the bottom from the opening in the section where the extended portion 134b is not formed, size of the bottom surface of the extended portion 134b and the shape of the extended portion 134b, which may be determined by considering such factors as the material cost, acceptable size, ease of molding and the ratio of the capacity (air volume) of the air reservoir 135 to the volume of the insecticidal technical product 133. Specifically, depth "t1" of the bottom from the opening in the section where the extended portion 134b is formed is preferably from about 1 to 10 mm, and depth "t2" of the bottom from the opening in the section where the extended portion 134b is not formed is preferably from about 0.5 to 5 mm.

The heat transpiration apparatus of the present invention comprises the heat transpiration container 132 held in tilted posture on the heater surface 11 of the heater 13 with the extended portion 134b located at the top so that the insecticidal technical product 133 touches the gas-permeable film 135. When placed in such a tilted posture, a lower surface 134c of the extended portion 134b is inclined so that the insecticidal technical product 133 flows from within the extended portion 134b to the lower end in the container body 134. The angle θ1 which the lower surface 134c forms with the horizontal plane H is larger than 0° and less than 90°, and preferably from 2 to 45°. The angle θ2 between the lower surface 134c of the extended portion 134b forms and the bottom face 134d of the extended portion 134b is preferably larger than 90° and less than 180°. With this configuration, it becomes easier to flow the insecticidal technical product 133 from of the extended portion 134b downward when placed on the heater 138.

The inclination angle θ3 of the heat transpiration container 132 from the horizontal plane H is from 10° to 90°, and preferably from 40 to 60°. When the inclination angle θ3 is in this range, liquid surface 133a of the insecticidal technical product 133 can be easily observed visually through the gas-permeable film 135, and it becomes easy to know the remaining amount of the insecticidal technical product 133 in the heat transpiration container 132. When the inclination angle θ3 is less than this range, it becomes difficult to know the remaining amount of the insecticidal technical product 133.

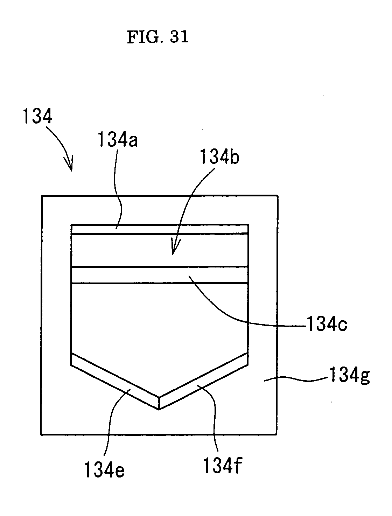

Fig. 31 is a plan view of the container body 134. As shown in Fig. 30 and Fig. 31, aperture of the container body 134 becomes narrower toward the lower end when the container body 134 is tilted. This shape of narrowing toward the lower end is formed by lower end faces 134e and 134f of the container body 134. Since the container body 134 having the shape of narrowing toward the lower end is held on the heater surface 137 as shown in Fig. 30, lowering speed of the liquid surface 16a of the insecticidal technical product 133 increases as the remaining liquid diminishes, making it more easier to know the end of thermal transpiration. The angle θ4 formed by the lower end face 134e (or the lower end face 134f) and the plane including the flange 134g is preferably in a range from 70° to 90°. This design minimizes the amount of liquid that remains stuck on the inner edge of the aperture of the container body 134.

The angle θ5 formed by the upper end face 134a of the container body 134 and the plane including the flange 134g is preferably in a range from 70° to 90°. This design minimizes the amount of liquid that remains stuck on the inner edge of the aperture of the container body 134.

Heating temperature of the insecticidal technical product 133, temperature of the insecticidal technical product 133 and the surface temperature of the gas-permeable film 135 may be similar to those described previously.

The gas-permeable film 135 is transparent or semi-transparent, which makes it easier to visually check the amount of the remaining insecticidal technical product 133 and recognize the end. The gas-permeable film 135 does not allow the liquid insecticidal technical product 133 to permeate, but allows the gaseous component transpired from the insecticidal technical product 133 to permeate. A material similar to that of the gas-permeable film 4 described previously may be used for the gas-permeable film 135.

The container body 134 is made of a heat resistant material such as aluminum or other metal or a heat resistant resin that is molded by a known method. After filling the container body 134 with a predetermined amount of insecticidal technical product 133 so as to provide a liquid layer and the air reservoir 136, the gas-permeable film 135 is placed so that the periphery thereof is located on the flange 134g and bonded by thermal fusing or the like. Filling with the insecticidal technical product 133 may be carried out by injecting the insecticidal technical product 133 into the extended portion 134b. Since the extended portion 134b is deep from the opening to the bottom, the insecticidal technical product 133 can be easily injected and is less likely to spill.

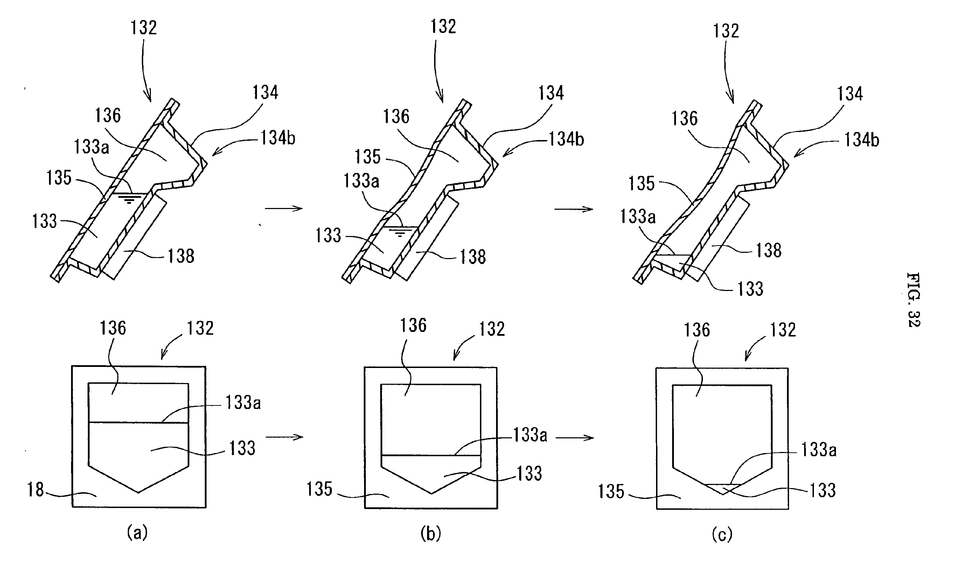

The heater 138 heats the heat transpiration container 132 particularly on portions other than the extended portion 134b. The insecticidal technical product 133 is transpired by heating the heat transpiration container 132 with the heater 138. Fig. 32(a) to (c) are explanatory diagrams showing the process of the insecticidal technical product 133 in the heat transpiration container 132 being transpired. Fig. 32(a) shows the early stage of transpiration, Fig. 32 (b) shows the intermediate stage of transpiration and Fig. 32(c) shows the latter stage of transpiration, sectional view at the top and plan view at the bottom. The heat transpiration container 132 of the present invention can prevent the film 135 from touching the bottom surface of the container body 134 from the early stage till the latter stage of transpiration, and allows it to visually know the amount of remaining insecticidal technical product 133 precisely because the film 135 is transparent or semi-transparent.

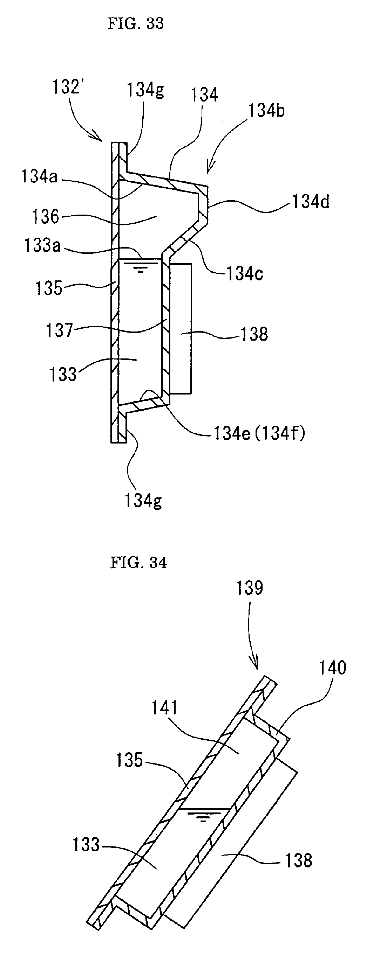

Fig. 33 shows an example in which the heat transpiration container 132' is held on the heater surface 11 that is erected vertically. This form also makes it possible to prevent the film 135 from touching the bottom surface of the container body 134 as the amount of the insecticidal technical product 133 decreases, since the heat transpiration container 132' has the insecticidal technical product 133 and the air reservoir 136 therein, similarly to the case of tilted mounting described above. With other respect, this embodiment is the same as that shown in Fig. 30, and description will be omitted with the identical reference numerals assigned to the corresponding components.

While the heater 138 heats the heat transpiration container 132 particularly on portions other than the extended portion 134b in the embodiment described above, a heater may be provided also at the position of the extended portion 134b so as to heat the entire bottom surface of the heat transpiration container 132, 22. When the angle θ1 between the lower surface 134c and the horizontal plane H is smaller than 0°, that is, when the insecticidal technical product 133 cannot smoothly flow from within the extended portion 134b to the lower end in the container body 134, the heater 138 may be extended into the extended portion 134b in order to heat the extended portion 134b, too, so that the remaining insecticidal technical product 133 can be heated and transpired even when the insecticidal technical product 133 does not flow down from the extended portion 134b and remains in the extended portion 134b.

While the extended portion 134b protrudes from the bottom surface of the container body 134, it may protrude from one or both of the side faces in the direction of width. In case the extended portion protrudes in the direction of width, the angle between the lower end surface of the extended portion and the bottom surface of the extended portion (side face of the container body in the extended portion) is preferably larger than 90° and less than 180°.

While the heat transpiration container has the extended portion in the embodiment described above, the heat transpiration container of the present invention may have such a construction without extended portion as shown in Fig. 34. Fig. 34 is a schematic sectional view showing the heat transpiration container 139 of the present invention of which container body 140 does not include an extended portion being held in an inclined posture. The heat transpiration container of the present invention prevent the film 135 from touching the bottom surface of the container body 140 as the amount of the insecticidal technical product 133 decreases, despite the absence of the extended portion since the container has the air reservoir 141 therein as shown in Fig. 34. With other respect, this embodiment is the same as that shown in Fig. 30, and description will be omitted with the identical reference numerals assigned to the corresponding components.

The insecticidal technical product 133 may also be heated, instead of having the heat transpiration container 134 held directly on the heater surface 137 as in the embodiment described above, also by providing a gap or other intermediate object between the heat transpiration container 134 and the heater surface 137. Further, the surface of the film 135 can be heated on the film 135 side by using the heater 138 etc.

While the embodiment described above deals with a case where the aperture of the container body has a shape of pentagon (Fig. 31), there is no limitation on the aperture shape which may be triangle, rectangle, hexagon, circle, oval or other shape.

The heat transpiration container of the present invention and the heat transpiration apparatus that employs the same can be used to exterminate vermin similarly to those described previously.

While the foregoing description has dealt with cases where the transpiring liquid is heated to transpire, the transpiring liquid may be exposed to open air so as to spontaneously transpire at the normal temperature in case the transpiring liquid is volatile at normal temperature.

(VI)

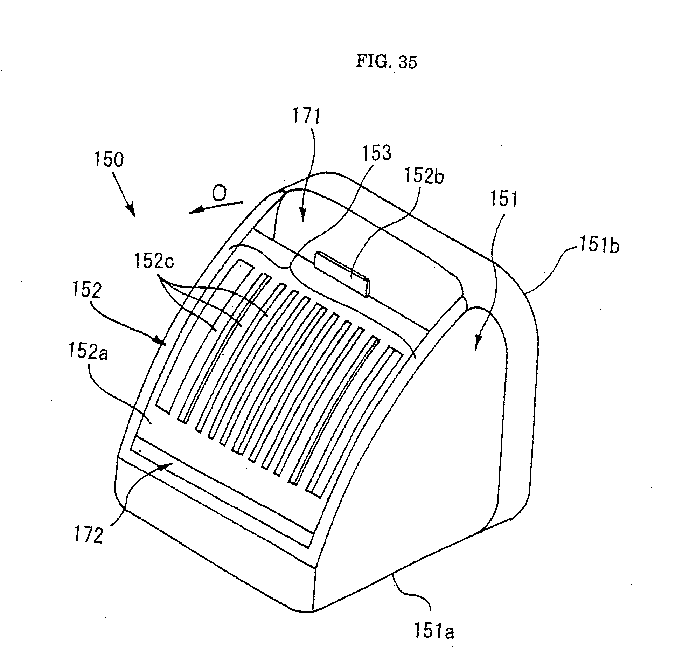

Fig. 35 is an overall perspective view showing a first embodiment of the heat transpiration apparatus according to the present invention. The heat transpiration apparatus 150 of this embodiment has an instrument body 151 and a lid 152 provided so as to close or open the instrument body 151. The lid 152 has a lid surface 152a that is inclined (for example, from 10° to 60°) with respect to the horizontal plane (direction parallel to the ground surface) when closed on the instrument body 151, the lid surface 152a having a plurality of apertures 153. The plurality of apertures 153 are formed in elongated shape to extend vertically. The plurality of apertures 153 are divided by pillars 152c of the lid 152 in the longitudinal direction.

The lid 152 has a thumb tab 152b formed at the upper end. The user can put a finger on the thumb tab 152b and apply a force as indicated by an arrow in the drawing, so as to swing open the lid 152 around an axis located near the lower end thereof.

In the heat transpiration apparatus 150 of this embodiment, proportion of the area occupied by the plurality of apertures 153 to the area of the lid surface 152a is 40% or higher (45% in this embodiment) to the extent that is practical. Proportion of the apertures refers to the ratio of the area occupied by the plurality of apertures 153 to the area of the lid surface 152a.

When the plurality of apertures 153 are formed with the proportion of area less than 40%, transpiration becomes insufficient due to sticking of the transpired chemical in the gap of the aperture 153 to remain therein and the air flow being hampered.

By forming the plurality of apertures 153 with proportion of area not less than 40%, such a trouble can be avoided as transpiration becomes insufficient due to sticking of the transpired chemical in the gap of the aperture 153 to remain therein and the air flow being hampered.

The plurality of apertures 153 are formed, in addition to the proportion of area being 40% or higher, such that each aperture 153 has a dimension of 5 mm (2.5 mm in this embodiment) or less along the shorter side. This construction prevents a foreign matter from passing through the aperture 153 and touching the heating section or the transpiring liquid holding body which are not shown in Fig. 35. Since the lid 152 has such a construction that can be opened and closed, a foreign matter that has stuck in the aperture can be prevented from touching the heating section by opening the lid 152.

Thus the present invention provides the heat transpiration apparatus that can be conveniently used while maintaining the transpiration efficiency of chemical at a satisfactory level.

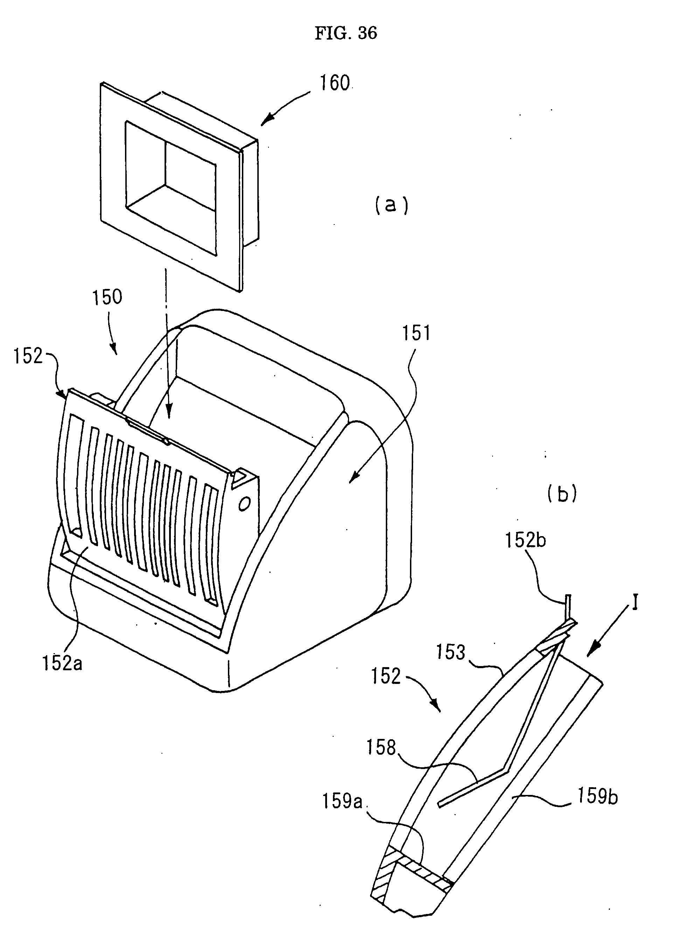

Fig. 36(a) is an overall perspective view showing the heat transpiration apparatus 150 with the lid 152 opened. Fig. 36(b) is a sectional view of the lid 152 in the longitudinal direction (vertical direction in the drawing).

As shown in Fig. 36(a), the lid 152 is opened and a transpiring liquid holding body 160 is set on the inner surface of the lid 152 (surface opposite to the lid surface 152a). A heating section to be described later is not shown in this drawing.

As shown in Fig. 36(b), the lid 152 has a lead spring 158 as a retainer therein. The leaf spring 158 that is bent like a dog leg is supported at the top of the lid 152 in the form of cantilever and extends downward in a direction to approach the instrument body 151 (the direction departing from the lid 152), while being bent in the middle so that the distal portion is inclined to approach the lid 152.

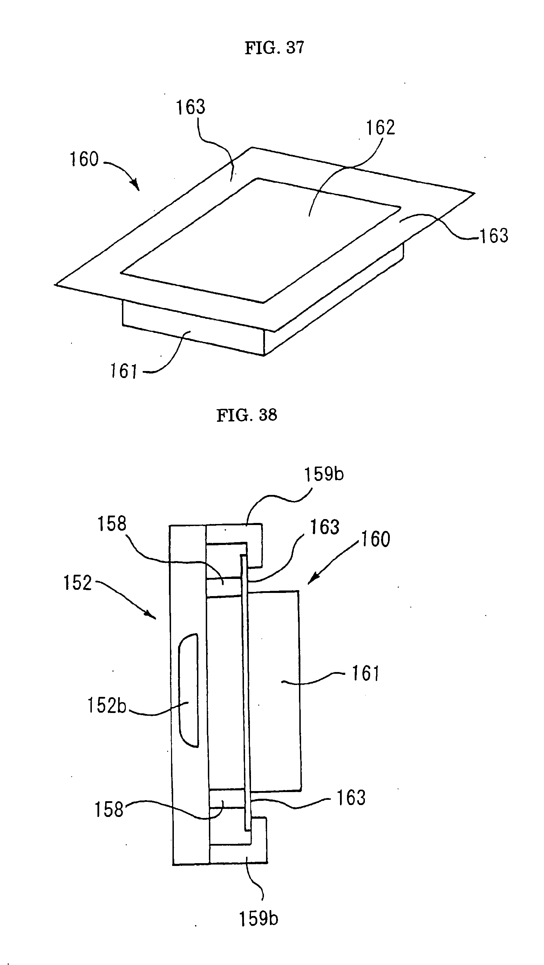

Fig. 37 shows an example of the transpiring liquid holding body 160. The transpiring liquid holding body 160 has such a constitution as the gas-permeable film 162 covers an upper aperture of a container 161 that has substantially rectangular parallelepiped containing the transpiring liquid, and a flange-like side rim 163 is provided along the upper periphery of the container 161. The container 161 made be, for example, a metal. The transpiring liquid holding body 160 is free of loading that blocks the liquid flow since the container is heated on the bottom thereof to cause the chemical to transpire through the gas-permeable film 162.

Fig. 38 shows the transpiring liquid holding body 160 attached to the lid 152, viewed in the direction of arrow I in Fig. 36(b). The lid 152 has L-shaped guides 159b, 159b provided at the edges on both sides of the lid 152. The guide 159b extends substantially in parallel to the lid surface 152a. The leaf springs 158 are provided in a pair, disposed on the inner surface of the lid 152 with a space kept from each other in the direction of width, each of the leaf springs 158 being located near the guide 159b.

With the lid 152 opened, the side edge 163 of the transpiring liquid holding body 160 is inserted between the leaf spring 158 and the guide 159b. The transpiring liquid holding body 159 is inserted till the distal end of the transpiring liquid holding body 160 makes contact with a rib stopper 159a shown in Fig. 36(b). Thus the side edge 163 of the transpiring liquid holding body 160 is interposed between the bent portion of the leaf spring 158 and the guide 159b. The side edge may also be held by another retainer (for example, a small piece) that does not pose obstruction to the use, instead of the leaf spring.

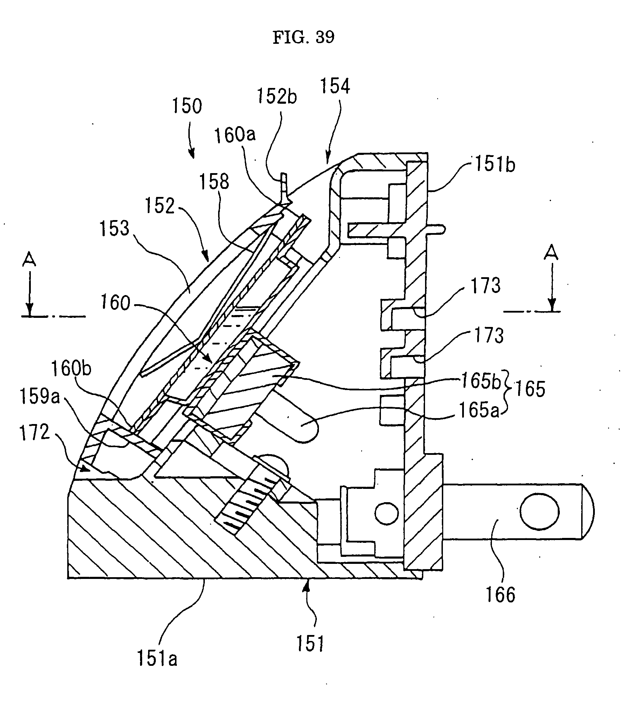

Fig. 39 is an overall sectional view showing the heat transpiration apparatus 150 with the transpiring liquid holding body 160 mounted thereon and the lid 152 closed.

As shown in Fig. 39, the portion of the leaf spring 158 near the bending presses the inclined transpiring liquid holding body 160 against the heating section 165. When the lid 152 is closed, the transpiring liquid holding body 160 is pressed against the heating section 165 so that the leaf spring 158 undergoes greater elastic deformation than before closing the lid 152. As a result, the leaf spring 158 firmly holds the transpiring liquid holding body 160 with a strong elastic force. The transpiring liquid holding body 160 is held in the state of inclined by an angle of, for example, from 10° to 90° with respect to the horizontal plane.

When supplied with electric power through terminals 165a, heat of the heating section 165 is transmitted to the heating element 165b so as to heat the transpiring liquid holding body 160 over a certain area of the back surface thereof. Then the chemical such as insecticide transpires through the gas-permeable film in front of the transpiring liquid holding body 160 and is released to the outside through the apertures 153 of the lid 152.

A power plug 166 is provided on a back section 151b that is erected vertically on the bottom 151a of the instrument body 151. The plug 166 may also be provided so as to protrude from the bottom 151a of the instrument body 151. The plug 166 may also be provided movably on the instrument body 151 so as to be able to assume either of the state of protruding from the back section 151b or the state of protruding from the bottom 151a. By providing the movable plug 166, it is made possible to set the heat transpiration apparatus either on a wall receptacle or a table-top receptacle unit having a power outlet in the top surface thereof.

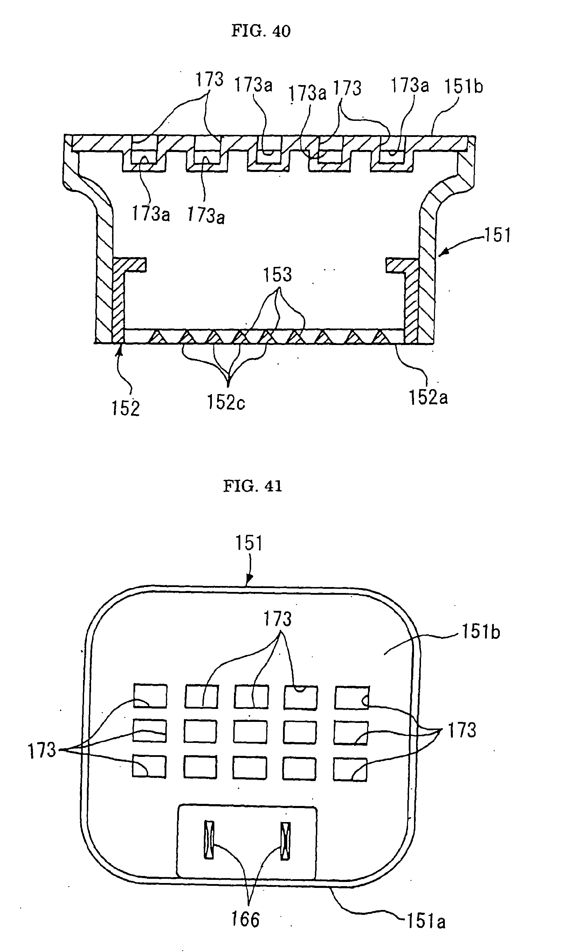

Fig. 40 is a sectional view of the heat transpiration apparatus 150 viewed in the direction of arrow A of Fig. 39. The leaf spring 158, the transpiring liquid holding body 160 and the heating section 165 are not shown in Fig. 40.

As shown in Fig. 40, the pillars 152c of the lid 152 of triangular cross section are provided with the base of the triangle being located on the side of the lid surface 152a. However, cross section of the pillars 152c of the lid 152 is not limited to triangular, and may be trapezoidal or other desired shape. Base of the triangle may also not be placed on the lid surface 152a side, and may be located on the back of the lid.

In the heat transpiration apparatus 150 of the present invention, transpiration aperture 154 is formed between the instrument body 151 and the upper end of the lid 152 (upper portion of the lid 152 when viewed from the left in Fig. 39). The transpiration aperture 154 opens vertically upward at the upper end 20a of the transpiring liquid holding body 160. The transpiration aperture 154 may be provided on the instrument body 151 instead of in the gap with the instrument body 151. That is, in the heat transpiration apparatus 150 of the present invention, when the lid 152 is closed on the instrument body 151, the transpiration aperture 153 may be formed between the instrument body 151 and the upper end of the lid 152, and/or in the instrument body 151

The transpired chemical tends to deposit particularly heavily on a portion of the instrument body located above the transpiring liquid holding body 160 in the heat transpiration apparatus 150, and the transpired chemical that has deposited tends to decrease the rate of transpiration by blocking the aperture.

When the transpiration aperture 154 is provided to open vertically upward at the upper end 160a of the transpiring liquid holding body 160, the transpired chemical diffuses to the outside of the heat transpiration apparatus 150 through the transpiration aperture 154. Thus decrease in the transpiration efficiency of chemical due to deposition of the transpired chemical on the inside of the heat transpiration apparatus can be suppressed.

In the heat transpiration apparatus 150 of the present invention, a first air intake port 172 is formed in the gap between the instrument body 151 and the lower end of the lid 152 (lower end portion of the lid 152 when viewed from the left in Fig. 39). The first air intake port 172 may also be provided in the instrument body, instead of the gap between the instrument body 151 and the lid 152. That is, in the heat transpiration apparatus of the present invention, the first air intake port 172 may be formed in the gap between the instrument body 151 and the lower end of the lid 11, and/or in the instrument body 151, under the condition that the lid 152 is closed on the instrument body 151.

In this embodiment, the first air intake port 172 is provided so as to open at a position lower than the lower end 160b of the transpiring liquid holding body 160 in the vertical direction.

Air outside of the heat transpiration apparatus 150 is introduced through the first air intake port 172 into the instrument body 151. Since the transpiration of the chemical is accelerated by the introduced air, the transpiration efficiency of chemical is improved.

The gap between the instrument body 151 and the lower end of the lid 152 is preferably set in a range from 1 mm to 8 mm.

The heat transpiration apparatus 150 of the present invention has a plurality of second air intake ports 173 provided on a back section 151b that is erected vertically on the bottom 151a of the instrument body 151.

Fig. 41 shows the heat transpiration apparatus of Fig. 39 viewed from the right in the drawing (front view of the back section 11b).

As shown in Fig. 39 to Fig. 41, the plurality of second air intake ports 173 are provided on the back section 151b, three in the longitudinal direction (vertical direction in Fig. 41) and five in lateral direction (from left to right in the drawing). Number and arrangement of the second air intake ports 173 are not limited to this constitution. The plurality of second air intake ports 173 may have the same or different sizes and may be disposed randomly on the back section instead of aligned arrangement.

Each of the second air intake ports 173 is formed as a recess, and holes 173a are formed on the lower side face that delimits the recess so as to communicate with the inside of the instrument body 151. Each of the second air intake ports 173 is formed so that a pointed foreign matter which is not shown in the drawing put into the second air intake ports 173 from the outside of the instrument body 151 is stopped by the bottom of the recess. With this construction, the heat transpiration apparatus 150 can prevent a foreign matter that accidentally enters the instrument body 151 from damaging the heating section 165 or the like by means of the second air intake ports 173.

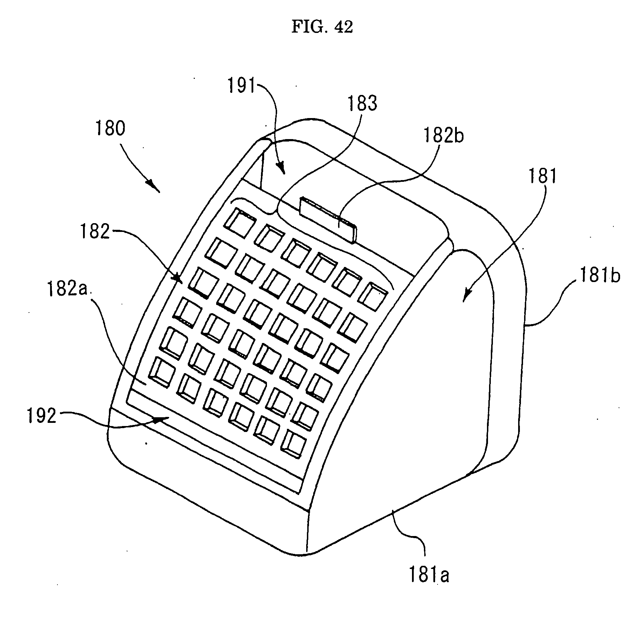

Fig. 42 is an overall perspective view showing a second embodiment of the heat transpiration apparatus according to the present invention. In the description described below, members having similar construction and function as those of the members described above will be assigned with the identical or corresponding reference numerals and description will be simplified or omitted.

As shown in Fig. 42, heat transpiration apparatus 1220 of this embodiment has a plurality of square or rectangular apertures 183 divided in substantially grating pattern formed in the lid 182.

In the heat transpiration apparatus 1220 of this embodiment, proportion of the area of the plurality of square or rectangular apertures 183 to the area of the lid surface 182a of the lid 182 is set to 40% or higher (45% in this embodiment).

When the proportion of the plurality of apertures is set less than 40%, loading occurs in which the transpired chemical deposits and remains in the gap of the apertures 183, resulting in a decrease in the transpiration efficiency of chemical.

In this embodiment, by setting the proportion of the area of the plurality of apertures 183 to 40% or higher, such a situation is prevented as loading occurs in which the transpired chemical deposits and remains in the gap of the apertures 183.

In addition to setting the proportion of the area of the plurality of apertures 183 to 40% or higher, maximum dimension of the apertures 183 is set to 5 mm or less (2.5 mm in this embodiment).

The maximum dimension refers to the length of the largest portion of each aperture 183. In the case of the plurality of apertures 183 shown in Fig. 42, since diagonal line is the maximum dimension among the dimensions of the apertures 183, length of the diagonal line is set to 5 mm or less. Thus such a trouble can be prevented from occurring as a foreign matter enters through the aperture 183 to touch the heating section or the transpiring liquid holding body which are not shown in Fig. 35. Since the lid 182 can be opened and closed, even when a foreign matter is stuck and won't come out, the foreign matter can be prevented from touching the heating section by opening the lid 182. The lid 182 may also have such a construction that can not only be opened and closed but also be detached and reattached. The lid 182 may also have such a construction that can not be opened and closed, but can be simply detached and reattached.

Thus the present invention provides the heat transpiration apparatus that can be conveniently used while maintaining the transpiration efficiency of chemical at a satisfactory level.

In this embodiment, transpiration aperture 191 may be formed either between the instrument body 181 and the upper end of the lid 182, and/or in the instrument body 181.

Also first air intake port 192 may be formed either in the gap between the instrument body 181 and the upper end of the lid 182, and/or in the instrument body 181.

Moreover, although not shown in Fig. 42, such a constitution may also be employed as second air intake port is formed in the back section 181b of the instrument body 181. Construction, function and effect of the transpiration aperture 191, the first air intake port 192 and the second air intake port are the same as those described in conjunction with the first embodiment.

In this embodiment, the aperture 183 is not limited to square or rectangular shape. The aperture 183 may be, for example, a polygon such as triangle or pentagon, circle or oval.

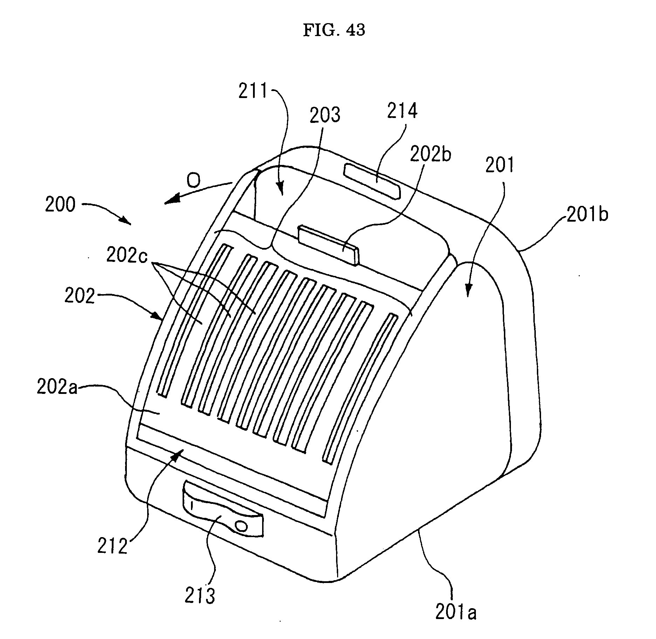

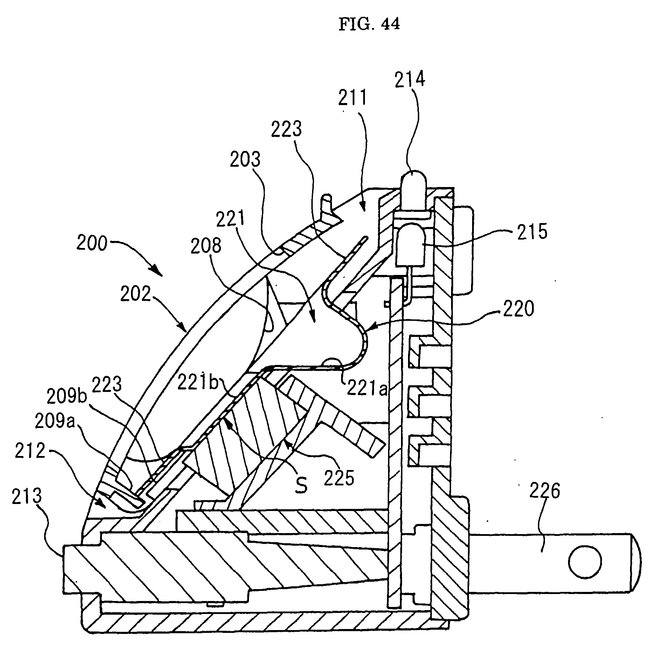

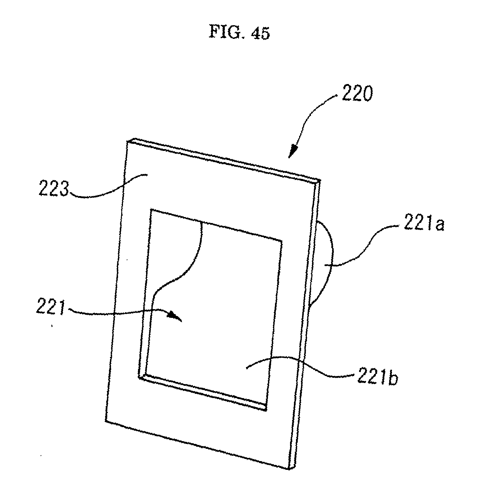

Fig. 43 is an overall perspective view showing a third embodiment of the heat transpiration apparatus according to the present invention. Fig. 44 is a sectional view of heat transpiration apparatus 200 of Fig. 43 in longitudinal direction (vertical direction in the drawing). Fig. 45 shows the transpiring liquid holding body 220 of Fig. 44. In the embodiment described below, members having similar construction and function as those of the members described above will be assigned with the identical or corresponding reference numerals and description will be simplified or omitted.

As shown in Fig. 43, the heat transpiration apparatus 200 of this embodiment has a plurality of apertures 203 formed in the lid 202 in elongated shape to extend vertically. The plurality of apertures 203 have substantially the same shape and size.

Proportion of the area of the plurality of apertures 203 to the area of the lid surface 72a of the lid 202 is set to 40% or higher (45% in this embodiment). Length of the apertures 203 along the shorter side is set to 5 mm or less (2.5 mm in this embodiment).

As shown in Fig. 43, 44, the heat transpiration apparatus 200 has a power switch 213 which is provided below the first air intake port 212 of the instrument body 201. The power switch 213 is constituted so as to be switched between ON position where power is supplied to the heating section and OFF position where power is not supplied. Electric power is supplied to the heating section 225 when the user places the power switch 213 in ON position.

The heat transpiration apparatus 200 has a display section 214 above the transpiration aperture 211 of the instrument body 201. The display section 214 is made of a transparent or semi-transparent resin or the like. A light emitter 215 is provided below the display section 214 in the instrument body 201, the light emitter 215 emitting light when the power switch 213 is placed in ON position. This causes the light of the light emitter 215 to emanate through the display section 214 to the outside of the instrument body 201. This allows the user to easily know through the display section 214 whether electric power is supplied to the heating section 225 of the heat transpiration apparatus 200.

Light may also be emitted by the power switch itself. For example, the power switch may emit green light when ON and red light when OFF. This improves the convenience of use since the position of the power switch can be known by the light emitted by the power switch 213.

As shown in Fig. 44, a positioning piece 208 that serves as retainer is provided on the inner surface of the lid 202 of this embodiment. The positioning piece 208 is located near the guide 209b. The positioning piece 208 is formed integratedly with the inner surface of the lid 202, and a pointed tip of the positioning piece 208 makes contact with the side edge 223 of the transpiring liquid holding body 220.

When the lid 202 is opened, side edge 83 of the transpiring liquid holding body 220 is inserted between the positioning piece 208 and the guide 209b. The transpiring liquid holding body 220 is inserted till the bottom of the side edge 83 makes contact with a rib stopper 209a. Thus the side edge 223 of the transpiring liquid holding body 220 becomes interposed between the tip of the positioning piece 208 and the guide 209b.

When the heated area S of the container 221 is made constant, effective period of the chemical can be adjusted from short to long period by changing the amount of chemical while capacity of the container 221 remains the same.

As shown in Fig. 45, the container 221 of the transpiring liquid holding body 220 has a deep bottom section 221a and a shallow bottom section 221b that is made shallower than the deep bottom section 221a. As shown in Fig. 44, the transpiring liquid holding body 220 is constituted so that the deep bottom section 221a is located above the shallow bottom section 221b when mounted on the lid 202.

When the transpiring liquid holding body 220 is mounted on the lid 202 as shown in Fig. 44, the heating section 225 makes contact with the surface of the shallow bottom section 221b on lower side thereof in Fig. 44.

The deep bottom section 221a and the shallow bottom section 221b of the transpiring liquid holding body 220 contain the chemical and are sealed with the gas-permeable film in advance. When electric power is supplied to the heating section 225, the heating section 225 heats the chemical contained in the shallow bottom section 221b of the transpiring liquid holding body 220 so as to transpire. In case the deep bottom section 221a also contains the chemical, the chemical moves down by gravity from the deep bottom section 221a if the chemical contained in the shallow bottom section 221b decreases through transpiration.

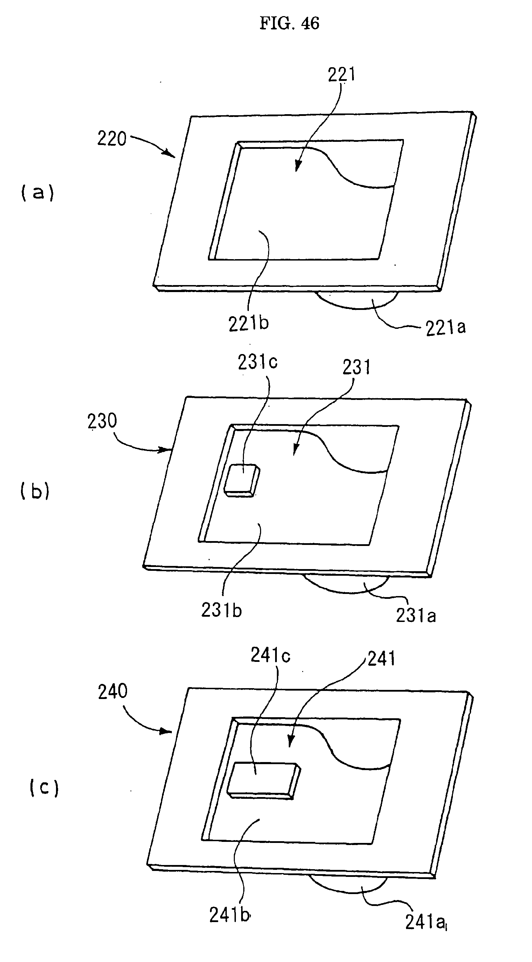

Fig. 46(a) to (c) show the transpiring liquid holding body. Fig. 46(a) shows the same as the transpiring liquid holding body 220 shown in Fig. 45. Fig. 46(b) shows a variation of the transpiring liquid holding body 220 of Fig. 46 (a). Fig. 46(c) shows another variation of the transpiring liquid holding body 220 of Fig. 46(a). In Fig. 46(a) to (c), the gas-permeable film and the chemical are not shown.

The transpiring liquid holding body 230 shown in Fig. 46(b) has a projection 231c provided erected on the shallow bottom section 221b on the gas-permeable film side. Therefore, when the container 231 of the transpiring liquid holding body 230 contains the chemical and is sealed with the gas-permeable film, amount of the chemical contained in the shallow bottom section 221b becomes less than the transpiring liquid holding body 220 by the volume of the projection 231c.

The shallow bottom section 241b of the transpiring liquid holding body 240 shown in Fig. 46(c) also has a projection 241c. The projection 241c has such a configuration as area of the erected portion in top view (viewed from above in Fig. 46(b) and (c)) is larger than the projection of the transpiring liquid holding body 240. As a result, amount of the chemical stored in the shallow bottom section 241b is less than that of the transpiring liquid holding body 220 and the transpiring liquid holding body 230.

Amount of the chemical stored in the shallow bottom section 231b, 241b can be controlled by adjusting the size of projection 231c, 241c of the transpiring liquid holding body 230, 240. Therefore, in case different amounts of chemical are contained in the transpiring liquid holding body 220, 230, 240 according to the scheduled period of use (for example, 30 days, 60 days, 90 days, etc.), the chemical is squeezed by the projection 231c, 241c into the deep bottom portion 231a, 241a so that such a problem can be suppressed as the chemical exists unevenly in the shallow bottom section 231b, 241b. In other words, in the state of being fitted with the lid 202 as shown in Fig. 44, liquid levels of the chemical in the transpiring liquid holding bodies 220, 230, 240 can be made substantially the same regardless of the difference in the amount of the chemical that has been sealed beforehand.

In case the lid 202 is not fitted on the transpiring liquid holding body 220, 230, 240, when the user checks the chemical through the gas-permeable film, since it appears as if substantially the same amount of the chemical is contained regardless of the difference in the amount of the chemical that has been sealed in the transpiring liquid holding bodies 220, 230, 240 beforehand, appearance of the transpiring liquid holding body can be prevented from deteriorating.

The present invention is not limited to the embodiments described above, and various variations and modifications can be made. For example, the transpiring liquid holding body may be inserted into the instrument body instead of the inside of the lid, and rib stopper or guide may be provided on the instrument body for securing the transpiring liquid holding body.

The present invention allows it to make various variations and modifications within the scope defined in the appended claims and, for example, the following means can be preferably employed.

(a) A space is kept between the heater and the heat transpiration container (cartridge) for indirect heating. This constitution prevents the transpired chemical from depositing on the instrument body and the wall surface due to rising air flow.

(b) A thermal insulation sheet is provided on the handle of the cartridge or a part of the tray is extended and/or surface undulation is provided so as to charge the cartridge in the instrument body from the side face thereof. This constitution prevents burning of hand by the cartridge.

(c) Evaporation is stabilized by heating the gas layer in the cartridge. This stabilizes the transpiration by indirect heating.

(d) Part of the tarnspirating film on the cartridge surface is printed with Al or a material (ink) not permeable to the chemical, so as to control the transpiration area. This enables it to control the transpiration rate according to the period of use.

(e) The technical product or the transpiring film are heated to a specific temperature. Consequently, the resulting product is excellent in diffusibility without causing decomposition of chemicals, and also exhibit proper transpiration.

(f) Additives such as particle size regulators (for example, aliphatic hydrocarbons) are added to the technical product. Consequently, diffusibility can be improved.

(g) Hot air is employed for transpiration. Consequently, diffusibility can be improved.

(h) Additives such as transpiration regulators (for example, compounds having a methylene group) are added. Consequently, transpiration can be efficiently regulated.

As the aliphatic hydrocarbon, for example, those having a particle size regulating effect selected from aliphatic hydrocarbons that are transpired by heating can be used as far as they do not exert an adverse influence on the practice of the present invention.

As the compound having a methylene group, for example, those containing an alicyclic compound comprising three of more methylene groups of the general formula $C_nH_{2n}$ (wherein n is 3 to 20) as a main component, and having a transpiration regulating effect can be used as a transpiration regulator (for example, alicyclic compound having a boiling point of 270 to 300°C/760 mmHg, a boiling point of 300 to 350°C/760 mmHg, a boiling point of 210 to 240°C/760 mmHg, a boiling point of 240 to 270°C/760 mmHg, or a boiling point of 270 to 300°C/760 mmHg) as far as they do not exert an adverse influence on the practice of the present invention.

In heat transpiration apparatus of which the lid cannot be opened, the cartridge may be taken out of the apparatus in such manners as follows:

(1) The cartridge receiving stage is moved up or down.
(2) The cartridge lift lever is provided on the side face of the instrument.
(3) The cartridge is inserted and removed from the side face in a frame that incorporates the switch.
(4) The cartridge is pushed up when a particular portion is pressed.
(5) The cartridge is caused to slide up and down along the lid.
(6) The cartridge is pushed up by a spring (pushbutton mechanism).
(7) Through hole is made to penetrate from left to right side faces. That is, the cartridge is inserted into the through hole on one of the side faces and, when it is has been used up, it is pushed out from the other side by inserting a new cartridge which is set by doing so.

[Examples]

The present invention will now be described by way of examples, but the present invention is not limited to only the following examples.

(I)

Example 1

1. Insecticidal technical product

Modified insecticidal technical products with compositions shown in Table 1 were used. These modified insecticidal technical products were prepared by adding additives described below to an insecticidal technical product transfluthrin.

Pigment: Plast Blue 8540 (manufactured by Arimoto Chemical Co., Ltd.)

Antioxidant M: Irganox 1010 (trade name of tetrakis[methylene3-(3,5-di-t-butyl-4-hydroxyphenyl)propionate]methane manufactured by CIBA-GEIGY Limited)

Antioxidant N: 3,5-di-t-butyl-4-hydroxytoluene (BHT)

Table 1

| No. | Compositions (wt%) | | | |
|---|---|---|---|---|
| | Transfluthrin | Pigment | Antioxidant | |
| | | | kind | |
| A | 99.997 | 0.003 | | |
| B | 99.97 | 0. 03 | | |
| C | 98.97 | 0.03 | M | 1 |
| D | 96.97 | 0.03 | M | 3 |
| E | 96.97 | 0.03 | N | 3 |

2. Heat transpiration container

The heat transpiration container 5 as shown in Fig. 2 was used. An aluminum plate measuring 35 mm by 30 mm by 0.15 mm in thickness having a recess 2 (measuring 30 mm by 18 mm by depth shown in Table 2) was used as the heat resistant sheet (container body). After laminating the gas-permeable film 4 on the top surface of the heat resistance sheet 1, the insecticidal technical product (hereinafter called the content liquid) is injected into the container with a syringe, and the injection hole was sealed with an adhesive. Injected amount of the liquid was 300 mg for the samples Nos.5 and 9, and 1200 mg for other samples shown in Table 2. The aluminum plate was an Al sheet 100 μm thick coated with cast polypropylene film (CPP) to a thickness of 50 μm as the sealant layer for improving the adhesion with the gas-permeable film 4 and the liquid tightness and coated with biaxially oriented polypropylene film on the back surface.

Table 2

| Sample No. | Film | Content liquid | Depth of recess 2 (mm) | Air intake port |
|---|---|---|---|---|
| 1 | OPP20/PE30 | B | 2 | Nothing |
| 2 | OPP20/PE30 | C | 2 | Nothing |
| 3 | OPP20/PE30 | D | 2 | Nothing |
| 4 | OPP20/PE30 | E | 2 | Nothing |
| 5 | OPP20/PE30 | C | 0. 5 | Nothing |
| 6 | Pereca | A | 2 | Nothing |
| 7 | Retort CPP30 | B | 2 | Nothing |
| 8 | OPP20/PE30 | C | 2 | Existence |
| 9 | OPP20/PE30 | C | 0. 5 | Existence |

The following materials were used as the gas-permeable film.

(i) OPP20/PE30: gas-permeable film made by laminating biaxially oriented polypropylene film (OPP) 20 μm thick and polyethylene film (PE) 30 μm thick.
(ii) Pereca: gas-permeable film made by laminating cast polypropylene film (CPP) 30 μm thick and microporous polyethylene terephthalate film (PET) 12 μm thick.
(iii) Retort CPP30: (cast) heat-stable polypropylene film (CPP) 30 μm thick

3. Test method

With a heat transpiration apparatus of which heater temperature (surface temperature of the radiation plate 7) was set to 95°C, the chemical was transpired by heating the heat transpiration container tilted by 50° from the horizontal plane on the entire surface of the bottom.
Insecticide component transpired from the heat transpiration container 5 was collected in a silica gel column. The insecticide component was extracted from the silica gel with chloroform and concentrated, before quantitative analysis with gas chromatograph to determine the amounts transpired at predetermined intervals.

4. Test results

Amounts transpired in the period from 67 hours to 72 hours and in the period from 192 hours to 198 hours after the start of heating are shown in Table 3.

Table 3

| Sample No. | Period from 68 hours to 72 hours after the start of heating | | Period from 192 hours to 198 hours after the start of heating | |
|---|---|---|---|---|
| | Total amounts transpired (mg/4 hours) | Average of amounts transpired (mg/2 hours) | Total amounts transpired (mg/6 hours) | Average of amounts transpired (mg/2 hours) |
| 1 | 0.92 | 0.46 | 2.20 | 0.73 |
| 2 | 0.90 | 0.45 | 2.44 | 0.81 |
| 3 | 1.58 | 0.79 | 2.55 | 0.85 |
| 4 | 1.04 | 0.52 | 1.87 | 0.62 |
| 5 | 1.95 | 0.98 | 6.27 | 2.09 |
| 6 | 0.22 | 0.11 | 0.69 | 0.23 |
| 7 | 2.37 | 1. 19 | 5.42 | 1.81 |
| 8 | 1.46 | 0. 73 | 3.15 | 1.05 |

Amounts transpired in the period from 12 hours to 18 hours of samples 5 and 9 are shown in Table 4.

Table 4

| Sample No. | Period from 12 hours to 18 hours after the start of heating | |
|---|---|---|
| | Total amounts transpired (mg/6 hours) | Average of amounts transpired (mg/2 hours) |
| 5 | 3.51 | 1.17 |
| 9 | 5.18 | 1.73 |

From Tables 3 and 4, it can be seen that the heat transpiration method of the present invention that uses the insecticidal technical product causes stable transpiration of the insecticide component.

Example 2

(Relation of transpiration rate to duration of heating)

The insecticidal technical product was transpired under the following conditions, and the amount transpired as determined similarly to Example 1. In the description that follows, "intermittent 12-hourheating" refers to a repetitive operation of a cycle consisting of 12 hours of energization and 12 hours of power off.

[I] 30-day use setting

(1) Test condition

a. Content liquid: A shown in Table 1
b. Heat transpiration container: The same container as that for sample No. 1 shown in Tale 1
c. Gas-permeable film: OPP20/PE30 (described previously)
d. Amount injected: 300 mg

e. Set temperature of heater: 85°C
f. Number of samples: 3
g. Transpired amount measuring period: As shown in tables in (2).

(2) Test results

Table 5

Intermittent 12-hour heating

| Sample No. | Unit | Amounts transpired | | | |
|---|---|---|---|---|---|
| | | Period from 0 hours to 12 hours after the start of heating | Period from 120 hours to 132 hours after the start of heating | Period from 240 hours to 252 hours after the start of heating | Period from 360 hours to 372 hours after the start of heating |
| 1 0 −① | mg/12hours | 9.16 | 9.99 | 10.12 | 9.19 |
| 1 0 −② | | 9.25 | 9.81 | 8.17 | 7.39 |
| 1 0 −③ | | 11.79 | 8.31 | 8.79 | 7.19 |
| Average | mg/hour | 0.84 | 0.78 | 0.75 | 0.66 |
| Average | mg/12 hours | 10.07 | 9.37 | 9.03 | 7.92 |

Table 6

Intermittent 8-hour heating

| Sample No. | Unit | Amounts transpired | | | |
|---|---|---|---|---|---|
| | | Period from 0 hours to 8 hours after the start of heating | Period from 120 hours to 128 hours after the start of heating | Period from 240 hours to 248 hours after the start of heating | Period from 360 hours to 368 hours after the start of heating |
| 1 0 −① | mg/8 hours | 7.67 | 6.68 | 5.95 | 5.92 |
| 1 0 −② | | 7.17 | 6.81 | 5.37 | 5.12 |
| 1 0 −③ | | 6.15 | 6.53 | 5.67 | 5.81 |
| Average | | 7.00 | 6.67 | 5.66 | 5.61 |
| Average | mg/hour | 0.87 | 0.83 | 0.71 | 0.70 |
| Average | mg/12 hours | 10.50 | 10.01 | 8.49 | 8.42 |

Table 7

Intermittent 3-hour heating

| Sample No. | | Amounts transpired | | | |
|---|---|---|---|---|---|
| | Unit | Period from 0 hours to 3 hours after the start of heating | Period from 120 hours to 123 hours after the start of heating | Period from 240 hours to 243 hours after the start of heating | Period from 360 hours to 363 hours after the start of heating |
| 1 0−① | mg/3 hours | 1.52 | 2.10 | 2.34 | 2.21 |
| 1 0−② | | 1.91 | 2.55 | 2.44 | 2.34 |
| 1 0−③ | | 1.83 | 2.20 | 1.83 | 1.91 |
| Average | | 1.75 | 2.28 | 2.20 | 2.15 |
| Average | mg/hour | 0.58 | 0.76 | 0.73 | 0.72 |
| Average | mg/12 hours | 7.00 | 9.13 | 8.81 | 8.60 |

EP 1 547 464 A1

Table 8

Continuous heating

| Sample No. | Unit | Amounts transpired | | | | | |
|---|---|---|---|---|---|---|---|
| | | Period from 0 hours to 2 hours after the start of heating | Period from 5 hours to 7 hours after the start of heating | Period from 10 hours to 12 hours after the start of heating | Period from 120 hours to 122 hours after the start of heating | Period from 240 hours to 242 hours after the start of heating | Period from 360 hours to 362 hours after the start of heating |
| 1 0 −① | mg/2 hours | 0.93 | 1.52 | 1.61 | 1.31 | 1.37 | 1.26 |
| 1 0 −② | | 0.93 | 1.39 | 1.42 | 1.27 | 1.49 | 0.85 |
| 1 0 −③ | | 0.79 | 1.45 | 1.53 | 1.75 | 1.71 | 1.39 |
| Average | | 0.88 | 1.45 | 1.52 | 1.44 | 1.52 | 1.17 |
| Average | mg/hour | 0.44 | 0.73 | 0.76 | 0.72 | 0.76 | 0.58 |
| Average | mg/12 hours | 5.29 | 8.71 | 9.11 | 8.64 | 9.13 | 6.99 |

EP 1 547 464 A1

[II] 60-day use setting

(1) Test condition

    a. Content liquid: A shown in Table 1
b. Heat transpiration container: The same container as that for sample No. 1 shown in Tale 2
c. Gas-permeable film: OPP20/PE30 (described previously)
d. Amount injected: 600 mg
e. Set temperature of heater: 85°C
f. Number of samples: 3
g. Transpired amount measuring period: As shown in tables in (2).

(2) Test results

Table 9

Intermittent 12-hour heating

| Sample No. | Unit | Amounts transpired | | | |
|---|---|---|---|---|---|
| | | Period from 0 hours to 12 hours after the start of heating | Period from 240 hours to 252 hours after the start of heating | Period from 480 hours to 492 hours after the start of heating | Period from 720 hours to 732 hours after the start of heating |
| 1 1—① | mg/12 hours | 10.88 | 10.33 | 9.09 | 8.29 |
| 1 1—② | mg/12 hours | 9.90 | 9.61 | 9.35 | 9.19 |
| 1 1—③ | mg/12 hours | 11.84 | 10.47 | 8.05 | 7.31 |
| Average | mg/hour | 0.91 | 0.84 | 0.74 | 0.69 |
| Average | mg/12 hours | 10.87 | 10.14 | 8.83 | 8.26 |

Table 10

Intermittent 8-hour heating

| Sample No. | Unit | Amounts transpired | | | |
|---|---|---|---|---|---|
| | | Period from 0 hours to 8 hours after the start of heating | Period from 240 hours to 248 hours after the start of heating | Period from 480 hours to 488 hours after the start of heating | Period from 720 hours to 728 hours after the start of heating |
| 1 1－① | mg/8 hours | 6. 70 | 6. 09 | 5. 49 | 5. 21 |
| 1 1－② | | 6. 33 | 6. 92 | 5. 98 | 5. 98 |
| 1 1－③ | | 6. 08 | 7. 90 | 5. 27 | 4. 85 |
| Average | | 6. 37 | 6. 97 | 5. 58 | 5. 35 |
| Average | mg/hour | 0. 80 | 0. 87 | 0. 70 | 0. 67 |
| Average | mg/12 hours | 9. 56 | 10. 45 | 8. 37 | 8. 02 |

EP 1 547 464 A1

Table 11

Intermittent 3-hour heating

| Sample No. | unit | Amounts transpired | | | |
|---|---|---|---|---|---|
| | | Period from 0 hours to 3 hours after the start of heating | Period from 240 hours to 243 hours after the start of heating | Period from 480 hours to 483 hours after the start of heating | Period from 720 hours to 723 hours after the start of heating |
| 1 1 −① | mg/3 hours | 1. 93 | 2. 40 | 2. 24 | 2. 30 |
| 1 1 −② | | 1. 82 | 1. 93 | 1. 88 | 2. 03 |
| 1 1 −③ | | 1. 78 | 2. 38 | 2. 11 | 2. 16 |
| Average | | 1. 84 | 2. 24 | 2. 08 | 2. 16 |
| Average | mg/hour | 0. 61 | 0. 75 | 0. 69 | 0. 72 |
| Average | mg/12 hours | 7. 38 | 8. 95 | 8. 31 | 8. 66 |

Table 12

Continuous heating

| Sample No. | | Amounts transpired | | | | | |
|---|---|---|---|---|---|---|---|
| | Unit | Period from 0 hours to 2 hours after the start of heating | Period from 5 hours to 7 hours after the start of heating | Period from 10 hours to 12 hours after the start of heating | Period from 240 hours to 242 hours after the start of heating | Period from 480 hours to 482 hours after the start of heating | Period from 720 hours to 722 hours after the start of heating |
| 1 1 —① | mg/2 hours | 1.07 | 2.11 | 1.86 | 1.52 | 1.55 | 1.45 |
| 1 1 —② | | 1.07 | 1.97 | 1.64 | 1.37 | 1.55 | 1.37 |
| 1 1 —③ | | 1.22 | 1.83 | 1.66 | 1.29 | 1.49 | 1.35 |
| Average | | 1.12 | 1.97 | 1.72 | 1.40 | 1.53 | 1.39 |
| Average | mg/hour | 0.56 | 0.99 | 0.86 | 0.70 | 0.76 | 0.70 |
| Average | mg/12 hours | 6.73 | 11.82 | 10.32 | 8.37 | 9.17 | 8.35 |

EP 1 547 464 A1

[III] 120-day use setting

(1) Test condition

a. Content liquid: A shown in Table 1
b. Heat transpiration container: The same container as that for sample No. 1 shown in Tale 2
c. Gas-permeable film: OPP20/PE30 (described previously)
d. Amount injected: 1200 mg
e. Set temperature of heater: 85°C
f. Number of samples: 3
g. Transpired amount measuring period: As shown in tables in (2).

2) Test results

2) Test results

Table 13

Intermittent 6-hour heating

| Sample No. | Unit | Amounts transpired | | | | |
|---|---|---|---|---|---|---|
| | | Period from 68 hours to 74 hours after the start of heating | Period from 192 hours to 198 hours after the start of heating | Period from 468 hours to 474 hours after the start of heating | Period from 678 hours to 684 hours after the start of heating | Period from 828 hours to 834 hours after the start of heating |
| 1 2 −① | mg/6 hours | 4. 23 | 6. 53 | 6. 39 | 5. 75 | 4. 42 |
| 1 2 −② | | 4. 20 | 6. 35 | 4. 76 | 4. 07 | 3. 51 |
| 1 2 −③ | | 3. 00 | 3. 60 | 3. 76 | 2. 96 | 2. 69 |
| Average | | 3. 81 | 5. 49 | 4. 97 | 4. 26 | 3. 54 |
| Average | mg/hour | 0. 64 | 0. 92 | 0. 83 | 0. 71 | 0. 59 |
| Average | mg/12 hours | 7. 62 | 10. 98 | 9. 94 | 8. 52 | 7. 08 |

EP 1 547 464 A1

Tables 5 to 13 show that the heat transpiration method of the present invention that uses the insecticidal technical product causes stable transpiration of the insecticide component regardless of whether transpiration is carried out intermittently or continuously. Tables 9 to 13 also show that the present invention causes stable transpiration of the insecticide component even in a long period of transpiration for 360 hours or more.

Example 3

(Influence of gas-permeable film on transpiration rate)

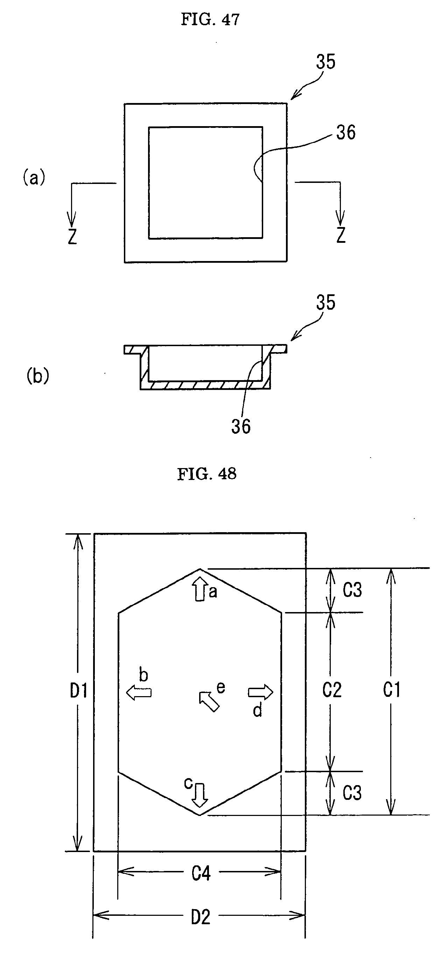

Rate of transpiration was compared between a case in which the gas-permeable film 4 was laminated on the heat transpiration container 35 shown in Fig. 47 and a case without lamination, and rate of transpiration was compared between a case in which the gas-permeable film 4 was in contact with the content liquid 3 and a case without contact, by evaporating the insecticidal technical product under the following conditions and determining the amount of transpiration similarly to Example 1.

(1) Test condition

a. Content liquid: A shown in Table 1
b. Heat transpiration container: The heat transpiration container 35 shown in Fig. 47(a) and (b) was used. The heat transpiration container has the shape shown in Fig. 47(a) and (b), measuring 33 mm by 25 mm having a recess 36 measuring 26 mm by 17 mm by 3 mm in depth. With other respects, the container was the same as that used in Example 1.
c. Gas-permeable film: Cast polypropylene film 50 μm in thickness
d. Amount injected: As shown in Table 14.
e. Set temperature of heater: 104°C With this setting, surface temperature of the gas-permeable film and temperature of the content liquid were as shown in Table 14. Surface temperature of the gas-permeable film was measured at the top surface and near the center of the film, and temperature of the content liquid was measured near the center of the content liquid.
f. Number of samples: 3 for each sample.
g. Transpired amount measuring period: From 6 hours to 12 hours after the start of heating.
h. Placement of heat transpiration container during heating: Horizontal

(2) Test results

Table 14

| Sample No. | Amount of content liquid (Amount injected) (mg) | Existence of Gas-permeable Film | Temperature of content liquid (°C) | Surface temperature of Gas-permeable film (°C) | Amounts transpired (Period from 6 hours to 12 hours after the start of heating) (mg/6 hours) |
|---|---|---|---|---|---|
| 1 3 — ① | 1700 | ※(1) Existence | 92.1 | 87.7 | 9.47 |
| 1 3 — ② | | | | | 11.98 |
| 1 3 — ③ | | | | | 11.70 |
| Average | | | | | 11.05 |
| 1 4 — ① | 300 | ※(2) Existence | 93.2 | 78.2 | 2.96 |
| 1 4 — ② | | | | | 2.81 |
| 1 4 — ③ | | | | | 1.95 |
| Average | | | | | 2.57 |
| 1 5 — ① | 1700 | Nothing | 92.6 | — | 10.56 |
| 1 5 — ② | | | | | 10.15 |
| 1 5 — ③ | | | | | 8.92 |
| Average | | | | | 9.88 |

※(1) : Film comes into contact with content liquid.
※(2) : Film does not come into contact with content liquid.

EP 1 547 464 A1

From Table 14, it can be seen that the amount transpired was higher in the sample No. 13 in which the film 4 was in contact with the content liquid 3 and in sample No. 15 in which the film 4 was not laminated, than in sample No. 14 in which the film 4 was not in contact with the content liquid 3.

Example 4

(Transpiration performance of various insecticidal technical product)

Transpiration performance was compared between three kinds of insecticidal technical product by evaporating the insecticidal technical product under the following conditions and determining the amount of transpiration similarly to Example 1.

(1) Test condition

   a. Content liquid: A shown in Table 15.
   b. Heat transpiration container: The same container as that used in Example 3.
   c. Gas-permeable film: Cast polypropylene film 50 μm in thickness was used for every sample. The test was conducted with the gas-permeable film 4 in contact with the content liquid 3 as shown in Fig. 3(a).
   d. Amount injected: 1700 mg.
   e. Set temperature of heater: 104°C
   f. Number of samples: 3 for each sample.
   g. Transpired amount measuring period: From 12 hours to 24 hours after the start of heating.
   h. Placement of heat transpiration container during heating: Horizontal

(2) Test results

Table 15

| Sample No. | Content liquid | Amounts transpired Period from 12 hours to 24 hours after the start of heating (mg/12 hours) |
|---|---|---|
| 16-① | Transfluthrin : 100 wt% | 24.1 |
| 16-② | | 28.3 |
| 16-③ | | 27.7 |
| Average | | 26.7 |
| 17-① | 「S-1264」: 100 wt% (manufactured by Sumitomo Chemical Co.,Ltd.) | 18.0 |
| 17-② | | 18.9 |
| 17-③ | | 17.2 |
| Average | | 18.0 |
| 18-① | Empenthrin : 100 wt% | 70.7 |
| 18-② | | 69.4 |
| 18-③ | | 62.2 |
| Average | | 67.4 |

From table 15, it can be seen that stable transpiration of the insecticidal technical product to release the insecticide component was achieved in all samples Nos.16 to 18.

Example 5

(Investigation of gas-permeable film)

1. Gas-permeable film

Composite films were prepared laminating two or three kinds of film as shown in Table 16. LLDPE, LDPE or CPP was laminated as a sealant layer directly on the heat transpiration container. "LLDPE/perforated LDPE/CPP" of sample No. 22 is made by laminating the films in this order, and the same applies to sample No. 23. Proportion of aperture in Table 16 refers to the ratio of total area of fine apertures made by laser beam to the area of the entire PET film.

Table 16

| Sample No. | Quality of material | Thickness | Others |
|---|---|---|---|
| 20 | OPP/LLDPE | OPP 20 $\mu$m<br>LLDPE 40 $\mu$m | |
| 21 | OPP/CPP | OPP 20 $\mu$m<br>CPP 40 $\mu$m | |
| 22 | LLDPE/perforated PET/LLDPE | LLDPE 30 $\mu$m<br>perforated PET 12 $\mu$m<br>LLDPE 30 $\mu$m | Proportion of aperture : 10.4% |
| 23 | CPP/perforated PET/CPP | CPP 30 $\mu$m<br>perforated PET 12 $\mu$m<br>CPP 30 $\mu$m | Proportion of aperture : 10.4% |
| 24 | PET/LLDPE | PET 20 $\mu$m<br>LLDPE 20 $\mu$m | |
| 25 | PET/CPP | PET 20 $\mu$m<br>CPP 20 $\mu$m | |

(i) LLDPE: Linear low density polyethylene
(ii) CPP: Cast polypropylene film
(iii) OPP: Biaxially oriented polypropylene film
(iv) PET: Polyethylene terephthalate

2. Heat transpiration container

The heat transpiration container 35 as shown in Fig. 47 was used. An aluminum plate measuring 42 mm by 38 mm by 0.1 mm in thickness having a recess 2 (measuring 26 mm by 17 mm by 3 mm in depth) was used as the heat resistant sheet 1 (container body). The recess 2 of this container as filled with transfluthrin containing 0.01% by weight of a pigment (Green No. 202) and was covered with the gas-permeable film 4 shown in Table 16 at the top.

3. Test method

With the heat transpiration container of which heater temperature (surface temperature of the radiation plate 7) was set to 100°C, the modified insecticidal technical product was transpired by heating the heat transpiration container 35 tilted by 50° from the horizontal plane on the entire surface of the bottom, and the amount of the effective component transpired similarly to Example 1.

Change in the appearance by the thermal transpiration was visually evaluated. In Table 17 that shows the test results, "○" and "Δ" indicate that the film can be practically used as the gas-permeable film.

Table 17

| Sample No. | Material | Change in the appearance | | | Amounts transpired (mg/12 hours) |
|---|---|---|---|---|---|
| | | swelling | breakage | crystallization on the film surface | |
| 20 | OPP/LLDPE | O | O | O | 10.2 |
| 21 | OPP/CPP | △ | O | O | 9.8 |
| 22 | LLDPE/perforated PET/LLDPE | O | O | O | 6.3 |
| 23 | CPP/perforated PET/CPP | O | △ | O | 5.2 |
| 24 | PET/LLDPE | O | O | O | 1.8 |
| 25 | PET/CPP | O | O | O | 2.0 |

From Table 17, it can be seen that the gas-permeable film made in a composite film of two or three layers by using LLDPE or CPP as sealant layer and laminating such a film as OPP, perforated PET and PET thereon is suitable for thermal transpiration since such problems are less likely to occur as swelling or breakage due to heating and crystallization of the insecticide component on the film surface.

(II)

Example 6

The following test was conducted by using the heat transpiration apparatus 50 shown in Fig. 47, so as to determine the effect of the change in liquid amount of the insecticidal technical product 45 in the heat transpiration container 41 on the amount transpirated.

1. Fabrication of heat transpiration container

The films (single film or composite film of two or three layers) shown in Table 18 were prepared for use in the samples. CPP, OPP, PE and PET in Table 18 represent the following materials.
    CPP: Cast polypropylene film
    OPP: Biaxially oriented polypropylene film
    PE: Low density polyethylene
    PET: Polyethylene terephthalate
"CPP/PET/CPP" of the outer film of sample No. 3 is a composite film made by laminating CPP, PET and CPP in this order, and the same applies to other samples. The inner film was provided with two liquid introducing port 52a, 52b formed therein as shown in Fig. 11.

Aluminum container having recess 46a measuring 26 mm by 17 mm by 3 mm in depth and flange 46b measuring 33 mm by 25 mm was used as the container body 46. The transpiration aperture of the container body 46 was covered by the inner film 47. Then the container body 46 was filled with a predetermined amount of insecticidal technical product 45 (transfluthrin) by using a feeder nozzle 59 shown in Fig. 13 assuming the early stage and latter stage of transpiration. The heat transpiration container 41 was made by placing the outer film 51 on the inner film 47 and sealing. The flange 46b and the periphery of the inner film 47, and the periphery of the inner film 47 and the periphery of the outer film 51 were bonded together by thermal fusion.

2. Test method

With the heat transpiration apparatus 50 shown in Fig. 47 of which heater 42 temperature (surface temperature of the radiation plate 5) was set to 100°C, the insecticidal technical product 45 was transpired by heating the heat transpiration container 41 tilted by 45° from the horizontal plane on the entire surface of the bottom. Amount of the insecticidal technical product 45 transpired in the period from 24 hours to 36 hours after heating was measured (n = 5). Results of measurements (mean values) are shown in Table 18. The decreasing rate shown in Table 18 was calculated by the following equation from the amount transpired assuming that the container held 1200 mg of insecticidal technical product 45 at the start of transpiration and 150 mg of the insecticidal technical product 45 at the end of transpiration.

$$\text{Decreasing rate (\%)} = \{(\text{Amount transpired in early stage}) - (\text{Amount transpired in latter stage})\} / (\text{Amount transpired in early stage}) \times 100$$

<Method to measure the amount transpired>

Insecticide component transpired from the heat transpiration container 41 was collected in a silica gel column. The insecticide component was extracted from the silica gel with chloroform and concentrated, before quantitative analysis with gas chromatograph to determine the amounts transpired over a period of 12 hours.

Table 18

| Sample No. | Inner film | Outer film | Amount of the insecticidal technical product (mg) | Amount transpired (mg/12 hours) | Decreasing rate (%) |
|---|---|---|---|---|---|
| 1 | CPP (50) | CPP (50) | 1200 | 13.4 | 11.9 |
| | | | 150 | 11.8 | |
| 2 | OPP (20)/PE (40) | PET (20)/CPP (40) | 1200 | 13.0 | 8.5 |
| | | | 150 | 11.9 | |
| 3 | OPP (20)/PE (40) | CPP (50)/PET (15)/CPP (50) | 1200 | 8.2 | 18.3 |
| | | | 150 | 6.7 | |

* Numbers of parentheses of each film shows thickness of films (unit: $\mu$m).

From Table 18 it can be seen that decreasing rate of the amount of transpiration is low even when the remaining insecticidal technical product 45 becomes small and the amount of transpiration is stable over the period from early stage to latter stage of transpiration in samples Nos.1 to 3 in which contact area between the insecticidal technical product 45 and the outer film was made larger by the use of capillary phenomenon.

Example 7

The inner film and outer film made of the materials with thickness shown in Table 19 with grating pattern of projections and recesses shown in Fig. 9 formed by embossing on the contact between the inner film and outer film were used. Aluminum container having recess 46a measuring 25 mm by 17 mm by 3 mm in depth and flange 46b measuring 33 mm by 25 mm was used as the container body 46. The amount of transpiration of the insecticidal technical product 45 and the decreasing rate were measured similarly to Example 6, except that each container body 46 held 1200 mg and 200 mg of the insecticidal technical product 45, respectively, with assuming the early stage and latter stage of transpiration.

The insecticidal technical product 45 was heated continuously for 36 hours, and the amount of transpiration of the insecticidal technical product 45 in a period from 24 hours to 36 hours after starting to heat was measured (n = 3), with the decreasing rate determined similarly to Example 6. Results of measurements (mean values) are shown in Table 19. The embossed pattern formed on the inner film 47 consists of projections 54 measuring 1.5 mm by 1.5 mm and recesses 53 measuring 0.5 mm in width and 0.5 mm in depth formed between the projections 54. OPP and PE in Table 19 represent the following materials.

OPP: biaxially oriented polypropylene film
PE: Low density polyethylene

Table 19

| Sample No. | Inner film | Outer film | Amount of the insecticidal technical product (mg) | Amount transpired (mg/12 hours) | Decreasing rate (%) |
|---|---|---|---|---|---|
| 4 | OPP (50 μm)/PE (40 μm) Formed by embossing | OPP (50 μm)/PE (40 μm) | 1200 | 14.7 | 22.4 |
| | | | 200 | 11.4 | |

* Numbers of parentheses of each film shows thickness of films (unit: μm).

From Table 19 it can be seen that the amount of transpiration is stable over the period from early stage to latter stage of transpiration in sample No. 4 having the embossed inner film 47.

53

Example 8

Unwoven fabric was used as the inner film 47 and the material shown in Table 20 was used as the outer film 51. Aluminum container having the recess 46a measuring 25 mm by 17 mm by 3 mm in depth and the flange 46b measuring 33 mm by 25 mm was used as the container body 46. The amount of transpiration of the insecticidal technical product 45 was measured similarly to Example 6, except that each container body 46 held 1200 mg and 200 mg of the insecticidal technical product 45, respectively, with assuming the early stage and latter stage of transpiration.

Sample No. 5 was made by thermal fusion of the inner film 47 (unwoven fabric) onto the PE side of the outer film 51 by means of heat press, and bonding the periphery of the inner film 47 onto the flange 46b of the container body 46 by thermal fusion. Sample No. 6 was made by thermal fusion of the flange 46b and periphery of the inner film 47 (unwoven fabric), and periphery of the inner film 47 and periphery of the outer film 51, without bonding the inner film 47 and the outer film 51 in portions other than the periphery (the portion that blocks the transpiration aperture of the container body 46). The insecticidal technical product 45 was heated continuously for 36 hours, and the amount of transpiration of the insecticidal technical product 45 in a period from 24 hours to 36 hours after starting to heat was measured (n = 4 for sample 5, n = 3 for sample 6), with the decreasing rate determined similarly to Example 6. Results of measurements (mean values) are shown in Table 20. For the unwoven fabric, STRATEC RW2030 (polypropylene with weight of fabric per unit area 30g/cm$^2$) manufactured by Idemitsu Petrochemical Co., Ltd. was used. OPP and PE in Table 3 represent the following materials.

OPP: Biaxially oriented polypropylene film
PE: Low density polyethylene

Table 20

| Sample No. | Inner film | Outer film | Amount of the insecticidal technical product (mg) | Amount transpired (mg/12 hours) | Decreasing rate (%) |
|---|---|---|---|---|---|
| 5 | Unwoven fabric (Contacting with outer film) | OPP (20)/PE (40) | 1200 | 14.7 | 11.6 |
|  |  |  | 200 | 13.0 |  |
| 6 | Unwoven fabric (Un-contacting with outer film) | OPP (20)/PE (40) | 1200 | 15.0 | 21.3 |
|  |  |  | 200 | 11.8 |  |

\* Numbers of parentheses of outer film shows thickness of films (unit: $\mu$ m).

From Table 20 it can be seen that decreasing rate of the amount of transpiration is low even when the remaining insecticidal technical product 45 becomes small and the rate of transpiration is stable over the period from early stage to latter stage of transpiration in samples Nos.5 and 6 in which contact area between the insecticidal technical product 45 and the outer film 51 was made larger by the use of unwoven fabric (liquid absorbing film). It can also be seen that the rate of transpiration is more stable in samples No. 5, in which the unwoven fabric was thermally fused with the outer film 51, than in sample No. 6.

(III)

Example 9

(Depth and surface temperature of recess)

Surface temperature of recess was investigated by using sample containers 61-1, 1-2 and 1-3 having the same dimensions and same shape except that depth h of the recess shown in Fig. 15(b) is different. The sample container was 0.15 mm in thickness, and was made by laminating a biaxially oriented polypropylene film 50 μm thick on one side (to become inside of container) of an aluminum sheet 100 μm thick. Dimensions in various portions of the sample container are as follows and are also shown in Fig. 48.

D1: 36 mm
D2: 28 mm
C1: 30 mm
C2: 20 mm
C3: 5 mm
C4: 20 mm

Each sample container was placed on the radiation plate 67 of which surface temperature was kept at 108.8°C. 30 minutes later, surface temperature of the bottom of the recess 62 was measured at measuring points indicated by arrows a to e in Fig. 48. A digital thermometer manufactured by Anritsu Meter Co., Ltd. was used in the measurement.

Table 21

| Depth of the Recess (mm) | Surface Temperature (°C) | | | | | |
|---|---|---|---|---|---|---|
| | a | b | c | d | e | Average |
| 0.5 | 101.7 | 100.9 | 103.4 | 100.4 | 104.9 | 102.3 |
| 1.0 | 101.4 | 99.7 | 100.1 | 96.9 | 101.2 | 99.9 |
| 2.0 | 102.1 | 99.4 | 100.9 | 92.6 | 99.2 | 98.8 |

From Table 21, it can be seen that surface temperature of the recess becomes lower as the depth of the recess becomes larger.

Example 10

(Flange area and recess surface temperature)

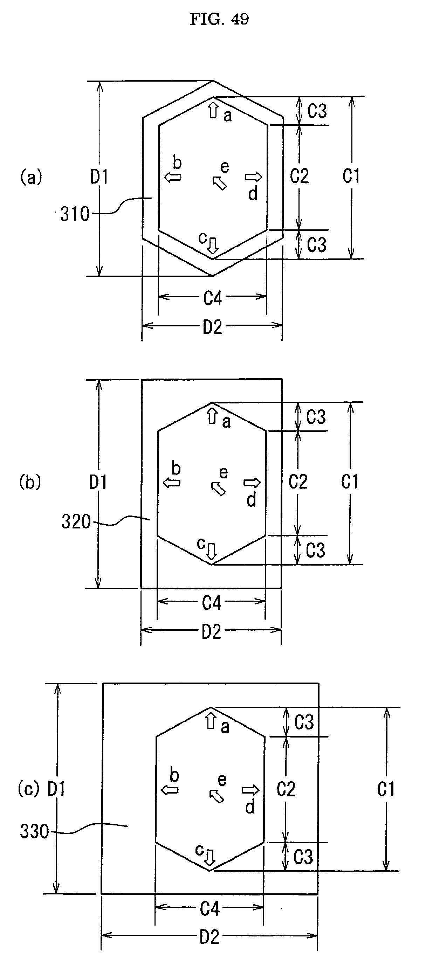

Three sample containers having flanges 310, 320 and 330 of different sizes (surface areas) were made as shown in Fig. 49(a) to (c). The sample container was 0.15 mm in thickness, and was made by laminating a biaxially oriented polypropylene film 50 μm thick on one side (to become inside of container) of an aluminum sheet 100 μm thick, similarly to example 9.

Dimensions and areas of various portions of the sample container are shown in Table 22. Depth of the recess was 2 mm in all samples. Surface temperature of the bottom of the recess was measured in each sample container similarly to example 9. The results are shown in Table 23.

Table 22

| Sample Container | Dimension (mm) | | | | | | Flange area (mm$^2$) |
|---|---|---|---|---|---|---|---|
| | D | D | C | C | C | C | |
| | 1 | 2 | 1 | 2 | 3 | 4 | |
| Fig. 49(a) | 34 | 23 | 30 | 20 | 5 | 20 | 236 |
| Fig. 49(b) | 36 | 28 | 30 | 20 | 5 | 20 | 508 |
| Fig. 49(c) | 36 | 40 | 30 | 20 | 5 | 20 | 940 |

Table 23

| Sample Container | Surface Temperature (°C) | | | | | |
|---|---|---|---|---|---|---|
| | a | b | c | d | e | Average |
| Fig. 49(a) | 103.7 | 105.1 | 103.1 | 104.6 | 105.9 | 104.5 |
| Fig. 49(b) | 99.4 | 101.6 | 98.3 | 100.0 | 103.1 | 100.5 |
| Fig. 49(c) | 92.0 | 97.6 | 90.8 | 94.0 | 98.4 | 94.6 |

From Tables 22 and 23, it can be seen that surface temperature of the recess becomes lower as the flange becomes larger.

Example 11

(Rib size and recess surface temperature)

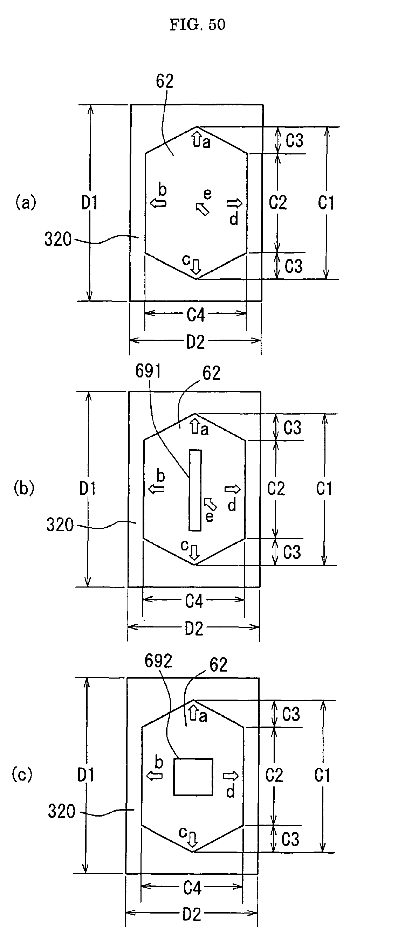

A sample container without rib in the recess 62 and two sample containers having ribs 691, 692 were made as shown in Fig. 50(a) to (c). The sample container was 0.15 mm in thickness, and was made by laminating a biaxially oriented polypropylene film 50 μm thick on one side (to become inside of container) of an aluminum sheet 100 μm thick, similarly to example 9. Depth of the recess was 1 mm in all samples. Dimensions of various portions shown in Fig. 50(a) to (c) are as follows.
　　　D1: 32 mm
　　　D2: 25 mm
　　　C1: 25 mm
　　　C2: 17 mm
　　　C3: 4 mm
　　　C4: 14 mm
Surface temperature of the bottom of the recess was measured in each sample container similarly to example 9. The results are shown together with rib size in Table 24.

Table 24

| Sample Container | Rib size (mm) | Surface Temperature (°C) | | | | | |
|---|---|---|---|---|---|---|---|
| | L×W×H | a | b | c | d | e | Average |
| Fig. 50(a) | No ribs | 106.9 | 103.0 | 105.2 | 99.8 | 104.5 | 103.9 |
| Fig. 50(b) | 14×1×1 | 106.2 | 99.5 | 105.8 | 100.0 | 102.3 | 102.8 |
| Fig. 50(c) | 7×5×1 | 102.8 | 100.3 | 102.9 | 96.9 | — | 100.9 |
| *L: Length, W: Width, H: Height | | | | | | | |

From Tables 24 it can be seen that surface temperature of the recess becomes lower as the rib becomes larger.

(IV)

Example 12

<Relation between tilting angle of heat transpiration container and amount of transpiration>

1. Heat transpiration container

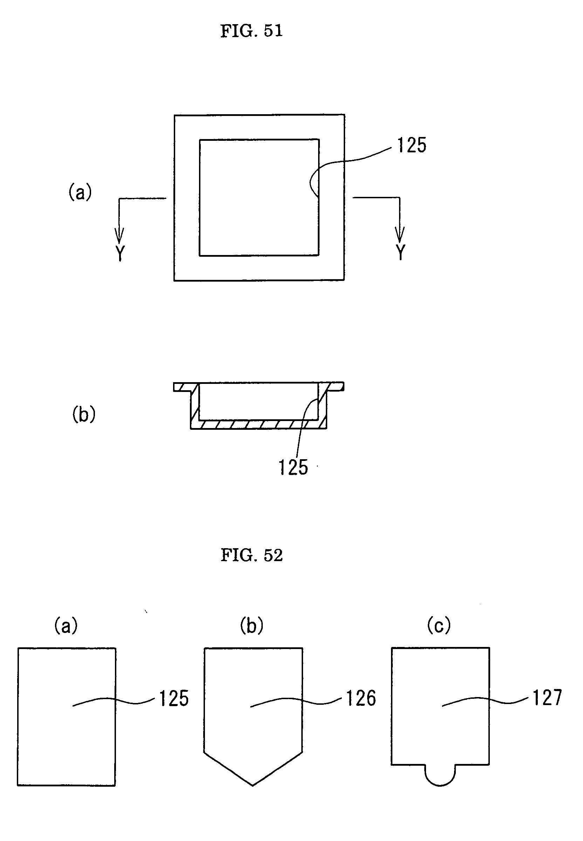

The container body having shape as shown in Fig. 51 was filled with 100 mg of transfluthrin as the insecticidal technical product in the recess 125, and top of the recess 125 was covered with gas-permeable film, thereby making the heat transpiration container. Specifications of the container body and the gas-permeable film were as follows.
The container body was made from aluminum sheet 100 μm thick measuring 33 mm by 25 mm having the recess 125 measuring 26 mm by 17 mm by 3 mm in depth formed at the center thereof as shown in Fig. 51. The aluminum sheet was coated with cast polypropylene film (CPP) to a thickness of 50 μm as the sealant layer and was coated with biaxially oriented polypropylene film on the back surface to form a thin film.

The gas-permeable film was a transparent film made by laminating biaxially oriented polypropylene film (OPP) 20 μm thick and polyethylene film (PE) 40 μm thick.

2. Test method

With the heat transpiration apparatus shown in Fig. 17 of which heater 42 temperature (surface temperature) was set to 74°C, the insecticidal technical product 765 was transpired by heating the heat transpiration container that was held on the heater surface and was tilted by 50° from the horizontal plane so that the transpiring liquid 76 made contact with the gas-permeable film 78, while heating on the entire surface of the bottom.

Insecticide component transpired from the heat transpiration container 72 was collected in a silica gel column. The insecticide component was extracted from the silica gel with chloroform and concentrated, before making quantitative analysis with gas chromatograph to determine the amount of transpiration over a period of 12 hours. The results are shown in Table 25. The amount of transpiration was averaged over five samples.

Table 25

| Heating time (hrs.) | 0-12 | 12-24 | 24-36 | 36-82 | 82-94 | 94-105 |
|---|---|---|---|---|---|---|
| Average of Amount transpired (mg/12 hours) | 4.12 | 3.67 | 3.80 | 4.27 | 3.92 | 4.05 |

From Table 25 it can be seen that the amount of transpiration is stabilized by heating the heat transpiration container 72 that was tilted so that the transpiring liquid 76 made contact with the gas-permeable film 78, and substantially constant amount of transpiration is maintained over a long period of time.

Example 13

<Evaluation of visibility from distance when pigment is added>

The container body having shape as shown in Fig. 51 was filled with 100 mg of transfluthrin containing the pigment with the concentration (% by weight) shown in Table 26 in the recess 50, and top of the recess 50 was covered with gas-permeable film, thereby making the heat transpiration container. Specifications of the container body and the gas-permeable film were as follows.

The container body was made from aluminum sheet 100 μm thick measuring 25 mm by 25 mm having the recess 125 measuring 17 mm by 17 mm by 2 mm in depth formed at the center thereof as shown in Fig. 51. The aluminum sheet was coated with cast polypropylene film (CPP) to a thickness of 50 μm as the sealant layer.

The gas-permeable film was a transparent film made by laminating microporous nylon (Ny) film 15 μm thick and cast polypropylene film (CPP) 50 μm thick.

2. Test method

The recess 125 of the container body was filled with 800 mg of transfluthrin containing the pigment added thereto, and top of the recess was covered with the gas-permeable film. Visibility of transfluthrin from a distance of 3 m was evaluated with the results shown in Table 26. In Table 26, "○" indicates that the object can be visually recognized, and "×" indicates that the object cannot be visually recognized.

Table 26

| Pigment | 0.5% | 0.1% | 0.03% | 0.01% | 0.003% | 0.001% | 0.0001% |
|---|---|---|---|---|---|---|---|
| Orange No. 403 | ○ | ○ | ○ | ○ | ○ | ○ | × |
| Green No. 202 | ○ | ○ | ○ | ○ | ○ | ○ | × |
| Purple No. 201 | ○ | ○ | ○ | ○ | ○ | ○ | × |
| Plast Blue 8540 | ○ | ○ | ○ | ○ | ○ | ○ | × |
| Red No. 225 | ○ | ○ | ○ | ○ | ○ | ○ | × |
| Scarlet (Red) | ○ | ○ | ○ | ○ | ○ | ○ | × |
| Yellow No. 204 | ○ | ○ | ○ | ○ | × | × | × |
| Green No. 202 + Yellow No. 204 (1:1) | ○ | ○ | ○ | ○ | ○ | ○ | × |
| Green No 202 + Purple No. 201 (1:1) | ○ | ○ | ○ | ○ | ○ | ○ | × |
| Purple No. 201 + Red No. 225 (1:1) | ○ | ○ | ○ | ○ | ○ | ○ | × |
| Green No. 202 + Orange No. 403 (3:2) | ○ | ○ | ○ | ○ | ○ | ○ | × |

From Table 26, it can be seen that adding 0.001% of pigment makes the insecticidal technical product contained in the heat transpiration container visible from a distance of 3 m.

Example 14

<Evaluation of heat resistance and light fastness of added pigment>

Similar to Example 13, recess 50 of a container body (Al 100 µm/cast polypropylene film 50 µm) was filled with 100 mg of transfluthrin containing 0.03% by weight of pigment shown in Table 27 added thereto, and top of the recess 50 was covered with the gas-permeable film, thereby making the heat transpiration container. Heat resistance of the pigment was evaluated after keeping the heat transpiration container 72 in an incubator controlled at 100°C for two weeks, and light fastness of the pigment was evaluated after one month of irradiation with scattered light. The results shown in Table 27. In Table 27, "○" indicates that there is not change in the color.

Table 27

| Pigment | Heat resistance of pigment (100°C, for two weeks) | Light fastness of pigment (one month under scattered light) |
|---|---|---|
| Orange No. 403 | ○ | ○ |
| Green No. 202 | ○ | ○ |
| Purple No. 201 | ○ | ○ |
| Green No. 202 + Purple No. 201 (1:1) | ○ | ○ |
| Purple No. 201 + Red No. 225 (1:1) | ○ | ○ |
| Green No. 202 + Orange No. 403 (3:2) | ○ | △* |

* Browning a little

From Table 27, it was confirmed that all the pigments used experienced no fading nor change in color after heating or irradiation with scattered light.

Example 15

<Evaluation of change in color tone and light fastness after thermal transpiration>

1. Heat transpiration container

The recess 125 of a container body having the shape shown in Fig. 51 was filled with 1240 mg of transfluthrin containing 0.03% by weight of pigment shown in Table 28 added thereto, and top of the recess 50 was covered with the gas-permeable film, thereby making the heat transpiration container. Specifications of the container body and the gas-permeable film were as follows.

The container body was made from aluminum sheet 100 μm thick measuring 33 mm by 25 mm having the recess 50 measuring 26 mm by 17 mm by 3 mm in depth formed at the center thereof as shown in Fig. 51. The aluminum sheet was coated with cast polypropylene film to a thickness of 50 μm as the sealant layer.

The gas-permeable film was a transparent film made by laminating cast polypropylene film (CPP) 70 μm.

2. Test method

With the heat transpiration apparatus shown in Fig. 17 of which heater temperature (surface temperature) was set to 100°C, the chemical was transpired by heating the heat transpiration container that was held on the heater surface and was tilted by 50° from the horizontal plane, while heating on the entire surface of the bottom of the container. When the liquid amount decreased to 100 mg through transpiration, change in color tone was evaluated and then light fastness (fading or change in color) was evaluated by irradiating with scattered light. The results are shown in Table 28.

Table 28

| Pigment | Change in color tone after transpiration | Light fastness (one month under scattered light) | Evaluation |
|---|---|---|---|
| Orange No. 403 | None | Good | ○ |
| Green No. 202 | None | Good | ○ |
| Purple No. 201 | None | Good | ○ |

From Table 28, it was confirmed that there was no change in color tone after transpiration, and the color did not fade after irradiation with scattered light.

Example 16

<Comparison of visibility with other heat transpiration apparatus >

Thermal transpiration was carried out similarly to example 15 by using resmethrin containing 0.03% by weight of Orange No. 403. Upon lapse of one fifth of the designed period use, visibility of the remaining amount of the liquid was evaluated by ten persons at distances of 1m, 2m and 3 m.

For the purpose of comparison, an absorbent wick type heat transpiration apparatus that sucks up the transpiring liquid with an absorbent wick and transpires it with heat, a solid mat type heat transpiration apparatus transpires the transpiring liquid adsorbed in a porous base material (solid mat) such as fibrous plate by heating it, and a gel formulation type heat transpiration apparatus that heats transpiring liquid included in a gel formulation were used, and visibility was evaluated at the time one fifth of the designed period of use elapsed. 0.03% by weight of Orange No. 403 was added to the transpiring liquid, the mat and the gel formulation. The results are shown in Table 29. In Table 29, "○" indicates that transpiring liquid is visually recognizable, "Δ" indicates that it is difficult but visually recognizable, and "×" indicates that it is not visually recognizable.

Table 29

| Heat transpiration apparatus | Visibility at distance | | |
|---|---|---|---|
| | 1 m | 2 m | 3 m |
| Heat transpiration apparatus of this invention | O | O | O |
| Absorbent wick type heat transpiration apparatus | O | O | △ |
| Solid mat type heat transpiration apparatus | × | × | × |
| Gel formulation type heat transpiration apparatus | ×* | ×* | ×* |

*It was not confirmed whether or not, it came to the end, although it was visually recognizable.

From Table 29, it was confirmed that in the heat transpiration apparatus of the present invention, adding the pigments to the insecticidal technical product made it more visually recognizable and easier to know the amount of the liquid that remains in the container than in the case of heat transpiration apparatus of the prior art.

Example 17

<Recognizing the end of transpiring liquid>

1. Heat transpiration container

Five container bodies having the recess 125 of the shape shown in Fig. 52(a) (hereinafter called the recess shape 1) were prepared. Recesses 125 of the container bodies were filled with 100 mg, 60 mg, 40 mg, 20 mg and 0 mg of the transpiring liquid, respectively, and were covered with the gas-permeable film at the top to make the heat transpiration containers. Transfluthrin containing 0.01% by weight of pigment (Green No. 202) was used as the transpiring liquid.

Five container bodies having the recess 126 of the shape shown in Fig. 52(b) and five container bodies having the recess 127 of the shape shown in Fig. 52(c) (hereinafter called the recess shapes 2 and 3) were prepared, and the heat transpiration containers were made similarly to those having the recess shape 1. The container bodies and the gas-permeable films used in Fig. 52(a) to (c) were made of the material in size substantially the same as those of Example 12.

2. Test method

A control heat transpiration container having the recess 125 of the recess shape 1 filled with 300 mg of the transpiring liquid was shown to a panel of 30 persons. Then the heat transpiration containers filled with 100 mg, 60 mg, 40 mg, 20 mg and 0 mg of the transpiring liquid were shown to the panel at a distance of 1 m to tell in which heat transpiration container they could determine the end (transpiring liquid was used up).

Similarly, the heat transpiration containers having the recesses of the recess shapes 2 and 3 were shown to the 30 persons, to tell in which heat transpiration container they could determine the end.

The results are shown in Table 30.

Table 30

| Recess shape | Number of persons determined the end (person) | | | | |
|---|---|---|---|---|---|
| | 100 mg | 60 mg | 40mg | 20 mg | 0 mg |
| 1 | 5 | 11 | 7 | 5 | 2 |
| 2 | 0 | 5 | 10 | 12 | 3 |
| 3 | 0 | 3 | 12 | 13 | 2 |

From Table 30, it can be seen that, since majority of the panel determined the end when less liquid remained and there was less variability in the number of persons determined the end in the cases of recess shapes 2 and 3, than the recess shape 1, the end can be more precisely determined when width of the recess of the heat transpiration container decreases to the end.

(V)

Examples

<Fabrication of heat transpiration container and heat transpiration apparatus>

The container body having shape as shown in Fig. 30 and Fig. 31 and dimensions and angles shown below was filled with 300 mg of transfluthrin as the insecticidal technical product 133, and aperture was covered with gas-permeable film 135 (a transparent film made by laminating biaxially oriented polypropylene film (OPP) 20 μm thick and a polyethylene film (PE) 40 μm thick), thereby making the heat transpiration container 132 that was held on the heater surface 137 so that the insecticidal technical product 133 touched the film 135. Ratio of the capacity (volume of air) of the air reservoir 136 to the volume of the insecticidal technical product 133 (liquid volume) in the container body was set to 1: 2.1.

The container body was made from aluminum sheet 250 μm thick having an extended portion 134b shown in Fig. 30 and Fig. 31.

t1: 5.2 mm

t2: 1 mm
θ1: 37°
θ2: 143°
θ3: 53°
θ4: 85°
θ5: 85°

<Test method>

The chemical was transpired by setting the heater temperature (surface temperature) to 110°C, and heating a portion of the bottom surface of the container body 134 other than the extended portion 134b, while observing to see whether the film 135 would touch the bottom of the container body 134 during the period early stage to latter stage of transpiration.

Comparative Examples

The chemical was transpired similarly to the example except that the container body 130 without the extended portion as shown in Fig. 53(a) was used and the heat transpiration container was filled with the insecticidal technical product 133 without providing the air reservoir, while observing to see whether the film 135 would touch the bottom surface 130a of the container body 130 during the period early stage to latter stage of transpiration.
Container body: Aluminum container made of Al sheet 100 μm thick

Depth of bottom from mouth: 3 mm

Test results of the Examples and the Comparative Examples are shown in Table 31. In the Table, "○" indicates that the film does not touch the bottom of the container body, and the decreasing rate of the insecticidal technical product is proportional to the lowering rate of the liquid level. "×" indicates that the film touches the bottom of the container body near the center thereof with much of the transpiring liquid located unevenly along the periphery, and the decreasing rate of the insecticidal technical product is not proportional to the lowering rate of the liquid level.

Table 31

| | Early stage of transpiration Liquid amount: 300 mg | Middle stage of transpiration Liquid amount: 150 mg | Latter stage of transpiration Liquid amount: 30 mg |
|---|---|---|---|
| Examples | O | O | O |
| Comparative Examples | O | × | × |

As shown in Table 31, amount of the remaining insecticidal technical product 133 could not be precisely determined in the comparative example, since center of the film 135 touched the bottom surface 130a of the container body 130 near the center thereof at a time after the middle of the transpiration period. In the Examples, in contrast, the film 135 did not touch the bottom of the container body 134, and amount of the remaining insecticidal technical product 133 could be precisely determined.

Test example

(Relation between surface temperature of gas-permeable film and liquid temperature)

After 30 minutes of use with a 30-day capacity cartridge (heat transpiration container made of Al) being set in a heat transpiration apparatus shown in Fig. 35, surface temperature of the film and liquid temperature were measured with a thermocouple.

<Test conditions>

Cartridge: 30-day capacity cartridge (similar shape as shown in Fig. 19(b))
Heater temperature: 109.5°C when incorporated in the instrument (individual temperature 105.7°C)
Ambient temperature: 25±1°C

<Test results>

Temperature of Al at the top: 81.7°C
Gas phase film temperature: 68.4°C
Liquid phase film temperature: 81.0°C (Liquid temperature: 84.1°C)

[INDUSTRIAL APPLICABILITY]

(I) The method of the present invention and the heat transpiration container used in the method in which the insecticidal technical product or the modified insecticidal technical product is heated to transpire can transpire the insecticide component more efficiently than the conventional heat transpiration method using a liquid absorbent wick that employs heat transpiration of insecticidal liquid with water added thereto. Thus the present invention can eliminate the drawbacks of the insecticidal liquid with water added thereto and makes a solvent unnecessary during heat transpiration. As a result, it becomes unnecessary to distinguish water-soluble and oil-based properties which is required when a solvent is used, and it is enabled to make the container and the apparatus compact.
(II) The method of the present invention has the merit of evaporating the transpiring liquid stably over an extended period of time.
(III) According to the present invention, when the transpiring liquid is transpired by heating the container that may be provided with a flange along the periphery of the recess that contains the transpiring liquid and further the rib formed at the bottom of the recess to protrude into the recess space as required, since the surface temperature in the recess is controlled by means of the depth of the recess, area of the flange or volume of the rib, thus desired temperature can be obtained even when the heater temperature remains constant and, even when the transpiring liquid is changed, the temperature can be set to an optimum level for the transpiring liquid, so that the transpiration rate of the transpiring liquid can be set freely.
(IV) According to the present invention, since the heat transpiration container is placed or held on the heater surface at such an angle as the transpiring liquid touches the gas-permeable film, amount of liquid in the container can be easily known from the liquid level and it can be easily determined when the transpiring liquid should be replaced. Heat transpiration at such an angle as the transpiring liquid touches the gas-permeable film has also such an effect as the amount of transpiration is stabilized over a long period of time.
(V) According to the present invention, since the gas-permeable film can be prevented from touching the bottom of the container body when the amount of the transpiring liquid decreases, amount of the transpiring liquid contained in the heat transpiration container can be known precisely.

According to the present invention, when the extended portion that is formed to protrude in the direction of depth and/or width of the container body for forming the air reservoir, it is made possible to set the liquid level in the container higher and prevent the appearance of the apparatus as the commercial value from being degraded while ensuring the predetermined volume of the air reservoir.

The present invention can provide the heat transpiration apparatus that can maintain a favorable level of transpiration efficiency of chemical and is convenient for use.

**Claims**

1. A heat transpiration method for the extermination of vermin, which comprises heating an insecticidal technical product or a modified insecticidal technical product containing an additive so as to transpire.

2. The heat transpiration method according to claim 1, wherein the insecticidal technical product or the modified insecticidal technical product is put into a container and the container is heated so as to transpire the insecticidal technical product or modified insecticidal technical product.

3. The heat transpiration method according to claim 1 or 2, wherein the insecticidal technical product or the modified insecticidal technical product is transpired while a gas-permeable film is put into contact with the liquid surface of the insecticidal technical product or modified insecticidal technical product.

4. The heat transpiration method according to claim 1 or 2, wherein liquid surface of the insecticidal technical product or the modified insecticidal technical product is exposed to the transpiration space.

5. A heat transpiration container used in the heat transpiration method of any one of claims 1 to 3, comprising a container body that contains an insecticidal technical product or a modified insecticidal technical product and a gas-permeable film that closes a transpiration aperture of the container body.

6. A heat transpiration container used in the heat transpiration method of any one of claims 1 to 3, comprising a container body that contains an insecticidal technical product or a modified insecticidal technical product containing an additive and a gas-permeable film that makes contact with the liquid surface of the insecticidal technical product or the modified insecticidal technical product at least during heat transpiration.

7. A heat transpiration apparatus comprising a container body that contains a transpiring liquid, an inner film and an outer film that are put one on another and close a transpiration aperture of the container body, feeding means for supplying the transpiring liquid between the inner film and the outer film and a heater for heating the container body, wherein at least the outer film among the inner film and the outer film is a gas-permeable film.

8. The heat transpiration apparatus according to claim 7, wherein the feeding means is a liquid introducing port located at a position where the inner film touches the transpiring liquid when the container body is held at such an angle as the transpiring liquid touches the inner film.

9. A heat transpiration container comprising a container body that contains a transpiring liquid and an inner film and an outer film that are put on one another and close a transpiration aperture of the container body, wherein at least the outer film among the inner film and the outer film is a gas-permeable film.

10. The heat transpiration container according to claim 9, wherein at least one of the inner film and the outer film is embossed to form a channel for supplying liquid.

11. A method of producing a heat transpiration container, which comprises closing a transpiration aperture of a container body with an inner film, piercing the inner film by a feeder nozzle that enters the container body for supplying a transpiring liquid, supplying the transpiring liquid through the feeder nozzle into the container body, and laminating an outer film onto the outer surface of the inner film for sealing.

12. The method of producing the heat transpiration container according to claim 11, wherein a through hole that is left after removing the feeder nozzle from the container body is used as the liquid introducing port of claim 8.

13. A heat transpiration apparatus comprising a container body that contains a transpiring liquid, an inner film and an outer film that are placed one on another and close a transpiration aperture of the container body and a heater for heating the container body, wherein the inner film is a liquid absorbent film, the outer film is a gas-permeable film and a part of the inner film makes contact with the transpiring liquid.

**14.** A heat transpiration container comprising a container body that contains a transpiring liquid and an inner film and an outer film that are put one on another and close a transpiration aperture of the container body, wherein the inner film is a liquid absorbent film and the outer film is a gas-permeable film.

**15.** The heat transpiration container according to claim 14, wherein the liquid absorbent film is an unwoven fabric.

**16.** A method for controlling a heat transpiration temperature, which comprises controlling the surface temperature in the recess by means of depth of the recess, in case that the container having the recess that contains the transpiring liquid is heated so as to transpire the transpiring liquid.

**17.** A method for controlling a heat transpiration temperature, which comprises controlling the surface temperature in the recess by means of the area of a flange, in case that the container having the flange provided along the periphery of the recess that contains the transpiring liquid is heated so as to transpire the transpiring liquid.

**18.** A method for controlling a heat transpiration temperature, which comprises controlling the surface temperature in the recess by means of the volume of a rib, in case that the container having the rib provided to protrude from the bottom of the recess that contains the transpiring liquid into the recess space is heated so as to transpire the transpiring liquid.

**19.** The method for controlling the heat transpiration temperature according to any one of claims 16 to 18, wherein the transpiring liquid is an insecticidal technical product.

**20.** A heat transpiration apparatus comprising a heat transpiration container comprising a container body that contains a transpiring liquid and a transparent or semi-transparent gas-permeable film that closes a transpiration aperture of the container body, which is placed or held on a heater surface at such an angle as the transpiring liquid touches the film.

**21.** A heat transpiration apparatus comprising a heat transpiration container comprising a container body that contains a transpiring liquid and a transparent or semi-transparent gas-permeable film that closes a transpiration aperture of the container body, which is placed or held on a heater surface that has such an angle as the transpiring liquid touches the film.

**22.** The heat transpiration apparatus according to claim 21, wherein the heat transpiration container is placed or held on the heater surface that has an angle of 10 to 90° from the horizontal plane.

**23.** The heat transpiration apparatus according to any one of claims 20 to 22, wherein the transpiration aperture of the container body has an inclined lower end that becomes narrower toward the end.

**24.** The heat transpiration apparatus according to any one of claims 20 to 23, wherein the transpiring liquid is an insecticidal technical product.

**25.** The heat transpiration apparatus according to any one of claims 20 to 24, wherein the transpiring liquid contains a colorant.

**26.** A heat transpiration method, which comprises placing or holding a heat transpiration container comprising a container body that contains a transpiring liquid and a transparent or semi-transparent gas-permeable film that closes a transpiration aperture of the container body is placed or held on the heater surface at such an angle as the transpiring liquid touches the film, and heating the transpiring liquid so as to transpire.

**27.** A heat transpiration container comprising a container body that opens at the top and contains a transpiring liquid therein and a transparent or semi-transparent gas-permeable film that closes the aperture of the container body, wherein there is provided, in the container body that is closed by the gas-permeable film, an air reservoir that prevents the gas-permeable film from touching the bottom of the container body as the transpiring liquid transpires.

**28.** A heat transpiration container comprising a container body that opens at the top and contains a transpiring liquid therein and a transparent or semi-transparent gas-permeable film that closes the aperture of the container body, wherein an extended portion is provided to protrude in the direction of depth and/or the direction of width of the container body at one end of the container body, so as to form an air reservoir.

**29.** The heat transpiration container according to claim 28, wherein a lower surface of the extended portion is formed in an inclined surface so that the transpiring liquid flows from within the extended portion to the lower end in the container body when the container body is placed in a vertical or tilted posture with the extended portion located at the top.

**30.** The heat transpiration container according to claim 29, wherein the angle between the lower surface of the extended portion and the bottom surface of the extended portion is larger than 90° and less than 180°.

**31.** A heat transpiration apparatus comprising a heater and the heat transpiration container of any one of claims 27 to 30 which is placed or held on the heater surface in a vertical or tilted posture so that the transpiring liquid touches the gas-permeable film.

**32.** A heat transpiration method which comprises heating the heat transpiration container of any one of claims 27 to 30 in a vertical or tilted posture so that the transpiring liquid touches the gas-permeable film to transpire the transpiring liquid.

**33.** A heat transpiration apparatus for heating a transpiring liquid holding body in a tilted posture by means of a heating section to transpire the liquid, comprising an instrument body that incorporates the heating section and a lid provided on the instrument body so as to cover the entire transpiring liquid holding body, wherein a plurality of elongated apertures are formed in the lid surface that faces the outside when the lid is closed on the instrument body, proportion of the plurality of apertures to the area of the lid surface is set to 40% or more, and each of the apertures is formed to be 5 mm or smaller along the shorter side thereof.

**34.** A heat transpiration apparatus for heating a transpiring liquid holding body by means of a heating section to transpire the liquid, comprising an instrument body that incorporates the heating section and a lid provided on the instrument body so as to cover the transpiring liquid holding body, wherein aperture section is formed in the lid surface that faces the outside when the lid is closed on the instrument body, while proportion of the aperture section to the area of the lid surface is set to 40% or more, and maximum size of the aperture section is formed to be 5 mm or smaller.

**35.** The heat transpiration apparatus according to claim 33, wherein each of the plurality of apertures becomes larger in length along the shorter side toward the transpiring liquid holding body.

**36.** The heat transpiration apparatus according to claim 35, wherein the lid has a plurality of pillars that divide the plurality of apertures along the longitudinal direction thereof and each of the pillars has triangular cross section.

**37.** The heat transpiration apparatus according to claim 33, wherein a transpiration aperture is formed in the gap between the instrument body and the upper end of the lid, and/or in the instrument body when the lid is closed on the instrument body.

**38.** The heat transpiration apparatus according to claim 37, wherein the transpiration aperture is provided so as to open vertically upward at the top end of the transpiring liquid holding body.

**39.** The heat transpiration apparatus according to claim 33, wherein a first air intake port is formed in the gap between the instrument body and the upper end of the lid, and/or in the instrument body when the lid is closed on the instrument body, and the first air intake port opens at a position lower in the vertical direction than the lower end of the transpiring liquid holding body.

**40.** The heat transpiration apparatus according to claim 33, wherein a second air intake port is provided at a position substantially opposite to the lid in the horizontal direction in the instrument body.

FIG. 1

(a)

(b)

FIG. 2

FIG. 3

(a)

(b)

FIG. 4

(a)

(b)

FIG. 5

FIG. 6

FIG. 7

(a)          (b)

FIG. 8

FIG. 9

FIG. 10

FIG. 11

FIG. 12

(a)

(b)

(c)

(d)

FIG. 13

FIG. 14

FIG. 15

(a)

D1

D2

62   63   61

69

X   X

(b)

63   69   61

62   h

FIG. 16

68   64

63   62

61   67

66

FIG. 17

FIG. 18

(a)

(b)

FIG. 19

(a)                    (b)                    (c)

81                     82                     83

FIG. 20

(a)                    (b)                    (c)

84                     85                     86

(d)                    (e)                    (f)

87                     88                     90

                       89                     91

FIG. 21

(a)　　　　　　　(b)　　　　　　　(c)

92　　　　　　　93　　　　　　　94

FIG. 22

(a)　　　　　　　(b)　　　　　　　(c)

95　　　　　　　96　　　　　　　97

FIG. 23

(a)　　　　　　　(b)

98

99

100

FIG. 24

(a)

(b)

(c)

101

102

103

FIG. 25

(a)

(b)

(c)

104

105

106

FIG. 26

110

107

108

L

76

109

FIG. 27

FIG. 28

FIG. 29

FIG. 30

FIG. 31

(a)                    (b)                    (c)

EP 1 547 464 A1

FIG. 32

FIG. 33

FIG. 34

FIG. 35

FIG. 36

(a)

(b)

FIG. 37

FIG. 38

FIG. 39

FIG. 40

FIG. 41

FIG. 42

FIG. 43

FIG. 44

FIG. 45

FIG. 46

(a)

220

221

221b

221a

(b)

231c

231

230

231b

231a

(c)

241c

241

240

241b

241a

FIG. 47

(a)

35

36

Z

Z

(b)

35

36

FIG. 48

D1

D2

C4

C1

C2

C3

C3

a

b

e

d

c

FIG. 49

FIG. 50

FIG. 51

(a)

125

Y                    Y

(b)

125

FIG. 52

(a)          (b)          (c)

125          126          127

FIG. 53

(a)

(b)

(c)

## INTERNATIONAL SEARCH REPORT

International application No.

PCT/JP03/06160

**A. CLASSIFICATION OF SUBJECT MATTER**
Int.Cl⁷ A01M1/20, A61L9/03

According to International Patent Classification (IPC) or to both national classification and IPC

**B. FIELDS SEARCHED**

Minimum documentation searched (classification system followed by classification symbols)
Int.Cl⁷ A01M1/20

Documentation searched other than minimum documentation to the extent that such documents are included in the fields searched

| | | | |
|---|---|---|---|
| Jitsuyo Shinan Koho | 1922–1996 | Toroku Jitsuyo Shinan Koho | 1994–2003 |
| Kokai Jitsuyo Shinan Koho | 1971–2003 | Jitsuyo Shinan Toroku Koho | 1996–2003 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2002-126068 A (Kao Corp.),<br>08 May, 2002 (08.05.02),<br>Full text; Figs. 1 to 2<br>(Family: none) | 1-3,5,6 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 29 September, 2003 (29.09.03) | 14 October, 2003 (14.10.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

105

## INTERNATIONAL SEARCH REPORT

| International application No. |
| --- |
| PCT/JP03/06160 |

**Box I    Observations where certain claims were found unsearchable (Continuation of item 2 of first sheet)**

This international search report has not been established in respect of certain claims under Article 17(2)(a) for the following reasons:

1. ☐  Claims Nos.:

   because they relate to subject matter not required to be searched by this Authority, namely:

2. ☐  Claims Nos.:

   because they relate to parts of the international application that do not comply with the prescribed requirements to such an extent that no meaningful international search can be carried out, specifically:

3. ☐  Claims Nos.:

   because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:
With respect to each of the inventions, the claims and special technical matters are as follows:
1. claims 1-3, 5 and 6 (all the technical matters do not emerge beyond the prior art)
2. claim 4 (opening the liquid surface of pesticide to be transpired into transpiration space)
3. claims 7-15 (inside film and outside film)
4. claims 16-19 (regulating thermal transpiration temperature)
5. claims 20-26 (mounting or holding the transpiration liquid on a heater
(continued to extra sheet)

1. ☐  As all required additional search fees were timely paid by the applicant, this international search report covers all searchable claims.

2. ☐  As all searchable claims could be searched without effort justifying an additional fee, this Authority did not invite payment of any additional fee.

3. ☐  As only some of the required additional search fees were timely paid by the applicant, this international search report covers only those claims for which fees were paid, specifically claims Nos.:

4. ☒  No required additional search fees were timely paid by the applicant. Consequently, this international search report is restricted to the invention first mentioned in the claims; it is covered by claims Nos.: 1-3, 5 and 6

**Remark on Protest**    ☐    The additional search fees were accompanied by the applicant's protest.

☐    No protest accompanied the payment of additional search fees.

Form PCT/ISA/210 (continuation of first sheet (1)) (July 1998)

| INTERNATIONAL SEARCH REPORT | International application No. |
|---|---|
| | PCT/JP03/06160 |

Continuation of Box No. II of continuation of first sheet(1)

surface at such an angle that the transpiration liquid is in contact with film)
    6.    claims 27-32 (air retention part)
    7.    claims 33-40 (lid body)

Form PCT/ISA/210 (extra sheet) (July 1998)